# EUROPEAN PATENT APPLICATION

(11) **EP 4 083 069 A1**
(43) Date of publication of application: **02.11.2022**
(21) Application number: 20907635.5
(22) Date of filing: 28.12.2020
(51) Int. Cl.: C07K 16/28, C12N 15/13, A61K 39/395, A61P 35/00

(54) **ANTI-OX40 ANTIBODY AND USE THEREOF**

(30) Priority: 27.12.2019 CN 201911383060; 19.06.2020 US 202063041532 P
(71) Applicant: HiFiBiO (HK) Limited, Central Hong Kong 999077 (CN)
(72) Inventor: ZHANG, Hongkai, Tianjin 300350 (CN); WANG, Yuan, Tianjin 300350 (CN); LU, Yun-Yueh, Cambridge, Massachusetts 02139 (US); SHEN, Yuqiang, Cambridge, Massachusetts 02139 (US); BELTRAMINELLI, Nicola Arturo Aldo, Cambridge, Massachusetts 02139 (US); SCHWEIZER, Liang, Cambridge, Massachusetts 02139 (US); ADRIAN, Francisco, Cambridge, Massachusetts 02139 (US); RAUE, Klaus Andreas, Cambridge, Massachusetts 02139 (US); ZHANG, Qian, Cambridge, Massachusetts 02139 (US); GAN, Jinping, Cambridge, Massachusetts 02139 (US)
(74) Representative: CH Kilger Anwaltspartnerschaft mbB
(86) International application number: PCT/CN2020/140186
(87) International publication number: WO 2021/129872

(57) **Abstract**

Provided are an anti-OX40 antibody, a composition including the anti-OX40 antibody, a nucleic acid encoding the anti-OX40 antibody, a method for preparing the anti-OX40 antibody, and a use of the anti-OX40 antibody.

## Description

### Technical Field

The present invention relates to a novel anti-OX40 antibody, a composition including the anti-OX40 antibody, a nucleic acid encoding the anti-OX40 antibody, a method of preparing the anti-OX40 antibody, and a use of the anti-OX40 antibody.

### Background Art

Antitumor immune responses in patients with solid tumors have been enhanced by treatment with certain biological agents. For example, two anti-PD-1 monoclonal antibodies: nivolumab (OPDIVO^{®}) and pembrolizumab (KEYTRUDA^{®}), have been approved in the US and EU for the treatment of diseases such as unresectable or metastatic melanoma and metastatic non-small cell lung cancer. Treatment of patients with these drugs has produced an anti-tumor response as measured by improvement in progression-free survival and/or overall survival. There is still a need in the art for more cancer treatment products and methods to complement the existing standard of care.

PD-1 and CTLA-4 play an immunosuppressive role in the process of T cell activation, thereby inhibiting the immune killing function of T cells to tumor cells. Therefore, blocking monoclonal antibodies against these two targets can relieve this immunosuppression and restore the anti-tumor immune function of T cells. In addition to such inhibitory immune checkpoint molecules, activating immune checkpoint molecules gradually become the new targets for drug development.

Activated immune checkpoint molecules mainly refer to the costimulatory signaling molecules-T cell costimulatory receptors in T cell activation, which belong to the tumor necrosis factor receptor (TNFR) family. They can be used to regulate the proliferation, activation and differentiation of T cells, including OX40, CD40, 4-1BB and GITR, among others.

The OX40 receptor, also known as CD134 and TNFRSF4 (tumor necrosis factor receptor superfamily member 4), is a member of the TNFR superfamily of receptors. Unlike CD28, it is not constitutively expressed on resting naive T cells. OX40 is a secondary costimulatory immune checkpoint molecule expressed 24 to 72 hours after the activation. Its ligand OX40L (also known as CD252, TNFSF4) is also not expressed on resting antigen-presenting cells, but is expressed after the activation. The expression of OX40 is dependent on the full activation of T cells.

OX40 binds to its ligand OX40L to transmit costimulatory signals. The interaction between OX40 and OX40L can recruit TNFR-related molecules (TRAFs) with the intracellular domain of OX40 to form a signaling complex containing IKKα and IKKβ, as well as PI3k and PKB (Akt). OX40 can also cooperate with the TCR signaling to enhance intracellular Ca²⁺ through an unknown mechanism, thereby enhancing NFAT entry into the nucleus. OX40 activates the canonical NF-κB1 pathway or the non-canonical NF-κB2 pathway, PI3k/PKB and NFAT pathways, thereby regulating the genes for T cell division and survival, as well as promoting the transcription of cytokine genes and the expression of cytokine receptors. It is essential for cell survival. OX40 signaling can cause the downregulation of CTLA-4 and Foxp3.

When OX40 binds to its ligand OX40L, it helps improve the immune system's ability to respond, which includes: 1. increasing the survival and expansion of effector T cells and memory T cells, and increasing the secretion of cytokines (such as IL-2, IL-4, IL-5, IFN-γ); 2. reducing the immunosuppressive activity of regulatory T cells and further amplifying the effect of T cell activation. In the tumor microenvironment, immune activation can lead to OX40 expression. This can enhance the activation and proliferation of effector T cells and suppress the regulatory T cells, resulting in complex antitumor immune responses. At present, several clinical projects involving anti-OX40 antibody in the treatment of cancer can be retrieved on the Clinical Trials website.

There is a need in the art for more novel anti-OX40 antibodies to provide new cancer treatment options.

### Summary of the Invention

The present invention addresses the above need by providing a novel anti-OX40 antibody that can specifically bind to and activate OX40.

In one aspect, the present invention provides an isolated anti-OX40 antibody or an antigen-binding fragment thereof, which includes: a heavy chain variable region having a heavy chain CDR1 structural domain as shown in SEQ ID NO: 1, a heavy chain CDR2 structural domain as shown in SEQ ID NO: 2, and a heavy chain CDR3 structural domain as shown in SEQ ID NO: 3; and a light chain variable region having a light chain CDR1 domain as shown in SEQ ID NO: 9, a light chain CDR2 domain as shown in SEQ ID NO: 10, and a light chain CDR3 domain as shown in SEQ ID NO: 11.

In one aspect, the present invention provides an antibody-drug conjugate, which includes an OX40 antibody or antigen-binding fragment thereof as described herein and an additional therapeutic agent; preferably, the anti-OX40 antibody or antigen-binding fragment thereof is linked to the additional therapeutic agent by a linker.

In one aspect, the present invention provides a nucleic acid encoding the anti-OX40 antibody or antigen-binding fragment thereof as described herein.

In one aspect, the present invention provides an expression vector including a nucleic acid as described herein.

In one aspect, the present invention provides a host cell including a nucleic acid as described herein or an expression vector as described herein.

In one aspect, the invention provides a method for preparing an anti-OX40 antibody or antigen-binding fragment thereof as described herein, including culturing the host cell as described herein under a condition suitable for the expression of the antibody or antigen-binding fragment thereof, and recovering the expressed antibody or antigen-binding fragment thereof from the culture medium.

In one aspect, the present invention provides a pharmaceutical composition, including an anti-OX40 antibody or antigen-binding fragment thereof as described herein, or an antibody-drug conjugate as described herein, or a nucleic acid as described herein, or an expression vector as described herein; and a pharmaceutically acceptable carrier.

In one aspect, the present invention provides an anti-OX40 antibody or antigen-binding fragment thereof as described herein, or an antibody-drug conjugate as described herein, or a pharmaceutical composition as described herein, for use in the treatment of cancer.

In one aspect, the present invention provides a method for treating cancer, including administering to a subject in need thereof a therapeutically effective amount of an anti-OX40 antibody or antigen-binding fragment thereof as described herein, or an antibody-drug conjugate as described herein, or a pharmaceutical composition as described herein, thereby treating the cancer.

In one aspect, the present invention provides the use of an anti-OX40 antibody or antigen-binding fragment thereof as described herein, or an antibody-drug conjugate as described herein, or a pharmaceutical composition as described herein in the preparation of a medicament for the treatment of cancer.

In one aspect, the present invention provides an anti-OX40 antibody or antigen-binding fragment thereof as described herein, or an antibody-drug conjugate as described herein, or a pharmaceutical composition as described herein, for use to do one or more of the following: inhibit Treg function (for example, inhibit the suppressive function of Treg), kill cells expressing OX40 (for example, cells expressing a high level of OX40), enhance an effector T cell function and/or enhance a memory T cell function, reduce tumor immunity, enhance T cell function and/or deplete OX40-expressing cells.

In one aspect, the invention provides a use of an anti-OX40 antibody or antigen-binding fragment thereof as described herein, or an antibody-drug conjugate as described herein, or a pharmaceutical composition as described herein, in the preparation of a medicament to do one of the following: inhibit Treg function (for example, inhibit the suppressive function of Treg), kill cells expressing OX40 (for example, cells expressing a high level of OX40), enhance an effector T cell function and/or enhance a memory T cell function, reduce tumor immunity, enhance T cell function and/or deplete OX40-expressing cells.

In one aspect, the present invention provides a pharmaceutical combination, including an anti-OX40 antibody or antigen-binding fragment thereof as described herein, or an antibody-drug conjugate as described herein, or a pharmaceutical composition as described herein, and one or more additional therapeutic agents.

In one aspect, the present invention provides a kit including an anti-OX40 antibody or antigen-binding fragment thereof as described herein, or an antibody-drug conjugate as described herein, or a pharmaceutical composition as described herein, and preferably further including a drug delivery device.

In some embodiments, an antibody of the present invention includes one or more point mutations in the amino acid sequence thereof, where the point mutations are designed to improve the developability of the antibody. In a preferred embodiment, the one or more point mutations render the antibody more stable during expression in a host cell, purification during manufacture and/or formulation, and/or administration to a subject. In a preferred embodiment, the one or more point mutations render the antibody less likely to aggregate during manufacture and/or formulation. In some embodiments, the present invention provides a therapeutic antibody with minimized or reduced developability issues. For example, the hydrophobicity and/or the optimized charge can be removed or reduced by means of substituting one or more amino acids in the sequence thereof (for example, in one or more of the CDRs thereof).

One embodiment of the present invention is a monoclonal antibody or antigen-binding fragment thereof, where the monoclonal antibody or antigen-binding fragment thereof is capable of specifically binding to an epitope of OX40 corresponding to amino acid residues 56 to 74 of SEQ ID NO: 33 (SEQ ID NO: 34: CSRSQNTVCRPCGPGFYN).

Another embodiment of the present invention is a monoclonal antibody or antigen-binding fragment thereof capable of specifically binding to OX40, where the monoclonal antibody or antigen-binding fragment thereof does not interfere with the binding of an OX40 ligand to OX40.

Another embodiment of the present invention is a monoclonal antibody or antigen-binding fragment thereof, where the binding of the monoclonal antibody or antigen-binding fragment thereof does not interfere with the binding of an OX40 ligand to OX40 in a trimeric or multimeric aggregated state.

Another embodiment of the present invention is a monoclonal antibody or antigen-binding fragment thereof capable of specifically binding to OX40, where the binding of the antibody to OX40 results in agonistic signal transduction along with endogenous OX40 ligand signal transduction.

Another embodiment of the present invention is a monoclonal antibody or antigen-binding fragment thereof of any one of the above embodiments, where OX40 is human OX40.

Another embodiment of the present invention is a monoclonal antibody or antigen-binding fragment thereof of the above embodiments, where the epitope includes SEQ ID NO: 2.

Another embodiment of the present invention is a monoclonal antibody or antigen-binding fragment thereof of the above embodiments, where the antibody binds to OX40 with a K_{D} of about 1 nM to about 10 nM.

Another embodiment of the present invention is a monoclonal antibody or antigen-binding fragment thereof according to the above embodiments, where the binding of the monoclonal antibody or antigen-binding fragment thereof to OX40 does not downregulate OX40 expression or reduce the amount of OX40 present on the cell surface.

Another embodiment of the present invention is a method of treating cancer with the monoclonal antibody, where the cancer is a solid tumor or a non-solid tumor, or the cancer is a cancer having the OX40 expression on the cell surface of tumor-infiltrating T cells.

Another embodiment of the present invention is a method of detecting OX40 in a sample, where the method includes contacting the sample with a monoclonal antibody or antigen-binding fragment thereof of the present invention.

Another embodiment of the present invention is a method of determining an OX40 level in a subject, the method including:
a) obtaining a sample from the subject;
b) contacting the sample with a monoclonal antibody or antigen-binding fragment thereof of the present invention; and
c) determining the level of OX40 in the subject.

The sample is a tissue sample or a blood sample, or a cancer tissue sample.

### Brief Description of the Drawings

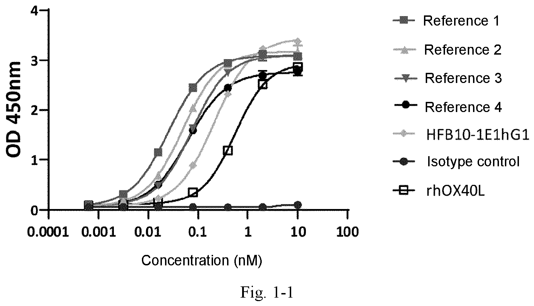

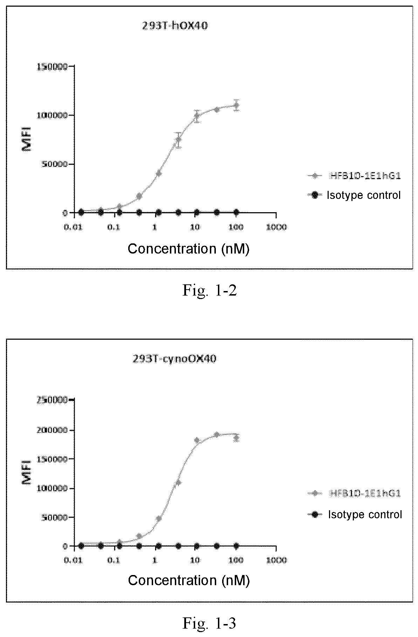

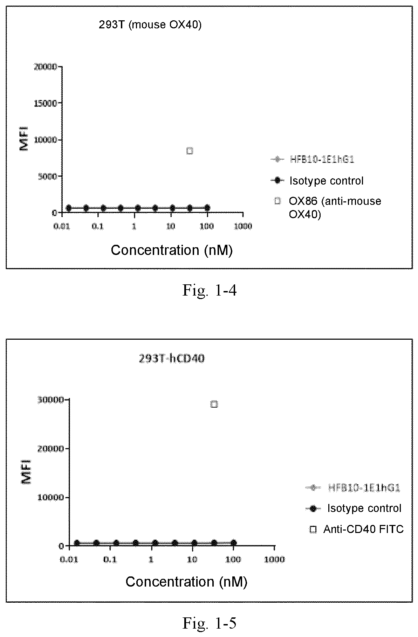

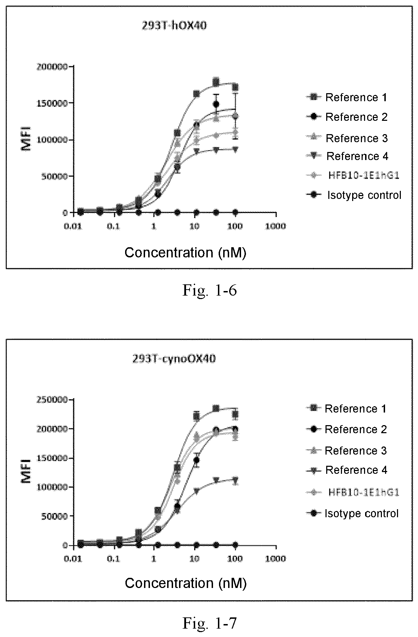

Fig. 1-1, the binding of HFB10-1E1hG1 to OX40 protein. The EC80 of HFB10-1E1hG1 is 0.71 nM.
Fig. 1-2, the binding of HFB10-1E1hG1 to human OX40 protein expressed on the surface of 293T cells. EC50 was 2 nM (MFI).
Fig. 1-3, the binding of HFB10-1E1hG1 to cynomolgus monkey OX40 protein expressed on the surface of 293T cells. EC50 was 2.9 nM (MFI).
Fig. 1-4, the binding of HFB10-1E1hG1 to mouse OX40 protein expressed on the surface of 293T cells. HFB10-1E1hG1 does not bind to mouse OX40 protein (MFI) expressed on the surface of 293T cells.
Fig. 1-5, the binding of HFB10-1E1hG1 to human CD40 protein expressed on the surface of 293T cells. HFB10-1E1hG1 does not bind to human CD40 protein (MFI) expressed on the surface of 293T cells.
Fig. 1-6, the binding of HFB10-1E1hG1, reference 1, reference 2, reference 3 and reference 4 to human OX40 protein expressed on the surface of 293T cells, respectively. EC50 of HFB10-1E1hG1 is 2.02 nM (MFI); EC50 of reference 1 is 2.67 nM (MFI); EC50 of reference 2 is 4.17 nM (MFI); EC50 of reference 3 is 1.92 nM (MFI); and EC50 of reference 4 is 2.11 nM (MFI).
Fig. 1-7, the binding of HFB10-1E1hG1, reference 1, reference 2, reference 3 and reference 4 to cynomolgus monkey OX40 protein expressed on the surface of 293T cells, respectively. EC50 of HFB10-1E1hG1 is 2.94 nM (MFI); EC50 of reference 1 is 2.91 nM (MFI); EC50 of reference 2 is 6.13 nM (MFI); EC50 of reference 3 is 2.57 nM (MFI); EC50 of reference 4 is 3.54 nM (MFI).

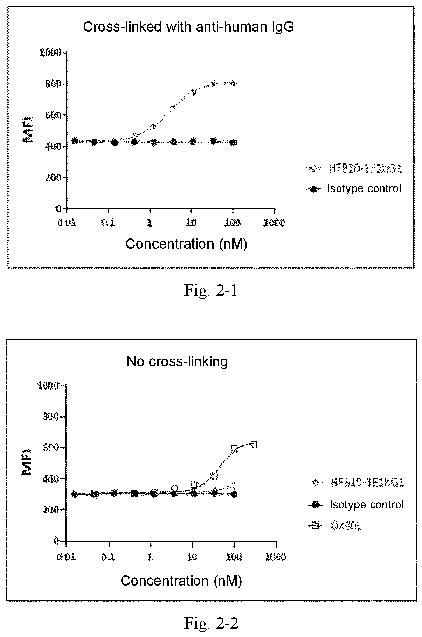

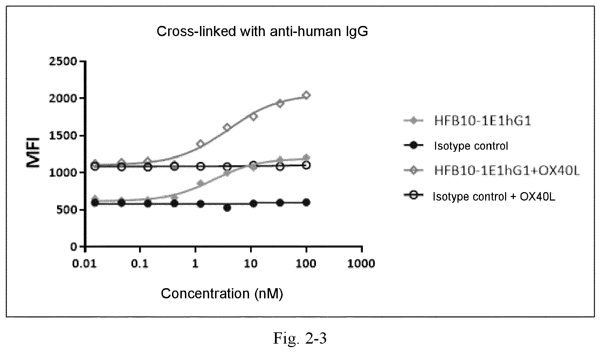

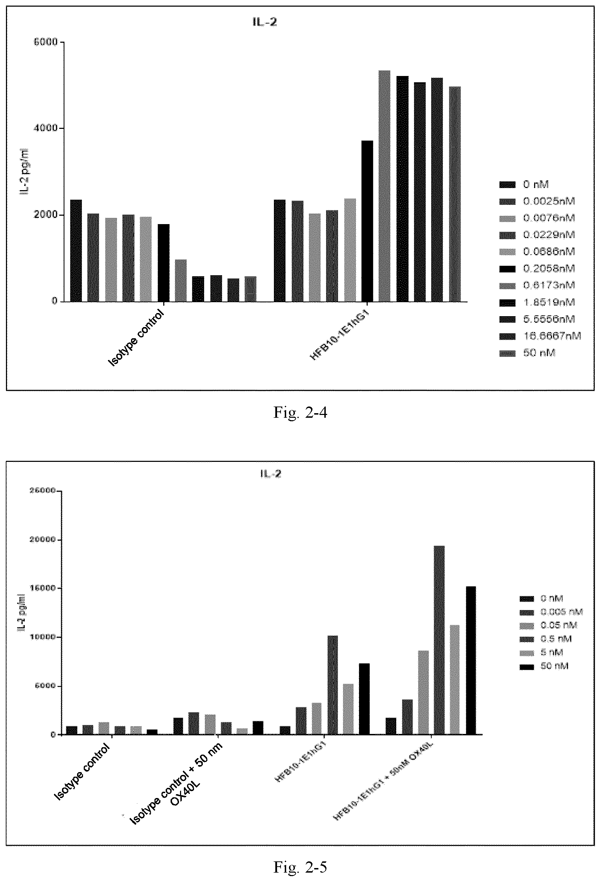

Fig. 2-1, the agonistic activity of HFB10-1E1hG1 in Jurkat reporter cells. The EC50 of HFB10-1E1hG1 is 2.9 nM (MFI of GFP) in the case of cross-linking with anti-human IgG.
Fig. 2-2, the agonistic activity of HFB10-1E1hG1 in Jurkat reporter cells. Without cross-linking with anti-human IgG, the EC50 of HFB10-1E1hG1 is much greater than when it is cross-linked with anti-human IgG. Trimeric OX40L recombinant protein alone can activate NF-kb signaling with EC50=45 nM (MFI of GFP) without cross-linking with anti-human IgG.
Fig. 2-3, the agonistic activity of HFB10-1E1hG1 in Jurkat reporter cells. HFB10-1E1hG1 shows a cooperative agonistic effect with OX40L in the case of cross-linking with anti-human IgG, and the addition of HFB10-1E1hG1 with OX40L enhances the MFI of GFP as compared to a single component.
Fig. 2-4, the agonistic activity of HFB10-1E1hG1 in primary CD4⁺ T cells. The agonistic activity of HFB10-1E1hG1 in primary CD4⁺ T cells is assayed by IL-2 secretion, with an EC50 of 0.2 nM.
Fig. 2-5, the agonistic activity of HFB10-1E1hG1 in primary CD4⁺ T cells. In primary CD4⁺ T cells, HFB10-1E1hG1 shows a cooperative agonistic effect with OX40L.
Fig. 3-1, the pharmacokinetic testing of HFB10-1E1hG1.
Fig. 4-1, in vivo antitumor efficacy of HFB10-1E1hG1.HFB10-1E1hG1 significantly inhibits tumor growth as compared to the PBS control.
Fig. 4-2, in vivo antitumor efficacy of HFB10-1E1hG1. HFB10-1E1hG1 shows no side effects that can result in significant weight loss in mice.
Fig. 4-3, in vivo antitumor efficacy (tumor size) of HFB10-1E1hG1 at different doses.
Fig. 4-4, in vivo antitumor efficacy (body weight) of HFB10-1E1hG1 at different doses.
Fig. 5-1, the accelerated stability experiment of HFB10-1E1hG1.SDS-PAGE results show that HFB10-1E1hG1 has good stability under different temperature conditions.
Fig. 5-2, the degradation experiment of HFB10-1E1hG1. SDS-PAGE results show that HFB10-1E1hG1 has good stability under different pH conditions.
Fig. 5-3, the oxidative stress experiment of HFB10-1E1hG1.SDS-PAGE results show that HFB10-1E1hG1 has good stability under oxidative stress.
Fig. 5-4, the freeze-thaw experiment of HFB10-1E1hG1. SDS-PAGE results show that HFB10-1E1hG1 has good stability under freeze-thaw conditions.
Fig. 6, the binding of HFB10-1E1hG1 to activated monkey CD4⁺ T cells. Symbols: actual data point; curve: nonlinear curve fit using a four-parameter model. The binding of HFB10-1E1hG1 to activated T cells isolated from three donors: #200107 (A: % positive cells, and B: MFI), #M20Z013009 (C: percentage of positive cells, and D: MFI), and # 20Z025008 (E: percentage of positive cells, and F: MFI).
Fig. 7, the mean concentrations of Bmk 1 and HFB10-1E1hG1 in WT mice versus time. Symbols: the mean of actual data points.
Fig. 8, the mean concentrations of Bmk 1 and HFB10-1E1 hG1 in hOX40-KI mice versus time. Symbols: the mean of actual data points.
Fig 9, the percentage of CD8⁺ T cells in CD45⁺ cells of tumor tissue.
Fig. 10, the percentage of Treg in CD4⁺ T cells of tumor tissue.
Fig. 11, the Ki67 expression in CD4⁺ and CD8⁺ T cells of tumor tissue.
Fig. 12, the PD-1 expression in CD4⁺ and CD8⁺ T cells of tumor tissue.
Fig. 13 shows the epitope of the anti-OX40 antibody HFB301001, where the epitope is mapped onto a 3D crystal structure model of OX40 and OX40L in complex.
Fig. 14A to 14B show the agonistic activity of the OX40 ligands measured with a cell-based bioluminescence assay. Fig. 14A shows the activity of individual OX40 ligands; Fig. 14B shows the activity of OX40 ligands in the presence of anti-OX40 antibody HFB301001, Benchmark 1 or Benchmark 2. The y-axis shows the relative luminescence (RLU).
Fig. 15A to 15B show the OX40 expression on CD4⁺ T cells after antibody treatment.

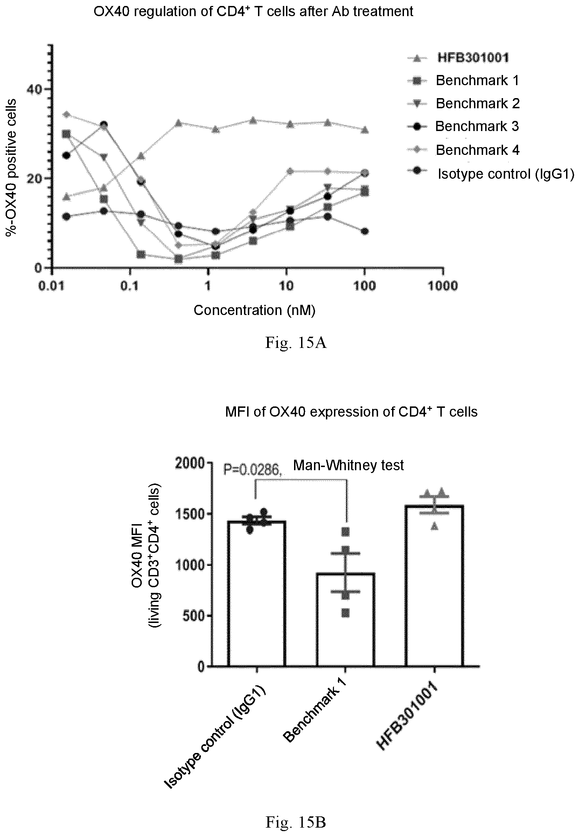

Fig. 15A shows the percentage of OX40 positive CD4⁺ T cells isolated from human PBMCs after the treatment with different concentrations of anti-OX40 antibody, as measured by flow cytometry. Fig. 15B shows the expression of OX40 on CD4⁺ T cells 24 hours after the third treatment with 10 mg/kg anti-OX40 antibody in the MC-38 murine colorectal cancer model in human OX40 knock-in mice, as measured by flow cytometry. The expression is measured as mean fluorescence intensity (MFI).

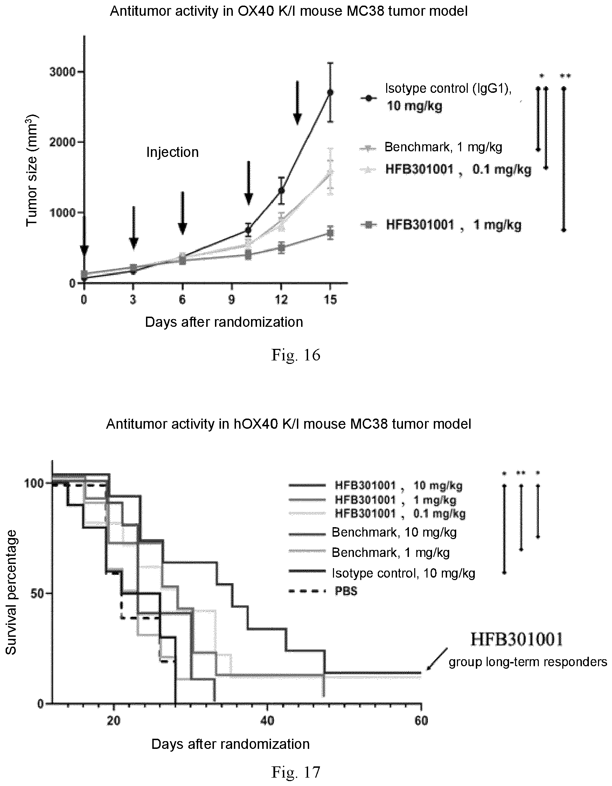

Fig. 16 shows the tumor size in the MC-38 tumor model in human OX40 (hOX40) knock-in (K/I) mice after the treatment with isotype control (10 mg/kg), anti-OX40 antibody benchmark 1 (1 mg/kg), or anti-OX40 antibody HFB301001 (1 mg/kg, 0.1 mg/kg). The treatment time points are indicated by arrows, error bars indicate standard error of the mean, and the significance is calculated by one-way ANOVA at the last time point (^{∗}: p-value < 0.05, ^{∗∗}: p-value < 0.01).
Fig. 17 shows the percent survival of human OX40 (hOX40) knock-in (K/I) mice with the MC-38 murine colorectal cancer model after the treatment with anti-OX40 antibody HFB301001 (10 mg/kg, 1 mg/kg, 0.1 mg/kg), anti-OX40 antibody benchmark 1 (10 mg/kg or 1 mg/kg), isotype control (10 mg/kg), or phosphate-buffered saline (PBS) treatment. The significance is calculated by the log-rank test (^{∗}: p-value < 0.05, ^{∗∗}: p-value < 0.01).

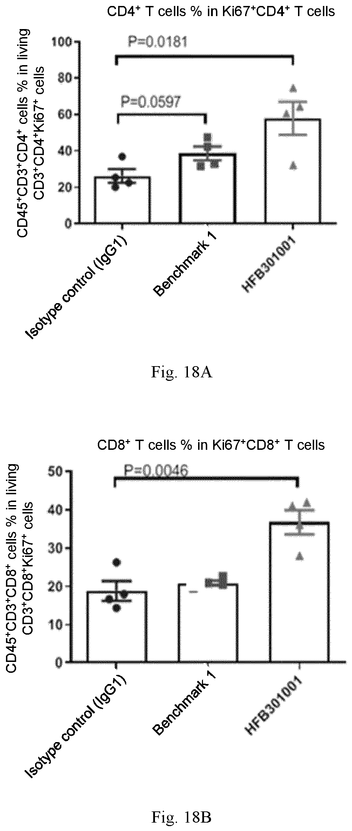

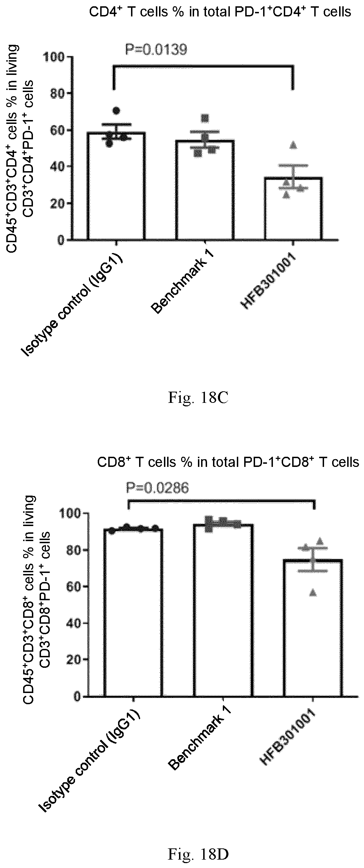

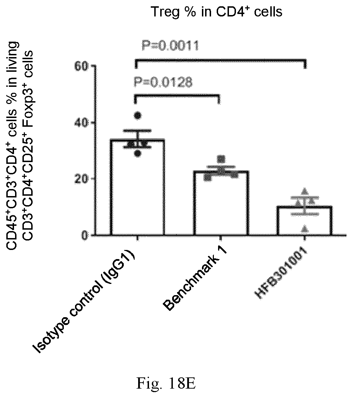

Fig. 18A to 18E show flow cytometry measurements of immune cell populations within the tumor microenvironment in the MC-38 murine colorectal cancer model in human OX40 (hOX40) knock-in (KI) mice after the treatment with control (IgG1), OX40 antibody benchmark 1 or anti-OX40 antibody HFB301001. Fig. 18A shows the percentage of CD3⁺CD4⁺ Ki67⁺ T cells in viable CD45⁺CD3⁺CD4⁺ cells. Fig. 18B shows the percentage of CD3⁺CD8⁺ Ki67⁺ T cells in viable CD45⁺CD3⁺CD8⁺ cells. Fig. 18C shows the percentage of CD3⁺CD4⁺ PD-1⁺ T cells in viable CD45⁺CD3⁺CD4⁺ cells. Fig. 18D shows the percentage of CD3⁺CD8⁺ PD-1⁺ T cells in viable CD45⁺CD3⁺CD8⁺ cells. Fig. 18E shows the percentage of CD3⁺CD4⁺CD25⁺Foxp3⁺ cells in viable CD45⁺CD3⁺CD4⁺ cells.

### Description of the Embodiments

In one aspect, the present invention provides an isolated anti-OX40 antibody or an antigen-binding fragment thereof, which includes: a heavy chain variable region having a heavy chain CDR1 structural domain as shown in SEQ ID NO: 1, a heavy chain CDR2 structural domain as shown in SEQ ID NO: 2, and a heavy chain CDR3 structural domain as shown in SEQ ID NO: 3; and a light chain variable region having a light chain CDR1 domain as shown in SEQ ID NO: 9, a light chain CDR2 domain as shown in SEQ ID NO: 10, and a light chain CDR3 domain as shown in SEQ ID NO: 11.

In one embodiment, the anti-OX40 antibody or antigen-binding fragment thereof as described herein includes a heavy chain variable region as shown in SEQ ID NO: 4, or a heavy chain variable region having a homology of at least 80%, 81%, 82%, 83%, 84%, 85%, 86%, 87%, 88%, 89%, 90%, 91%, 92%, 93%, 94%, 95%, 96%, 97%, 98%, 99% or 100% with SEQ ID NO: 4; and a light chain variable region as shown in SEQ ID NO: 12, or a light chain variable region having a homology of at least 80%, 81%, 82%, 83%, 84%, 85%, 86%, 87%, 88%, 89%, 90%, 91%, 92%, 93%, 94%, 95%, 96%, 97%, 98%, 99% or 100% with SEQ ID NO: 12.

In one embodiment, the anti-OX40 antibody or antigen-binding fragment thereof as described herein further includes a heavy chain constant region and a light chain constant region; preferably, the heavy chain constant region is a heavy chain constant region as shown in SEQ ID NO: 5, or a heavy chain constant region having a homology of at least 80%, 81%, 82%, 83%, 84%, 85%, 86%, 87%, 88%, 89%, 90%, 91%, 92%, 93%, 94%, 95%, 96%, 97%, 98%, 99% or 100% with SEQ ID NO: 5; and/or preferably, the light chain constant region is a light chain constant region as shown in SEQ ID NO: 13, or a light chain constant region having a homology of at least 80%, 81%, 82%, 83%, 84%, 85%, 86%, 87%, 88%, 89%, 90%, 91%, 92%, 93%, 94%, 95%, 96%, 97%, 98%, 99% or 100% with SEQ ID NO: 13.

In one embodiment, the anti-OX40 antibody or antigen-binding fragment thereof as described herein further includes a heavy chain signal peptide linked to the heavy chain variable region and/or a light chain signal peptide linked to the light chain variable region; preferably, the heavy chain signal peptide is a heavy chain signal peptide as shown in SEQ ID NO: 6, or a heavy chain signal peptide having a homology of at least 80%, 81%, 82%, 83%, 84%, 85%, 86%, 87%, 88%, 89%, 90%, 91%, 92%, 93%, 94%, 95%, 96%, 97%, 98%, 99% or 100% with SEQ ID NO: 6; and/or preferably, the light chain signal peptide is a light chain signal peptide as shown in SEQ ID NO: 14, or a light chain signal peptide having a homology of at least 80%, 81%, 82%, 83%, 84%, 85%, 86%, 87%, 88%, 89%, 90%, 91%, 92%, 93%, 94%, 95%, 96%, 97%, 98%, 99% or 100% with SEQ ID NO: 14.

In one embodiment, the anti-OX40 antibody or antigen-binding fragment thereof as described herein is an IgG antibody or an antigen-binding fragment thereof, and preferably an IgG1 antibody or an antigen-binding fragment thereof.

In one embodiment, the anti-OX40 antibody or antigen-binding fragment thereof as described herein is a monoclonal antibody or an antigen-binding fragment thereof.

In one embodiment, the antigen-binding fragment thereof as described herein is Fab, Fab', F(ab')2, Fv, scFv or sdAb.

In one aspect, the present invention provides an antibody-drug conjugate, which includes the anti-OX40 antibody or antigen-binding fragment thereof as described herein, and an additional therapeutic agent; preferably, the anti-OX40 antibody or antigen-binding fragment thereof is linked to the additional therapeutic agent via a linker.

In one aspect, the present invention provides a nucleic acid, encoding the anti-OX40 antibody or antigen-binding fragment thereof as described herein.

In one embodiment, the nucleic acid as described herein includes a heavy chain variable region nucleotide encoding sequence as shown in SEQ ID NO: 20, and/or a light chain variable region nucleotide encoding sequence as shown in SEQ ID NO: 28; and preferably, the nucleic acid further comprising a heavy chain constant region nucleotide encoding sequence as shown in SEQ ID NO: 21, and/or a light chain constant region nucleotide encoding sequence as shown in SEQ ID NO: 29.

In one aspect, the present invention provides an expression vector, which includes the nucleic acid as described herein.

In one aspect, the present invention provides a host cell, which includes the nucleic acid as described herein or the expression vector as described herein.

In one aspect, the present invention provides a method for preparing the anti-OX40 antibody or antigen-binding fragment thereof as described herein, which includes culturing the host cell as described herein under a condition suitable for expressing the antibody or antigen-binding fragment thereof, and recovering an expressed antibody or antigen-binding fragment thereof from a culture medium.

In one aspect, the present invention provides a pharmaceutical composition, which includes the anti-OX40 antibody or antigen-binding fragment thereof as described herein, or the antibody-drug conjugate as described herein, or the nucleic acid as described herein, or the expression vector as described herein; and a pharmaceutically acceptable carrier.

In one embodiment, the anti-OX40 antibody or antigen-binding fragment thereof as described herein, or the antibody-drug conjugate as described herein, or the pharmaceutical composition as described herein, is used in the treatment of cancer. In one embodiment, the cancer is selected from squamous cell carcinoma (for example, epithelial squamous cell carcinoma), lung cancer (comprising small cell lung cancer, non-small cell lung cancer, adenocarcinoma of the lung, and squamous cell carcinoma of the lung), peritoneal carcinoma, hepatocellular carcinoma, gastric cancer (comprising gastrointestinal cancer and gastrointestinal stromal cancer), pancreatic cancer, glioblastoma, cervical cancer, ovarian cancer, liver cancer, bladder cancer, urinary tract cancer, liver tumor, breast cancer, colon cancer, rectal cancer, colorectal cancer, endometrial or uterine cancer, salivary gland cancer, kidney cancer, prostate cancer, vulvar cancer, thyroid cancer, liver cancer, anal cancer, penile cancer, melanoma, superficial diffuse melanoma, malignant lentigo melanoma, acral melanoma, nodular melanoma, multiple myeloma and B-cell lymphoma, chronic lymphocytic leukemia (CLL), acute lymphoblastic leukemia (ALL), hairy cell leukemia, chronic myeloblastoid leukemia, and post-transplantation lymphoproliferative disorder (PTLD), abnormal vascular proliferation associated with keloids, edema (for example, associated with brain tumors), and Meigs syndrome, brain tumor and brain cancer, head and neck cancer, and associated metastases.

In one aspect, the present invention provides a method for the treatment of cancer, which includes: administering to a subject in need thereof a therapeutically effective amount of the anti-OX40 antibody or antigen-binding fragment thereof as described herein, or the antibody-drug conjugate as described herein, or the pharmaceutical composition as described herein, so as to treat the cancer. In one embodiment, the cancer is selected from squamous cell carcinoma (for example, epithelial squamous cell carcinoma), lung cancer (comprising small cell lung cancer, non-small cell lung cancer, adenocarcinoma of the lung, and squamous cell carcinoma of the lung), peritoneal carcinoma, hepatocellular carcinoma, gastric cancer (comprising gastrointestinal cancer and gastrointestinal stromal cancer), pancreatic cancer, glioblastoma, cervical cancer, ovarian cancer, liver cancer, bladder cancer, urinary tract cancer, liver tumor, breast cancer, colon cancer, rectal cancer, colorectal cancer, endometrial or uterine cancer, salivary gland cancer, kidney cancer, prostate cancer, vulvar cancer, thyroid cancer, liver cancer, anal cancer, penile cancer, melanoma, superficial diffuse melanoma, malignant lentigo melanoma, acral melanoma, nodular melanoma, multiple myeloma and B-cell lymphoma, chronic lymphocytic leukemia (CLL), acute lymphoblastic leukemia (ALL), hairy cell leukemia, chronic myeloblastoid leukemia, and post-transplantation lymphoproliferative disorder (PTLD), abnormal vascular proliferation associated with keloids, edema (for example, associated with brain tumors), and Meigs syndrome, brain tumor and brain cancer, head and neck cancer, and associated metastases.

In one aspect, the present invention provides a use of the anti-OX40 antibody or antigen-binding fragment thereof as described herein, or the antibody-drug conjugate as described herein, or the pharmaceutical composition as described herein in the preparation of a medicament for the treatment of cancer. In one embodiment, the cancer is selected from squamous cell carcinoma (for example, epithelial squamous cell carcinoma), lung cancer (comprising small cell lung cancer, non-small cell lung cancer, adenocarcinoma of the lung, and squamous cell carcinoma of the lung), peritoneal carcinoma, hepatocellular carcinoma, gastric cancer (comprising gastrointestinal cancer and gastrointestinal stromal cancer), pancreatic cancer, glioblastoma, cervical cancer, ovarian cancer, liver cancer, bladder cancer, urinary tract cancer, liver tumor, breast cancer, colon cancer, rectal cancer, colorectal cancer, endometrial or uterine cancer, salivary gland cancer, kidney cancer, prostate cancer, vulvar cancer, thyroid cancer, liver cancer, anal cancer, penile cancer, melanoma, superficial diffuse melanoma, malignant lentigo melanoma, acral melanoma, nodular melanoma, multiple myeloma and B-cell lymphoma, chronic lymphocytic leukemia (CLL), acute lymphoblastic leukemia (ALL), hairy cell leukemia, chronic myeloblastoid leukemia, and post-transplantation lymphoproliferative disorder (PTLD), abnormal vascular proliferation associated with keloids, edema (for example, associated with brain tumors), and Meigs syndrome, brain tumor and brain cancer, head and neck cancer, and associated metastases.

In one aspect, the present invention provides a use of the anti-OX40 antibody or antigen-binding fragment thereof as described herein, or the antibody-drug conjugate as described herein, or the pharmaceutical composition as described herein in one or more of the following: inhibiting Treg function (for example, inhibiting the suppressive function of Treg), killing cells expressing OX40 (for example, cells expressing a high level of OX40), enhancing an effector T cell function and/or enhancing a memory T cell function, reducing tumor immunity, enhancing T cell function and/or depleting OX40-expressing cells.

In one aspect, the present invention provides a use of the anti-OX40 antibody or antigen-binding fragment thereof as described herein, or the antibody-drug conjugate as described herein, or the pharmaceutical composition as described herein in the preparation of a medicament for one or more of the following: inhibiting Treg function (for example, inhibiting the suppressive function of Treg), killing cells expressing OX40 (for example, cells expressing a high level of OX40), enhancing an effector T cell function and/or enhancing a memory T cell function, reducing tumor immunity, enhancing T cell function and/or depleting OX40-expressing cells.

In one aspect, the present invention provides a pharmaceutical combination, which includes the anti-OX40 antibody or antigen-binding fragment thereof as described herein, or the antibody-drug conjugate as described herein, or the pharmaceutical composition as described herein; and one or more additional therapeutic agents.

In one aspect, the present invention provides a kit, which includes the anti-OX40 antibody or antigen-binding fragment thereof as described herein, or the antibody-drug conjugate as described herein, or the pharmaceutical composition as described herein, and preferably further comprising a drug delivery device.

Some embodiments of the present invention provide an agonistic antibody against OX-40. It resulted in no or less down-regulation of the OX-40 receptor as compared to other agonistic anti-OX-40 antibodies. This lack of receptor down-regulation or reduced receptor down-regulation may be due to the fact that the agonistic antibody provided by the present invention can recognize an epitope thereof. The agonistic antibody provided by the present invention may also have optimized binding kinetics, especially compared to other agonistic anti-OX-40 antibodies known in the art.

It is to be understood that one, some, or all of the features of various embodiments described herein may be combined to form further embodiments of the present invention. These and other aspects of the present invention are obvious to a person skilled in the art. These and other embodiments of the present invention will be further described in detail below.

Two polynucleotide or polypeptide sequences are said to be "identical" if the sequences of the nucleotides or amino acids in the two sequences are the same when described for the maximal enantiomeric alignment. Comparisons between two sequences are typically performed by comparing the sequences over a comparison window to identify and compare local regions of sequence similarity. A "comparison window" as used herein refers to a segment having at least about 20 (usually about 30 to about 75, or about 40 to about 50) contiguous positions; after the two sequences are optimally aligned, a sequence can be compared to a reference sequence having the same number of contiguous positions.

The optimal alignment of sequences for comparison can be performed using programs in the suite of bioinformatics software (Inc., Madison, WI) using default parameters. This program implements several alignment schemes described in the following references: Dayhoff, M.O., 1978, A model of evolutionary change in proteins-Matrices for detecting distant relationships. In Dayhoff, M.O. (ed.) Atlas of Protein Sequence and Structure, National Biomedical Research Foundation, Washington DC, Vol. 5, Supplement 3, pp. 345 to 358 pages; Hein J., 1990, Unified Approach to Alignment and Phylogenes, pp. 626-645, Methods in Enzymology, Vol. 183, Academic Press, Inc., San Diego, CA; Higgins, D.G. and Sharp, P.M., 1989, CABIOS 5: 151-153; Myers, E.W. and Muller W., 1988, CABIOS 4:11-17; Robinson, E.D., 1971, Comb. Theor. 11:105; Santou, N., Nes, M., 1987, Mol.Biol.Evol.4:406-425; Sneath, P.H.A. and Sokal, R.R., 1973, Numerical Taxonomy the Principles and Practice of Numerical Taxonomy, Freeman Press, San Francisco, CA; Wilbur, W.J. and Lipman, D.J., 1983, Proc. Natl. Acad. Sci. USA 80:726-730.

In some embodiments, the "percent sequence identity/homology" is determined by comparing two optimally aligned sequences over a window of comparison having at least 20 positions, in which the portion of the polynucleotide or polypeptide sequence in the comparison window may include 20% or less, typically 5% to 15% or 10% to 12% of additions or deletions (that is, gaps) as compared to the reference sequence (which does not contain additions or deletions) for the optimal alignment of the two sequences. The percentage can be calculated as follows: determining the number of positions where the same nucleic acid base or amino acid residue occurs in both sequences to yield the number of matching positions, dividing the number of matching positions by the total number of positions in the reference sequence (that is, the window size), and then multiplying the result by 100 to yield the percent sequence identity/homology.

Alternatively, a variant may also be substantially homologous to the native gene or a portion or complement thereof. These polynucleotide variants can hybridize to naturally occurring DNA sequences encoding native antibodies (or complementary sequences) under moderately stringent conditions.

Suitable "moderately stringent conditions" include: pre-washing in 5X SSC, 0.5% SDS, 1.0 mM EDTA, at pH 8.0; hybridizing overnight at 50°C to 65°C in 5X SSC; followed by washing twice at 65°C for 20 minutes, with each wash using 2X, 0.5X and 0.2X SSC containing 0.1% SDS.

As used herein, a "highly stringent condition" or "high stringency condition" refers to one or some of the following conditions: (1) adopting low ionic strength and high temperature for washing, for example, use 0.015M sodium chloride/0.0015M sodium citrate/0.1% sodium lauryl sulfate at 50°C; (2) using a denaturant during hybridization, such as formamide, for example, with a 0.1% bovine serum albumin/0.1% polysucrose/0.1% polyvinylpyrrolidone/50 mM sodium phosphate buffer at pH 6.5, and at 42°C, 750 mM sodium chloride, 75 mM sodium citrate in 50% (v/v) formamide; or (3) at 42°C, using 50% formamide, 5X SSC (0.75 M NaCl, 0.075 M sodium citrate), 50 mM sodium phosphate (pH 6.8), 0.1% sodium pyrophosphate, 5X Denhardt hybridization solution, sonicated salmon sperm DNA (50 µg/mL), 0.1% SDS and 10% dextran sulfate, and at 42°C, using 0.2X SSC (sodium chloride/sodium citrate) to wash and at 55°C, using 50% formamide to wash, followed by using a high stringency wash solution of 0.1X SSC containing EDTA to wash at 55°C. A person skilled in the art knows how to optionally adjust the temperature, ionic strength, etc., in order to accommodate factors such as probe length and the like.

A person of ordinary skill in the art understands that due to the degeneracy of the genetic code, there are many nucleotide sequences encoding polypeptides as described herein. Some of these polynucleotides have minimal homology to the nucleotide sequence of any native gene. However, the present invention specifically contemplates polynucleotides that vary due to differences in codon usage. Furthermore, alleles of genes including the polynucleotide sequences provided herein are within the scope of the present invention. An allele is an endogenous gene that changes due to one or more mutations, such as deletions, additions and/or substitutions of nucleotides. The resulting mRNAs and proteins may, but do not have to, have altered structure or function. An allele can be identified using certain standard techniques such as hybridization, amplification and/or database sequence comparison.

The polynucleotides of the present invention can be obtained using chemical synthesis, recombinant methods or PCR. Methods of chemical polynucleotide synthesis are well known in the art and need not be described in detail herein. A person skilled in the art can use the sequences provided herein and commercially available DNA synthesizers to generate the desired DNA sequences.

For the production of polynucleotides using recombinant methods, the polynucleotide comprising the desired sequence can be inserted into a suitable vector, and the vector can be further introduced into a suitable host cell for replication and amplification, as described herein. A polynucleotide can be inserted into a host cell by any means known in the art. Cells can be transformed by direct uptake, endocytosis, transfection, F-hybridization, or electroporation in order to introduce an exogenous polynucleotide. Once introduced, the exogenous polynucleotide can be maintained within the cell as a non-integrating vector (such as a plasmid) or integrated into the host cell genome. The polynucleotide so amplified can be isolated from the host cell by certain methods known in the art. Reference may be made to, for example, Sambrook et al., 1989.

Alternatively, PCR allows for the replication of DNA sequences.

RNA can be obtained using the isolated DNA in a suitable vector and inserting it into a suitable host cell; as the cell replicates and transcribes DNA into RNA, the RNA can then be isolated using certain method known to a person skilled in the art.

Suitable cloning and expression vectors can include various components such as promoters, enhancers, and other transcriptional regulatory sequences. The vector can also be constructed to allow subsequent cloning of the antibody variable domains into a different vector.

Suitable cloning vectors can be constructed according to standard techniques or can be selected from a large number of cloning vectors available in the art. The chosen cloning vector may vary depending on the host cell intended to be used. However, useful cloning vectors will generally have the ability to replicate themselves. They may have a single target for a particular restriction endonuclease and/or may carry genes for markers that can be used to select clones containing the vector. Suitable examples include plasmids and bacterial viruses, for example, pUC18, pUC19, Bluescript (for example, pBS SK+) and derivatives thereof, mp18, mp19, pBR322, pMB9, ColE1, pCR1, RP4, phage DNA, and shuttle vectors such as pSA3 and pAT28. These and many other cloning vectors are available from commercial suppliers such as BioRad, Strategene and Invitrogen.

Expression vectors are further provided. The expression vectors are typically certain replicable polynucleotide constructs, which contain a polynucleotide according to the present invention. It is implied that the expression vector must be replicable in the host cell, either as episomal or as an integral part of chromosomal DNA thereof. Suitable expression vectors include, but are not limited to, plasmids, viral vectors including adenoviruses, adeno-associated viruses, retroviruses, cosmids, and the expression vectors disclosed in PCT Publication No. WO 87/04462. Carrier components may typically include, but are not limited to, one or more of the following: a signal sequence; an origin of replication; one or more marker genes; suitable transcriptional control elements (such as promoters, enhancers and terminators). For expression (that is, translation), one or more transcriptional control elements are also typically required, such as ribosome binding sites, translation initiation sites and stop codons.

The vector containing the polynucleotide of interest and/or the polynucleotide can be introduced into the host cell by any of a number of suitable means. The means include electroporation, transfection with calcium chloride, rubidium chloride, calcium phosphate, DEAE-dextran or other substances; microprojectile bombardment; lipofection; and infection (for example, where the vector is an infectious agent, such as pox virus). The choice of introduction vector or polynucleotide generally depends on the characteristics of the host cell.

The antibodies of the present invention include human antibodies prepared, expressed, produced or isolated by recombinant methods, for example, antibodies expressed using recombinant expression vectors transfected into host cells (will be further described in Section II below), antibodies isolated from recombinant combinatorial human antibody libraries (will be further described in Section III below), antibodies isolated from human immunoglobulin gene transgenic animals (for example, mice) (see, for example, Taylor, L.D. et al. (1992) Nucl. Acids Res. 20: 6287-6295), or antibodies prepared, expressed, produced or isolated by any other method involving splicing of human immunoglobulin gene sequences into other DNA sequences. Such recombinant human antibodies have variable and constant regions derived from human germline immunoglobulin sequences (see Kabat, E.A., et al. (1991) Sequences of Proteins of Immunological Interest, Fifth Edition, U.S. Department of Health and Human Services, NIH Publication No. 91-3242).

Antibodies or antibody portions of the present invention can be prepared by recombinantly expressing immunoglobulin light chain genes and heavy chain genes in host cells. For recombinant expression of an antibody, a host cell is transfected with one or more recombinant expression vectors carrying a DNA fragment(s) encoding the immunoglobulin light and heavy chains of the antibody such that the light and heavy chains can be expressed in the host cell. The antibody is also preferably secreted into the medium in which the host cell is cultured, from which the antibody can be recovered. Standard recombinant DNA methodology can be used to obtain antibody heavy chain genes and antibody light chain genes, introduce these genes into recombinant expression vectors, and then introduce the vectors into host cells. Herein, the standard method can be, for example, the method described in Sambrook, Fritsch and Maniatis (eds.), Molecular Cloning; A Laboratory Manual, Second Edition, Cold Spring Harbor, N.Y., (1989), Ausubel, F.M. et al. (eds.) Current Protocols in Molecuar Biology, Greene Publishing Associates, (1989), and US Patent No. 4,816,397 to Boss et al.

Antibodies or antigen-binding fragments thereof can be produced recombinantly using suitable host cells. Nucleic acids encoding the antibodies or antigen-binding fragments thereof can be cloned into expression vectors, which can then be introduced into host cells such as E. coli cells, yeast cells, insect cells, simian COS cells, Chinese hamster ovary (CHO) cells, or myeloma cells, where the cells do not additionally produce immunoglobulins, so as to obtain antibody synthesis in recombinant host cells. Among the many cells well known in the art, the preferred host cells include CHO cells, human embryonic kidney (HEK) 293 cells, and Sp2.0 cells.

Antibody fragments can be produced by proteolytic or other degradation of full-length antibodies by recombinant methods or by chemical synthesis. Polypeptide fragments of antibodies (particularly shorter polypeptides of up to about 50 amino acids) can be conveniently made by chemical synthesis. Methods for the chemical synthesis of proteins and peptides are known in the art and are commercially available.

The antibodies or antigen-binding fragments thereof of the present invention can be affinity-matured. For example, affinity-matured antibodies can be prepared from procedures known in the art (Marks et al., 1992, Bio/Technology, 10: 779-783; Barbas et al., 1994, Proc Nat. Acad. Sci, USA 91: 3809-3813; Schier et al., 1995, Gene, 169: 147-155; Yelton et al., 1995, J. Immunol., 155: 1994-2004; Jackson et al, 1995, J. Immunol., 154(7): 3310-9; Hawkins et al., 1992, J. Mol. Biol., 226: 889-896; and WO2004/058184).

### Antibody variants

In some embodiments, the present invention includes the amino acid sequence variants of the antibodies provided herein. For example, it may be desirable to improve the binding affinity and/or other biological properties of the antibody. Amino acid sequence variants of the antibody can be prepared by introducing appropriate modifications into the nucleotide sequence encoding the antibody, or by peptide synthesis. Such modifications include, for example, deletions, and/or insertions and/or substitutions of residues within the amino acid sequence of the antibodies. Any combination of deletions, insertions, and substitutions can be made to obtain the final construct, as long as the final construct possesses the desired characteristics, for example, antigen binding.

In some embodiments, an antibody of the present invention may include one or more point mutations in its amino acid sequence. The point mutations are designed to improve the developability of the antibody. For example, Raybould et al., "Five computational developability guidelines for therapeutic antibody profiling," PNAS, Mar. 5, 2019, 116 (10) 4025-4030, describe a Therapeutic Antibody Profiling Tool (TAP): a computational tool for building homology models of downloadable variable domain sequences, testing them against five developability guidelines, and reporting potential sequence responsibilities and canonical forms. The author further provides TAP which is freely available at: opig.stats.ox.ac.uk/webapps/sabdab-sabpred/TAP.php. In addition to achieving the desired affinity to the antigen, there are many obstacles to the development of therapeutic monoclonal antibodies. These obstacles include inherent immunogenicity, chemical and conformational instability, self-association, high viscosity, multispecificity, poor expression, and the like. For example, high levels of hydrophobicity, particularly in highly variable complementarity determining regions (CDRs), have been repeatedly implicated in aggregation, viscosity and multispecificity. Asymmetry in the net charge of the heavy and light chain variable domains is also associated with self-association and viscosity at high concentrations. Positively and negatively charged patches in the CDRs are associated with high clearance rates and poor expression levels. Product heterogeneity (for example, through oxidation, isomerization, or glycosylation) is often caused by specific sequence motifs that are prone to post-translational or co-translational modifications. Computational tools are available to facilitate the identification of sequence responsibility. Warszawski also describes methods for optimizing antibody affinity and stability through automated design of variable light chain-heavy chain interfaces. Warszawski et al. (2019), Optimizing antibody affinity and stability by the automated design of the variable light-heavy chain interfaces, PLoS Comput Biol 15 (8): e1007207, https://doi.org/10.1371/journal.pcbi.1007207. Additional methods can be used to identify potential developability issues for candidate antibodies; in addition, in some preferred embodiments of the present invention, one or more point mutations can be introduced into the candidate antibody by conventional methods to solve such problems, thereby obtaining the optimized therapeutic antibodies of the present invention.

### a) Substitution, insertion, and deletion variants

In some embodiments, antibody variants with one or more amino acid substitutions are provided. Sites of interest for alternative mutagenesis include HVRs and FRs. Conservative substitutions are shown in Table A under the heading "preferred substitutions." More substantial changes are provided in Table A under the heading "exemplary substitutions" and are described further below with reference to the amino acid side chain classes thereof. Amino acid substitutions can be introduced into the antibody of interest and the product can be screened for the desired activity, for example retained/improved antigen binding, decreased immunogenicity, or improved ADCC or CDC.

**Table A**

| Initial residue | Exemplary substitution | Preferred substitution |
|---|---|---|
| Ala (A) | Val; Leu; Ilw | Val |
| Arg (R) | Lys; Gln; Asn | Lys |
| Asn (N) | Gln; His; Asp; Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; Nle | Leu |
| Leu (L) | Nle; Ile; Val; Mey; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Nle | Leu |

According to common side chain properties, amino acids can be grouped as follows:
(1) Hydrophobic: Nle, Met, Ala, Val, Leu, IIe;
(2) Neutral, hydrophilic: Cys, Ser, Thr, Asn, Gin;
(3) Acidic: Asp, Glu;
(4) Basic: His, Lys, Arg;
(5) Residues affecting chain orientation: Gly, Pro;
(6) Aromatic: Trp, Tyr, Phe.

A non-conservative substitution would entail substituting a member of one of these classes for another class.

One class of substitutional variants involves substituting one or more hypervariable region residues of a parent antibody (for example, a humanized or human antibody). In general, the resulting variant selected for further study will have certain biological properties altered (for example, improved) relative to the parent antibody (for example, increased affinity, decreased immunogenicity) and/or will substantially retain certain biological properties of the parent antibody. Exemplary substitutional variants are affinity matured antibodies, which can be conveniently generated, for example, using phage display-based affinity maturation techniques such as those described herein. Briefly, one or more HVR residues can be mutated, and variant antibodies can be presented on phage and screened for specific biological activity (for example, binding affinity).

Changes (for example, substitutions) can be made to the HVR, for example, to improve antibody affinity. HVR "hot spots," i.e., residues encoded by codons that undergo mutation with high frequency during the somatic maturation process (see, for example, Chowdhury, Methods Mol. Biol. 207: 179-196 (2008)), and/or residues that contact the antigen can have such changes, in which the resulting variant VH or VL is tested for binding affinity. Affinity maturation by secondary library construction and reselection has been described, for example, in Hoogenboom et al., Methods in Molecular Biology 178: 1-37 (eds. O'Brien et al., Human Press, Totowa, NJ, (2001)). In some embodiments of affinity maturation, diversity is introduced into variable genes selected for maturation by various methods (for example, error-prone PCR, strand shuffling, or oligonucleotide-directed mutagenesis). Next, a secondary library is created. The library is then screened to identify any antibody variants with the desired affinity. Another approach to introducing diversity involves an HVR-directed approach, in which several HVR residues (for example, 4-6 residues at a time) are randomized. HVR residues involved in antigen binding can be specifically identified, for example, using alanine scanning mutagenesis or modeling. In particular, CDR-H3 and CDR-L3 are often targeted.

In some embodiments, substitutions, insertions, or deletions may occur within one or more HVRs, as long as such changes do not substantially reduce the ability of the antibody to bind to the antigen. For example, conservative changes (e.g., conservative substitutions, as provided herein) can be made to the HVR that do not substantially reduce binding affinity. For example, such changes can be outside of antigen-contacting residues in the HVR. In some embodiments of the variant VH and VL sequences provided above, each HVR is unchanged or contains no more than 1, 2 or 3 amino acid substitutions.

One method that can be used to identify residues or regions of an antibody that can be targeted for mutagenesis is referred to as "alanine scanning mutagenesis," as described by Cunningham and Wells (1989) Science, 244: 1081-1085. In this method, residues or groups of target residues (for example, charged residues such as arg, asp, his, lys, and glu) are identified and neutralized or negatively charged with amino acids (for example, alanine acid or polyalanine) substitutions to determine whether the interaction of the antibody with the antigen is affected. Further substitutions can be introduced at amino acid positions that demonstrate functional sensitivity to the initial substitution. Alternatively or additionally, the crystal structure of the antigen-antibody complex can be used to identify contact points between the antibody and the antigen. As an alternative candidate(s), such contact residues and adjacent residues may be targeted or eliminated. Variants can be screened to determine whether they contain desired properties.

Amino acid sequence insertions include amino- and/or carboxy-terminal fusions ranging in length from 1 residue to polypeptides containing 100 or more residues, as well as intrasequence insertions of single or multiple amino acid residues. Examples of terminal insertions include antibodies with an N-terminal methionyl residue. Other insertional variants of antibody molecules include fusions of the N- or C-terminus of the antibody with an enzyme (for example, for ADEPT) or a polypeptide that prolongs the serum half-life of the antibody.

### b) Glycosylation variants

In some embodiments, the antibodies provided herein can be altered to increase or decrease the degree of antibody glycosylation. Addition or deletion of glycosylation sites to an antibody can be conveniently accomplished by altering the amino acid sequence such that one or more glycosylation sites are created or eliminated.

In the case of an antibody including an Fc region, the carbohydrate to which it is attached can be altered. Natural antibodies produced by mammalian cells typically contain branched, biantennary oligosaccharides that are typically N-linked to Asn297 of the CH2 domain of the Fc region, see, for example, Wright et al., TIBTECH 15: 26-32 (1997). Oligosaccharides can include various carbohydrates, for example, mannose, N-acetylglucosamine (GlcNAc), galactose, and sialic acid, as well as fucose attached to the GlcNAc in the "backbone" of the biantennary oligosaccharide structure. In some embodiments, the oligosaccharides in the antibodies of the invention can be modified to create antibody variants with certain improved properties.

In one embodiment, antibody variants are provided that have carbohydrate structures lacking fucose attached (directly or indirectly) to the Fc region. For example, the amount of fucose in such antibodies can be 1% to 80%, 1% to 65%, 5% to 65%, or 20% to 40%. The amount of fucose can be determined by calculating the average amount of fucose within the sugar chain at Asn297 relative to the sum of all sugar structures attached to Asn297 (e.g., complex, hybrid and high mannose structures), as measured by MALDI-TOF mass spectrometry, for example, as described in WO 2008/077546. Asn297 refers to the asparagine residue located at about position 297 in the Fc region (Eu numbering of Fe region residues). However, Asn297 can also be located about ±3 amino acids upstream or downstream of position 297, for example, between positions 294 and 300, due to minor sequence variations in the antibody. Such fucosylated variants may have improved ADCC function. Reference may be made, for example, to US Patent Publication Nos. US 2003/0157108 (Presta, L.), US 2004/0093621 (Kyowa Hakko Kogyo Co., Ltd). Examples of publications involving "defucosylated" or "fucose-deficient" antibody variants include: US 2003/0157108; WO 2000/61739; WO 2001/29246;
US 2003/0115614; US 2002/0164328; US 2004/0093621; US 2004/0132140; US 2004/0110704; US 2004/0110282; US 2004/0109865; WO 2003/085119; WO 2003/084570; WO 2005/035586; TO 2005/035778; TO 2005/053742;TO 2002/031140; Okazaki et al, J. Mol. Biol. 336: 1239-1249 (2004); Yamane-Ohnuki et al, Biotech. Bioeng. 87: 614 (2004). Examples of cell lines capable of producing defucosylated antibodies include Lec I3CHO cells deficient in protein fucosylation (Ripka et al., Arch. Biochem. Biophys. 249: 533-545 (1986); U.S. Patent Application No. US 2003/0157108A1, Presta, L; and WO 2004/056312A1, Adams et al.), and knockout cell lines, such as α-1,6-fucosyltransferase gene FUT8 knockout CHO cells (see, for example, Yamane-Ohnuki et al., Biotech. Bioeng. 87: 614 (2004); Kanda, Y. et al., Biotechnol. Bioeng., 94(4): 680-688 (2006); and WO 2003/085107).

Antibody variants are further provided that have bipartite oligosaccharides, for example, in which the biantennary oligosaccharide attached to the Fc region of the antibody is bisected by GlcNAc. Such antibody variants may have reduced fucosylation and/or improved ADCC function. Examples of such antibody variants are described, for example, in WO 2003/011878 (Jean-Mairet et al.); US Patent No. 6,602,684 (Umana et al.); and US 2005/0123546 (Umana et al.). Antibody variants having at least one galactose residue in the oligosaccharide attached to the Fc region are also provided. Such antibody variants may have improved CDC function. Such antibody variants are described, for example, in WO 1997/30087 (Patel et al.); WO 1998/58964 (Raju, S.); and WO 1999/22764 (Raju, S.).

### c) Fc region variants

In some embodiments, one or more amino acid modifications can be introduced into the Fc region of the antibodies provided herein, thereby generating Fc region variants. Fc region variants may comprise human Fc region sequences (for example, human IgGl, IgG2, IgG3 or IgG4 Fc regions) comprising amino acid modifications (for example, substitutions) at one or more amino acid positions.

In some embodiments, the present invention includes antibody variants possessing some but not all effector functions. The effector functions make it a desirable candidate for applications for the applications described later. The in vivo half-life of the antibody is important; certain effector functions such as complement and ADCC are unnecessary or detrimental. In vitro and/or in vivo cytotoxicity assays can be performed to confirm the reduction/depletion of CDC and/or ADCC activity. For example, Fc receptor (FcR) binding assays can be performed to ensure that the antibody lacks FcyR binding (and thus likely lacks ADCC activity), but retains FcRn binding ability. NK cells, the primary cells that mediate ADCC, express FcγRIII only, whereas monocytes express FcyRI, FcyRII and FcyRIII. FcR expression on hematopoietic cells is summarized in Table 3 on page 464 of Ravetch and Kinet, Annu. Rev. Immunol. 9: 457-492 (1991). Non-limiting examples of in vitro assays for assessing ADCC activity of molecules of interest are described in U.S. Patent No. 5,500,362 (see, e.g., Hellstrom, I. et al., Proc. Nat'l Acad. Sci USA 83: 7059-7063 (1986) and Hellstrom, I et al., Proc. Nat'l Acad. Sci. USA 82: 1499-1502 (1985)); 5,821,337 (see Bruggemann, M. et al., J. Exp. Med. 166: 1351-1361 (1987)). Alternatively, non-radioactive assays (see, for example, the ACT I^{™} non-radioactive cytotoxicity assay for flow cytometry (Cell Technology, Inc. Mountain View, CA) and the CytoTox96 non-radioactive cytotoxicity assay (Promega, Madison, WI)) can be used. Useful effector cells for such assays include peripheral blood mononuclear cells (PBMC) and natural killer (NK) cells. Alternatively or additionally, the ADCC activity of the molecule of interest can be assessed in vivo, for example, in animal models, such as those disclosed in Clynes et al., Proc Nat'l Acad Sci USA 95: 652-656 (1998). Clq binding assays can also be performed to confirm that the antibody is unable to bind Clq and therefore lacks CDC activity. See, for example, Clq and C3c binding ELISA in WO 2006/029879 and WO 2005/100402. To assess complement activation, CDC assays can be performed (see, for example, Gazzano-Santoro et al., J. Immunol. Methods 202: 163 (1996); Cragg, M. S. et al., Blood 101: 1045-1052 (2003); and Cragg, M. S. and M. J. Glennie, Blood 103: 2738-2743 (2004)). FcRn binding and in vivo clearance/half-life assays can also be performed using methods known in the art (see, for example, Petkova, S.B. et al., Int'l. Immunol. 18(12): 1759-1769 (2006)).

Antibodies with reduced effector function include those with substitution of one or more of Fc region residues 238, 265, 269, 270, 297, 327 and 329 (US Patent No. 6,737,056). Such Fc mutants include Fc mutants with substitutions at two or more of amino acid positions 265, 269, 270, 297 and 327, including the so-called "DANA" Fc mutant in which residues 265 and 297 are each replaced by alanine (US Patent No. 7,332,581).

Certain antibody variants with improved or reduced binding to FcRs are described (see, for example, US Patent No. 6,737,056; WO 2004/056312, and Shields et al., J. Biol. Chem. 9(2):6591-6604 (2001)).

In some embodiments, the antibody variant can include an Fc region with one or more amino acid substitutions that improve ADCC, such as substitutions at positions 298, 333, and/or 334 (EU numbering of residues) of the Fc region.

In some embodiments, changes are made to the Fc region that result in altered (i.e., improved or reduced) C1q binding and/or complement-dependent cytotoxicity (CDC), for example, as described in US Patent No. 6,194,551, WO 99/51642, and Idusogie et al., J. Immunol. 164: 4178-4184 (2000).

Antibodies with extended half-life and improved binding to the neonatal Fc receptor (FcRn) are described in US 2005/0014934A1 (Hinton et al.). The neonatal Fc receptor (FcRn) is responsible for the transfer of maternal IgG to the fetus (Guyer et al., J. Immunol. 117: 587 (1976) and Kim et al. J. Immunol. 24: 249 (1994)). Those antibodies can include an Fc region with one or more substitutions therein that improve binding of the Fc region to FcRn. Such Fc variants include those at Fc region residues 238, 256, 265, 272, 286, 303, 305, 307, 311, 312, 317, 340, 356, 360, 362, 376, 378, 380, 382, one or more of 413, 424 or 434 with substitution, for example substitution of Fc region residue 434 (US Pat. No. 7,371,826).

See also Duncan and Winter, Nature 322: 738-40 (1988); US Patent No. 5,648,260; US Patent No. 5,624,821; and WO 94/29351, which focus on other examples of Fc region variants.

### d) Cysteine-engineered antibody variants

In some embodiments, it may be desirable to create cysteine-engineered antibodies, e.g., "thioMAbs," where one or more residues of the antibody are replaced with cysteine residues. In certain specific embodiments, the substituted residues are present at accessible sites of the antibody. By replacing those residues with cysteine, the reactive thiol group is thus positioned at an accessible site of the antibody; in addition, this can be used to conjugate antibodies to other moieties, such as drug moieties or linker-drug moieties, to create immunoconjugates, as described further herein. In some embodiments, cysteine may be substituted for any one or more of the following residues: V205 of the light chain (Kabat numbering); A118 of the heavy chain (EU numbering); and those of the Fc region of the heavy chain S400 (EU numbering method). Cysteine-engineered antibodies can be generated as described, for example, in US Pat. No. 7,521,541.

### e) Antibody derivatives

In some embodiments, the antibodies provided herein can be further modified to contain additional non-proteinaceous moieties known in the art and readily available. Suitable moieties for antibody derivatization include, but are not limited to, water-soluble polymers. Non-limiting examples of water-soluble polymers include but are not limited to polyethylene glycol (PEG), ethylene glycol/propylene glycol copolymer, carboxymethyl cellulose, dextran, polyvinyl alcohol, polyvinylpyrrolidone, poly-1,3-dioxolane, poly-1,3 , 6-trioxane, ethylene/maleic anhydride copolymer, polyamino acid (homopolymer or random copolymer), dextran or poly(n-vinylpyrrolidone) polyethylene glycol, propylene glycol homopolymers, propylene oxide/ethylene oxide copolymers, polyoxyethylated polyols (for example, glycerol), polyvinyl alcohol, and mixtures thereof. Polyethylene glycol propionaldehyde may have advantages in production due to its stability in water. The polymers can be of any molecular weight and can be branched or unbranched. The number of polymers attached to the antibody can vary, and if more than one polymer is attached, they can be the same or different molecules. In general, the number and/or type of polymer used for derivatization can be determined based on the following considerations: this may include, but is not limited to, the specific property or function that the antibody is to improve, whether the antibody derivative will be used for therapy under specified conditions, and the like.

In another embodiment, conjugates of antibodies and non-proteinaceous moieties that can be selectively heated by exposure to radiation are provided. In one embodiment, the non-proteinaceous moiety is a carbon nanotube (Kam et al., Proc. Nat'l. Acad. Sci. USA 102: 11600-11605 (2005)). The radiation can be of any wavelength and includes, but is not limited to, the wavelengths that are not harmful to ordinary cells, but heat the non-proteinaceous moiety to a temperature at which cells in the vicinity of the antibody-non-proteinaceous moiety are killed.

### Assays

The anti-OX40 antibodies provided herein can be identified, screened, or characterized for their physical/chemical properties and/or biological activity by a variety of assays known in the art.

### 1. Binding assays and other assays

In one aspect, the antibodies of the invention are tested for their antigen-binding activity, for example, by known methods such as ELISA, Western blotting, and the like. OX40 binding can be determined using methods known in the art; exemplary methods are disclosed herein. In one embodiment, binding is measured using a radioimmunoassay. An exemplary radioimmunoassay is illustrated. The OX40 antibody is iodinated and a competition reaction mixture is prepared containing a fixed concentration of iodinated antibody and decreasing concentrations of serial dilutions of unlabeled OX40 antibody. OX40-expressing cells (for example, BT474 cells stably transfected with human OX40) are added to the reaction mixture. After incubation, cells are washed to separate free iodinated OX40 antibody from OX40 antibody bound to cells. The level of bound OX40 iodide antibody can be determined, for example, by counting the radioactivity associated with the cells, and binding affinity is determined using standard methods. In another embodiment, the ability of an OX40 antibody to bind surface-expressed OX40 (for example, on a subset of T cells) is assessed using flow cytometry. Peripheral leukocytes (for example, from human, cynomolgus, rat or mouse) are obtained and cells are blocked with serum. Labeled OX40 antibodies can be added in serial dilutions and T cells are also stained to identify T cell subsets (using methods known in the art). Following sample incubation and washing, cells are sorted using a flow cytometer and data are analyzed using methods well known in the art. In another embodiment, surface plasmon resonance can be used to analyze OX40 binding. An exemplary surface plasmon resonance method is illustrated.

In another aspect, competition assays can be used to identify antibodies that compete with any of the anti-OX40 antibodies disclosed herein for binding to OX40. In certain embodiments, such competing antibodies bind the same epitope (for example, a linear or conformational epitope) that any of the anti-OX40 antibodies disclosed herein can bind. A detailed exemplary method for localizing epitopes bound by antibodies can be found in Morris (1996) "Epitope Mapping Protocols," Methods in Molecular Biology vol. 66 (Humana Press, Totowa, NJ). A competition assay is illustrated.

In an exemplary competition assay, a first labeled antibody (which binds OX, for example, mab 1A7.gr.1, mab 3C8.gr5) and a second unlabeled antibody (which is tested to compete with the first antibody's OX40 binding capacity) can be incubated with immobilized OX40 in solution. The secondary antibody can be present in the hybridoma supernatant. As a control, immobilized OX40 is incubated in a solution containing the first labeled antibody but not the second unlabeled antibody. After incubation under conditions that allow binding of the primary antibody to OX40, excess unbound antibody is removed and the amount of label bound to immobilized OX40 is measured. If the amount of label associated with immobilized OX40 is substantially reduced in the test sample compared to the control sample, this indicates that the secondary antibody competes with the primary antibody for binding to OX40, see Harlow and Lane (1988) Antibodies: A Laboratory Manual ch. 14 (Cold Spring Harbor Laboratory, Cold Spring Harbor, NY).

### 2. Activity Assays

In one aspect, assays for identifying biologically active anti-OX40 antibodies are provided. The biological activity herein can include, for example, binding OX40 (e.g. binding to human and/or cynomolgus monkey OX40), increasing OX40-mediated signal transduction (e.g. increasing NFkB-mediated transcription), depleting cells expressing human OX40 (e.g. T cells), depleting human OX40-expressing cells by ADCC and/or phagocytosis, enhancing T effector cell function (e.g., CD4+ effector T cells) (e.g., by increasing effector T cell proliferation and/or increasing effector T cell cytokine production (e.g., interferon γ)), enhancing memory T cell function (e.g., CD4+ memory T cells) (e.g., by increasing memory T cell proliferation and/or increasing memory T cell cytokine production (e.g. interferon γ)), inhibiting Treg suppression that regulates T cell function (e.g., by reducing effector T cell function (e.g., CD4+ effector T cell function)), and binding to human effector cells. Antibodies having such biological activities in vivo and/or in vitro are also provided.

In some embodiments, antibodies of the invention are tested for such biological activities.

T cell costimulation can be determined using methods known in the art, and exemplary methods are disclosed herein. For example, T cells (e.g., memory or effector T cells) can be obtained from peripheral leukocytes (e.g., isolated from human whole blood using Ficoll gradient centrifugation). Memory T cells (e.g., CD4+ memory T cells) or effector T cells (e.g., CD4+ Teff cells) can be isolated from PBMCs using methods known in the art. For example, Miltenyi CD4+ Memory T Cell Isolation Kit or Miltenyi Naive CD4+ T Cell Isolation Kit can be used. Isolated T cells are cultured in the presence of antigen presenting cells (e.g., irradiated CD32- and CD80-expressing L cells) and activated by the addition of anti-CD3 antibodies in the presence or absence of OX40 agonistic antibodies. The effect of agonistic OX40 antibodies on T cell proliferation can be measured using methods well known in the art. For example, the Cell Titer Glo kit (Promega) can be used and the results read on a multi-label reader (Perkin Elmer). The effect of agonistic OX40 antibodies on T cell function can be determined by analyzing cytokine production by T cells. In one embodiment, interferon gamma production by CD4+ T cells is determined, e.g., by measuring interferon gamma in cell culture supernatants. Methods for measuring interferon gamma are well known in the art.

Treg cell function can be assayed using methods known in the art, and exemplary methods are disclosed herein. In one example, the ability of Treg to suppress proliferation of effector T cells is assayed. T cells (e.g., isolation of memory T cells or naive T cells) are isolated from human whole blood using methods known in the art. Purified CD4+ naive T cells are labeled (e.g., with CFSE), and purified Treg cells are labeled with different reagents. Irradiated antigen-presenting cells (e.g., L cells expressing CD32 and CD80) are co-cultured with labeled purified naive CD4+ T cells and purified Treg. Co-cultures can be activated with anti-CD3 antibody and tested in the presence or absence of agonistic OMO antibody. After an appropriate time (e.g., 6 days of co-culture), FACS analysis is then used to track the level of CD4+ naive T cell proliferation by dye dilution in reduced marker staining (e.g., reduced CFSE marker staining).

OX40 signaling can be assayed using methods well known in the art, and exemplary methods are disclosed herein. In one embodiment, transgenic cells expressing human OX40 and a reporter gene including the NFkB promoter fused to a reporter gene (e.g., β luciferase) are generated. The addition of OX40 agonistic antibodies to cells can result in increased NFkB transcription, which can be detected using an assay against a reporter gene.

Phagocytosis can be measured, for example, by using monocyte-derived macrophages or U937 cells, a human histiocytic lymphoma cell line with the morphology and characteristics of mature macrophages. OX40-expressing cells can be added to monocyte-derived macrophages or U937 cells in the presence or absence of anti-OX40 agonistic antibodies. The cells are cultured for an appropriate period of time, the percentage of cells double stained for markers of 1) macrophages or U937 cells and 2) OX40-expressing cells are determined, and then the percent phagocytosis can be determined by dividing the foregoing by the total number of cells showing markers of cells expressing OX40 (for example, GFP). Analysis can be performed by flow cytometry. In another embodiment, the analysis can be performed by fluorescence microscopy analysis.

ADCC can be determined, for example, using methods well known in the art. Exemplary methods are described in the Definitions section. In some embodiments, the OX40 levels on OX40-expressing cells for testing in an ADCC assay are characterized. Cells are stained with a detectably labeled anti-OX40 antibody (e.g., PE-labeled), fluorescence levels are determined using flow cytometry, and results are presented as median fluorescence intensity (MFI). In another embodiment, ADCC can be analyzed by the CellTiter Glo assay kit and cell viability/cytotoxicity can be determined by chemiluminescence.

Response of various antibodies to FcyRIA, FcyRIIA, FcyRIIB, and two allotypes of FcyRIIIA (F158 and V158) can be measured with the binding affinity in ELISA-based ligand binding assays using the corresponding recombinant Fcγ receptors. The purified human Fcγ receptor is expressed as a fusion protein containing the ectodomain of the receptor γ chain linked to a C-terminal Gly/6xHis/glutathione S-transferase (GST) polypeptide tag. The binding affinities of the antibodies to those human Fcγ receptors can be determined as follows. For low affinity receptors, that is, FcγRIIA (CD32A), FcyRIIB (CD32B), and two allotypes of FcγRIIIA (CD16), F-158 and V-158, it can be obtained by cross-linking (at an approximate molar ratio of 1:3 antibody:cross-linking with F(ab')2 fragment of goat anti-human kappa chain (ICN Biomedical; Irvine, CA) ab') 2) as multimers to test the antibodies. Plates can be coated with anti-GST antibody (Genentech) and blocked with bovine serum albumin (BSA). After washing with phosphate-buffered saline (PBS) containing 0.05% Tween-20 and an ELx405^{™} plate washer device (Biotek Instruments; Winooski, VT), Fcγ receptors are then added to the plate at 25 ng/well and incubated at room temperature 1 hour. After washing the plate, serial dilutions of the test antibodies can be added as multimeric complexes and the plate is then incubated for 2 hours at room temperature. After washing the plate to remove the unbound antibody, the F(ab')2 fragment of goat anti-human F(ab')2 conjugated with horseradish peroxidase (HRP) (Jackson ImmunoResearch Laboratories; West Grove, PA) can be used to detect the antibodies bound to the Fcγ gamma receptors. The substrate, tetramethylbenzidine (TMB) (Kirkegaard and Perry Laboratories; Gaithersburg, MD), is then added. Depending on the Fcγ receptor tested, the plate can be incubated at room temperature for 5 to 20 minutes to allow color development. Reactions are then stopped with 1 M H₃PO₄ and absorbance at 450 nm is measured with a microplate reader (SpectraMax^{®} 190, Molecular Devices; Sunnyvale, CA). Dose-response binding curves are then generated by plotting mean absorbance values from duplicate antibody dilutions against antibody concentration. The value of the effective antibody concentration at which 50% of the maximal response from binding to Fcγ receptors detected (EC50) can be determined after fitting the binding curve with a four-parameter equation using SoftMax 190 (Molecular Devices).

To select for antibodies that induce cell death, loss of membrane integrity as demonstrated by uptake of, for example, propidium iodide (PI), trypan blue or 7AAD can be assessed relative to the control(s). PI uptake assays can be performed in the absence of complement and immune effector cells. OX40-expressing cells are incubated in medium alone or in medium containing the appropriate monoclonal antibody at a concentration of, for example, about 10 µg/ml. The cells are incubated for a certain period of time (for example, 1 or 3 days). After each treatment, cells are then washed and aliquoted. In some embodiments, cells are aliquoted into 35 mm strainer-capped 12x75 tubes (1 ml per tube, 3 tubes per treatment group) to remove cell clumps. PI (10 µg/ml) is then added to the tube. Samples can be analyzed using a FACSCAN^{™} flow cytometer and FACSCONVERT^{™} CellQuest software (Becton Dickinson).

Cells for use in any of the above in vitro assays include the cells or cell lines that naturally express OX40 or can be engineered to express OX40. Such cells include activated T cells that naturally express OX40, Treg cells and activated memory T cells. Such cells also include cell lines that express OX40 and cell lines that do not normally express OX40 but have been transfected with a nucleic acid encoding OX40. Exemplary cell lines provided herein for use with any of the above in vitro assays include transgenic BT474 cells (a human breast cancer cell line) expressing human OX40.

It is understood that any of the above assays can be performed using the immunoconjugates of the present invention in place of or in addition to anti-OX40 antibodies.

It is understood that any of the above assays can be performed using anti-OX40 antibodies and other therapeutic agents.

### Formulations and uses thereof

The antibodies or antigen-binding fragments thereof of the present invention can be formulated into a pharmaceutical composition. The pharmaceutical composition may further include certain pharmaceutically acceptable carriers, excipients and/or stabilizers (Remington: The Science and practice of Pharmacy, 20th Edition, 2000, Lippincott Williams and Wilkins, Ed. K.E. Hoover) in the form of for a freezing-dry formula or an aqueous solution. Acceptable carriers, excipients or stabilizers are non-toxic to recipients at dosages and concentrations, and may contain buffers such as phosphoric acid, citric acid, and other organic acids; antioxidants, including ascorbic acid and methionine; preservatives (such as octadecyldimethylbenzylammonium chloride; hexahydroxyquaternium chloride; algaecide; bensonin chloride; phenolic, butanol or benzyl alcohol; alkylparabens, such as methylparaben or propylparaben; catechol; resorcinol; cyclohexanol; 3-pentanol; and m-cresol; low molecular weight (less than about 10 residues) polypeptides; proteins, such as serum albumin, gelatin, or immunoglobulins; hydrophilic polymers, such as polyvinylpyrrolidone; amino acids, such as glycine, glutamine, asparagine, histidine, arginine, or lysine; monosaccharides, disaccharides and other sugars, including glucose, mannose or dextran; chelating agents, such as EDTA; sugars such as sucrose, mannitol, trehalose or sorbitol; salt-forming counter ions, such as sodium; metal complexes (for example, Zn-protein complexes); and/or non-ionic surfactants such as TWEEN^{™}, PLURONICS^{™} or polyethylene glycol (PEG). Pharmaceutically acceptable excipients will be further described herein.

The antibodies or antigen-binding fragments thereof of the present invention can be used for various therapeutic or diagnostic purposes. For example, the antibodies or antigen-binding fragments thereof of the invention can be used as affinity purification agents (e.g., for in vitro purification) and as diagnostic agents (e.g., for detection of expression in specific cells, tissues, or serum).

Exemplary therapeutic uses of the antibodies or antigen-binding fragments thereof of the invention include the treatment of cancer. The antibodies or antigen-binding fragments thereof of the present invention can also be used for preventive treatment.

For therapeutic applications, the antibodies or antigen-binding fragments thereof of the present invention can be administered to mammals, especially humans, by conventional techniques; such techniques can be intravenous (as a bolus or by continuous infusion over time), intramuscular, intraperitoneal, intracerebral, subcutaneous, intraarticular, intrasynovial, intrathecal, oral, topical, or by inhalation. The antibodies or antigen-binding fragments thereof of the present invention may also be administered by intratumoral, peritumoral, intralesional, or perilesional routes, as appropriate.

In some embodiments, the antibodies or antigen-binding fragments thereof of the present invention are administered subcutaneously. In some embodiments, the antibodies or antigen-binding fragments thereof of the invention are administered intravenously.

The pharmaceutical composition can be administered to a subject in need thereof at a frequency that may vary with the severity of the disease. In the case of preventive treatment, the frequency may vary depending on the susceptibility or predisposition to the subject's disease.

The composition may be administered to a patient in need thereof as a bolus injection or by continuous infusion. For example, bolus administration of the antibody presented as a Fab fragment can be administered in amounts from 0.0025 to 100 mg/kg body weight, 0.025 to 0.25 mg/kg, 0.010 to 0.10 mg/kg, or 0.10 to 0.50 mg/kg. For continuous infusion, the antibody presented as a Fab fragment may be 0.001 to 100 mg/kg body weight/min, 0.0125 to 1.25 mg/kg/min, 0.010 to 0.75 mg/kg/min, 0.010 to 1.0 mg/kg/min or an amount of 0.10 to 0.50 mg/kg/min and administered for 1 to 24 hours, 1 to 12 hours, 2 to 12 hours, 6 to 12 hours, 2 to 8 hours, or 1 to 2 hours.

For administration of antibodies presented as full-length antibodies (with intact constant regions), dosages may be from about 1 mg/kg to about 10 mg/kg, from about 2 mg/kg to about 10 mg/kg, from about 3 mg/kg to about 10 mg/kg, from about 4 mg/kg to about 10 mg/kg, from about 5 mg/kg to about 10 mg/kg, from about 1 mg/kg to about 20 mg/kg, from about 2 mg/kg to about 20 mg/kg, from about 3 mg/kg to about 20 mg/kg, from about 4 mg/kg to about 20 mg/kg, from about 5 mg/kg to about 20 mg/kg, about 1 mg/kg or more, about 2 mg/kg or more, about 3 mg /kg or more, about 4 mg/kg or more, about 5 mg/kg or more, about 6 mg/kg or more, about 7 mg/kg or more, about 8 mg/kg or more, about 9 mg/kg or more, about 10 mg/kg or more, about 11 mg/kg or more, about 12 mg/kg or more, about 13 mg/kg or more, about 14 mg/kg or more, about 15 mg/kg or more, about 16 mg/kg or more, about 17 mg/kg or more, about 19 mg/kg or more, or about 20 mg/kg or more. The frequency of administration may depend on the severity of the condition. The frequency may vary from three times a week to once every two or three weeks.

Alternatively, the composition can be administered to a patient via subcutaneous injection. For example, a dose of 1 to 100 mg of anti-OX40 antibody may be administered via subcutaneous or intravenous injection to a patient at a frequency of twice a week, once a week, once every two weeks, once every three weeks, once every four weeks, once every five weeks, once every six weeks, once every seven weeks, once every eight weeks, once every nine weeks, once every ten weeks, twice a month, once a month, once every two months, or once every three months.

In some embodiments, the half-life of an anti-OX40 antibody in humans can be about 5 days, about 6 days, about 7 days, about 8 days, about 9 days, about 10 days, about 11 days, about 12 days, about 13 days, about 14 days, about 15 days, about 16 days, about 17 days, about 18 days, about 19 days, about 20 days, about 21 days, about 22 days, about 23 days, about 24 days, about 25 days, about 26 days, about 27 days, about 28 days, about 29 days, about 30 days, from about 5 days to about 40 days, from about 5 days to about 35 days, from about 5 days to about 30 days, from about 5 days to about 25 days, from about 10 days to about 40 days, from about 10 days to about 35 days, from about 10 days to about 30 days, from about 10 days to about 25 days, from about 15 days to about 40 days , from about 15 days to about 35 days, from about 15 days to about 30 days, or from about 15 days to about 25 days.

In some embodiments, the pharmaceutical composition can be administered subcutaneously or intravenously every 2 to 6 weeks at the following doses: from about 0.1 mg/kg to about 10 mg/kg, from about 0.5 mg/kg to about 10 mg/kg, from about 1 mg/kg to about 10 mg/kg, from about 1.5 mg/kg to about 10 mg/kg, from about 2 mg /kg to about 10 mg/kg, from about 0.1 mg/kg to about 8 mg/kg, from about 0.5 mg/kg to about 8 mg/kg, from about 1 mg/kg to about 8 mg/kg, from about 1.5 mg/kg to about 8 mg/kg, from about 2 mg/kg to about 8 mg/kg, from about 0.1 mg/kg to about 5 mg/kg, from about 0.5 mg/kg to about 5 mg/kg, from about 1 mg/kg to about 5 mg/kg , from about 1.5 mg/kg to about 5 mg/kg, from about 2 mg/kg to about 5 mg/kg, about 0.5 mg/kg, about 1.0 mg/kg, about 1.5 mg/kg, about 2.0 mg/kg, about 2.5 mg/kg, about 3.0 mg/kg, about 3.5 mg/kg, about 4.0 mg/kg, about 4.5 mg/kg, about 5.0 mg/kg, about 5.5 mg/kg, about 6.0 mg/kg, about 6.5 mg/kg, about 7.0 mg/kg, about 7.5 mg/kg, about 8.0 mg/kg, about 8.5 mg/kg, about 9.0 mg/kg, about 9.5 mg/kg, or about 10.0 mg/kg.

In some embodiments, the pharmaceutical composition is administered subcutaneously or intravenously at a dose of about 2.0 mg/kg every 2 to 6 weeks. In some embodiments, the pharmaceutical composition is administered subcutaneously or intravenously every 2 to 6 weeks at a dose of from about 2.0 mg/kg to about 10.0 mg/kg.

In an exemplary embodiment, the pharmaceutical composition is administered subcutaneously every 2 weeks.

The antibodies or antigen-binding fragments thereof of the present invention can be used as monotherapy or in combination with other therapies to treat cancer.

### Definitions

Unless otherwise defined herein, scientific and technical terms used in connection with the present invention shall have the meanings commonly understood by those of ordinary skill in the art. Furthermore, unless otherwise required by context, singular terms shall include the plural and plural terms shall include the singular. In general, the terminologies and techniques used in connection with cell and tissue culture, molecular biology, immunology, microbiology, genetics, and protein and nucleic acid chemistry and hybridization described herein are those well-known and commonly used in the art.

An "antigen-binding fragment" of an antibody refers to a fragment of a full-length antibody that retains the ability to specifically bind to an antigen (preferably, having substantially the same binding affinity). Examples of antigen-binding fragments include: (i) a Fab fragment, which is a monovalent fragment composed of VL, VH, CL and CH1 domains; (ii) an F(ab')2 fragment, which is a bivalent fragment including two Fab fragments linked by a disulfide bond in the hinge region; (iii) an Fd fragment composed of VH and CH1 domains; (iv) an Fv fragment composed of the VL and VH domains of the one-armed antibody; (v) a dAb fragment (Ward et al. (1989) Nature 341: 544-546), which is composed of a VH domain; and (vi) an isolated complementarity determining region (CDR), a disulfide-linked Fvs (dsFv), an anti-idiopathic (anti-Id) antibody, and an intrabody. Furthermore, although the two domains (VL and VH) of the Fv fragment are encoded by different genes, they can be joined by a synthetic linker using a recombinant method. The synthetic linker allows them to be made into a single protein chain in which the VL and VH regions are paired to form a monovalent molecule (referred to as a single-chain Fv (scFv)); see, for example, Bird et al. Science 242: 423-426 (1988) and Huston et al., Proc. Nat'l. Acad. Sci. USA 85: 5879-5883 (1988)). Other forms of single-chain antibodies, such as diabodies, are also included in the present invention. Diabodies are a type of bivalent bispecific antibodies, in which the VH and VL domains are expressed on a single polypeptide chain, and if a linker is used in such a case, it would be too short to allow pairing between the two domains on the same chain, thereby forcing the domains to pair with the complementary domains of the other chain and creating two antigen-binding sites (see, for example, Holliger et al., Proc. Nat'l. Acad. Sci. USA 90: 6444-6448 (1993); and Poljak et al., 1994, Structure 2: 1121-1123).

An antibody "variable domain" refers to a variable region of an antibody light chain (VL) or a variable region of an antibody heavy chain (VH), alone or in combination. As known in the art, the variable regions of the heavy and light chains are each composed of three complementarity determining regions (CDRs), connected by four framework regions (FRs), and contribute to the formation of the antigen-binding site of the antibody.

Residues in the variable domains are numbered according to the Kabat numbering system; Kabat is the numbering system for heavy or light chain variable domains used in the compilation of antibodies, see Kabat et al., Sequences of Proteins of Immunological Interest, 5th ed., Public Health Service, National Institutes of Health, Bethesda, MD (1991). Using this numbering system, the actual linear amino acid sequence may contain fewer or additional amino acids, which correspond to shortenings or insertions of FRs or CDRs of the variable domains. For a given antibody, the Kabat numbering of residues can be determined by aligning the antibody's sequence with regions of homology to the "standard" Kabat numbering sequence. Various algorithms for assigning Kabat numbers are available. Unless otherwise stated herein, Kabat numbers are assigned to variable regions using the algorithm performed in Abysis (www.abysis.org) published in 2012.

Specific amino acid residue positions in antibodies, such as paratope residues, are also numbered according to the Kabat system.

A "complementarity determining region" (CDR) can be identified according to the definition of aggregation, AbM, contact and/or conformation of both Kabat, Chothia, Kabat and Chothia, or any method of CDR determination well known in the art, see, for example, Kabat et al., 1991, Sequences of Proteins of Immunological Interest, 5th Ed. (hypervariable regions); Chothia et al., 1989, Nature 342: 877-883 (structural loop structures). The AbM definition of a CDR is a compromise between Kabat and Chothia, the definition of "contact" using Oxford Molecular's AbM antibody modeling software CDRs is based on the visible antigen contacts described in MacCallum et al., 1996, J. Mol. Biol., 262: 732-745. The "conformational" definition of a CDR is based on the creation of residues that contribute to the enthalpy of antigen binding (see, for example, Makabe et al., 2008, Journal of Biological Chemistry, 283: 1156-1166); yet other CDR boundary definitions may not strictly adhere to one of the methods above, but may nonetheless overlap with at least a portion of the Kabat CDR, which may however not significantly affect antigen binding depending on a particular residue or group of residues, or even all of the CDR shortened or extended by predictions or experimental findings. As used herein, a CDR may refer to a CDR defined by any method known in the art, including combinations of the methods.

An "epitope" refers to a region or range of an antigen (Ag) to which an antibody specifically binds, for example, a region or range including the amino acid residues with which the antibody (Ab) interacts. Epitopes can be linear or non-linear (for example, conformational).

An antibody or antigen-binding fragment thereof binds substantially to the same epitope as another antibody or antigen-binding fragment thereof when the binding of the corresponding antibody or antigen-binding fragment thereof is mutually exclusive. That is, binding of one antibody or antigen-binding fragment thereof precludes simultaneous or sequential binding of another antibody or antigen-binding fragment thereof. An epitope is considered unique or not substantially identical if the antigen can accommodate simultaneous binding of two corresponding antibodies or antigen-binding fragments thereof.

The term "paratope" is derived from the above definition of "epitope" by a twist angle, and refers to the region or range of the antibody molecule involved in antigen binding, for example, the region or range that includes the residues that interact with the antigen. The paratope can be linear or conformational (such as discontinuous residues in a CDR).

The epitope/paratope of a given antibody/antigen binding pair can be defined and characterized at various levels of detail using various experimental and computational epitope mapping methods. The experimental methods include mutagenesis, X-ray crystallography, nuclear magnetic resonance (NMR) spectroscopy, hydrogen/deuterium exchange mass spectrometry (HX-MS), and various competitive binding methods. Since each method relies on unique principles, the description of an epitope is closely related to the method by which the epitope has been determined. Thus, the epitope/paratope of a given antibody/antigen pair may be defined differently depending on the mapping method employed.

At the most detailed level, the epitopes/paratopes used for the interaction between the antibody (Ab) and the antigen (Ag) can be determined by defining the spatial coordinates of the atomic contacts present in the Ag-Ab interaction and information about defining the relative contributions thereof to the thermodynamics of binding. To one extent, epitope/paratope residues can be characterized by defining the spatial coordinates of atomic contacts between Ag and Ab. In one aspect, the epitope/paratope residue can be defined by specific criteria, such as the distance between atoms in Ab and Ag (for example, the distance from the heavy atoms of the homologous antibody to the heavy atoms of the antigen is equal to or less than the approximate definitions thereof). In another aspect, epitope/paratopic residues can be characterized as participating in hydrogen bonding interactions with the cognate antibody/antigen, or with a water molecule that also has hydrogen bound to the cognate antibody/antibody (water-mediated hydrogen binding). In another aspect, epitope/paratope residues can be characterized by forming salt bridges with residues of the homologous antibody/antigen. In yet another aspect, epitope/paratope residues can be characterized as residues that have a non-zero variation in a blocked surface area (BSA) due to interaction with a cognate antibody/antigen. At a less detailed level, epitope/paratope can be characterized by functions, for example, by competitive binding with other Abs. The epitope/paratope can also be defined more generally as including amino acid residues where substitution by another amino acid would alter the characteristics of the interaction between the Ab and Ag (for example, alanine scanning).

Since the description and definition of an epitope depend on the epitope mapping method used and the fact that it is obtained at different levels of detail, it may be inferred that the comparison of epitopes of different Abs on the same Ag can be similar at different levels of detail. For example, this can be described at the amino acid level, such as the epitopes determined from X-ray structures; they are considered identical if they contain the same set of amino acid residues. If the binding of the corresponding antibodies is mutually exclusive, that is, epitopes characterized by competitive binding can be considered as overlapping if binding of one antibody excludes simultaneous or sequential binding of another antibody; also, if the antigen can accommodate simultaneous binding of two corresponding antibodies, an epitope is considered to be distinct (unique).

The epitopes and paratopes of a given antibody/antigen pair can be identified by routine methods. For example, the general location of an epitope can be determined by assessing the ability of an antibody to bind to various fragments or variant polypeptides, as described more fully previously herein. Specific residues within OX40 that can contact specific residues within an antibody can also be determined using certain routine methods. For example, antibody/antigen complexes can be crystallized. The crystal structure can be determined and used to identify specific sites of interaction between the antibody and the antigen.

The term "specific binding" is a term well known in the art, and methods for determining these specific bindings are also well known in the art. A molecule is considered to exhibit "specific binding" if it reacts with or binds to a particular cell or substance more frequently, more rapidly, for a longer duration, and/or with greater affinity than the molecule reacts or binds with a surrogate cell or substance. An antibody or antigen-binding fragment thereof "specifically binds" to a target if the antibody or antigen-binding fragment thereof binds to a target with the features of greater affinity, greater binding ability, greater ease, and/or having longer duration than other substances.

For example, an antibody or antigen-binding fragment thereof that specifically binds to OX40 is an antibody that binds to its cognate antigen with the features of greater affinity, greater binding ability, greater ease, and/or having longer duration than other antigens. For example, under standard binding assay conditions, an anti-OX40 antibody can specifically bind to human OX40 in a sample, but does not substantially recognize or bind to other molecules in the same sample. It is also understood that an antibody or antigen-binding fragment thereof that specifically binds to a first target may or may not specifically bind to a second target. Thus, the "specific binding" herein does not necessarily require (although it may include) exclusive binding. Usually, but not necessarily, the "binding" used herein means specifically binding.

Various assay modes can be used to select antibodies or antigen-binding fragments thereof that specifically bind a molecule of interest. For example, among many assays, solid-phase ELISA immunoassays, immunoprecipitation, Biacore^{™} (GE Healthcare), KinExA, fluorescence-activated cell sorting (FACS), Octet^{™} (Forte Bio, Inc.), and Western blot analysis can be used to identify specific antibodies or antigen-binding fragments thereof that bind to an antigen. Typically, specific binding can be at least twice the background signal or noise, more typically at least 10 times the background, at least 50 times the background, at least 100 times the background, at least 500 times the background, at least 1000 times the background, or at least 10,000 times the background.

The specificity of antibody binding can be assessed by determining the K_{D} value for specific binding between the antibody and OX40 and comparing the K_{D} value to the K_{D} value of a control antibody known not to bind to OX40. In general, an antibody "specifically" binds to an antigen when the K_{D} is about × 10⁻⁵ M or less.

When an antibody or antigen-binding fragment thereof does not bind to an antigen with the features of greater affinity, greater binding ability, greater ease, and/or having longer duration than the antibody or antigen-binding fragment thereof binding to other antigens, the antibody or antigen-binding fragment thereof "substantially does not bind" to the antigen. Typically, the binding will be no greater than twice the background signal or noise. In general, it binds to the antigen with a K_{D} of 1 × 10⁻⁴ M or greater, 1 × 10⁻³ M or greater, 1 × 10⁻² M or greater, or 1 × 10⁻¹ M or greater.

The term "compete" as used herein with respect to antibodies means that binding of a first antibody or antigen-binding portion thereof to an antigen reduces binding of a subsequent second antibody or antigen-binding portion thereof to the same antigen. In general, binding of the first antibody results in steric hindrance, a conformational change or binding to a common epitope (or portion thereof) such that binding of the second antibody to the same antigen is reduced. Standard competitive binding assays can be used to determine whether two antibodies compete with each other.

A suitable assay for antibody competition involves the use of Biacore technology. This technology can use surface plasmon resonance (SPR) technology. The degree of interaction is typically measured using a biosensor system, such as a system. For example, SPR can be used in an in vitro competitive binding inhibition assay to determine the ability of one antibody to inhibit the binding of a second antibody. Another assay for measuring antibody competition uses an ELISA-based method. Furthermore, a high-throughput method for "fractionation" of antibodies based on competition of antibodies is described in WO 2003/48731. Competition exists if one antibody or antigen-binding fragment thereof reduces the binding of another antibody or antigen-binding fragment thereof to OX40. For example, sequential binding competition assays can be used, with sequential addition of different antibodies. Primary antibodies can be added to achieve near-saturated binding, and then, the secondary antibody is added. If the binding of the second antibody to OX40 is undetectable or significantly reduced (for example, at least about 10%, at least about 20%, at least about 30%, at least about 40%, at least about 50%, at least about 60%, at least about 70%, at least about 80%, or at least about 90%) as compared with a case in which the first antibody is absent (the median value can be set as 100%), the two antibodies are then considered to compete with each other.

Competitive binding assays can also be performed. Binding of an antibody to an antigen is compared to the binding of a target to another binding partner with the target, such as another antibody or soluble receptor that otherwise binds to the target. The concentration at which 50% inhibition occurs is referred to as Kᵢ. Under ideal conditions, this Kᵢ is equal to K_{D}. Thus, in general, the measurement of Kᵢ can be conveniently used to provide an upper limit for K_{D}. Binding affinities associated with different molecular interactions (for example, comparison of binding affinities of different antibodies for a given antigen) can be compared by means of the comparison of K_{D} values for individual antibody/antigen complexes. K_{D} values for antibodies or other binding partners can be determined using methods established in the art.

An "Fc fusion" protein is a protein in which one or more polypeptides are operably linked to an Fc polypeptide. An Fc fusion combines an Fc region of an immunoglobulin with a fusion partner. The "Fc region" can be a native sequence Fc region or a variant Fc region. Although the boundaries of the Fc region of an immunoglobulin heavy chain can vary, the Fc region of a human IgG heavy chain is generally defined as extending from the amino acid residue at position Cys226 or Pro230 to a carboxy terminus thereof. The numbering of residues in the Fc region can be the numbering with the EU index, as described in Kabat et al., Sequences of Proteins of Immunological Interest, 5th edition, Public Health Service, National Institutes of Health, Bethesda, Md., 1991. The Fc region of an immunoglobulin typically contains two constant domains (CH₂ and CH₃). As known in the art, the Fc regions can exist in a dimer or monomer form.

The term "therapeutically effective amount" means an amount of an anti-OX40 antibody or antigen-binding fragment thereof, or a combination comprising such an antibody or antigen-binding fragment thereof, sufficient to achieve the intended purpose. The precise amount may depend on many factors including, but not limited to, the components and physical characteristics of the therapeutic composition, the intended patient population, individual patient considerations, etc., and can be determined by a person skilled in the art.

The term "treatment" includes prophylactic and/or therapeutic treatment. Treatment is considered prophylactic or preventative if administered prior to the clinical manifestation of the disease, disorder or condition. Therapeutic treatment includes, for example, reducing or reducing the severity or shortening the length of a disease, disorder or condition.

As used herein, the term "about" refers to +/- 10% of a value.

### Therapeutic methods and compositions

Any of the anti-human OX40 antibodies provided herein can be used in methods of therapy.

In one aspect, the anti-human OX40 agonistic antibody is provided for use as a medicament. In yet other aspects, the anti-human OX40 agonistic antibody is provided for use in the treatment of cancer. In some embodiments, the anti-human OX40 agonistic antibody is provided for use in a method of treatment. In some embodiments, an anti-human OX40 agonistic antibody is provided for use in a method of treating an individual with cancer, including administering to the individual an effective amount of the anti-human OX40 agonistic antibody. In one such embodiment, the method further includes administering to the individual an effective amount of at least one additional therapeutic agent, for example, as described below.

In one aspect, provided is an anti-human OX40 agonistic antibody for use in enhancing immune function (for example, by upregulating a cell-mediated immune response) in an individual with cancer, including administering to the individual an effective amount of the anti-human OX40 agonistic antibody. In one aspect, provided is an anti-human OX40 agonistic antibody for use in enhancing T cell function in an individual with cancer, including administering to the individual an effective amount of the anti-human OX40 agonistic antibody. In one aspect, provided is an anti-human OX40 agonistic antibody for use in depleting human OX40-expressing cells (for example, OX40-expressing T cells, for example, OX40-expressing Treg), including administering to the individual an effective amount of the anti-human OX40 agonistic antibody. In some embodiments, the depletion is achieved through ADCC. In some embodiments, the depletion is achieved by phagocytosis. Provided is an anti-human OX40 agonistic antibody for use in the treatment of an individual with tumor immunity.

In yet other aspects, the anti-human OX40 agonistic antibody is provided for use in the treatment of infections (for example, bacterial or viral or other pathogenic infections). In some embodiments, the present invention provides an anti-human OX40 agonistic antibody for use in a method of treating an individual with an infection, including administering to the individual an effective amount of the anti-human OX40 agonistic antibody. In some embodiments, the infection is a viral and/or bacterial infection. In some embodiments, the infection is a pathogen infection.

In yet another aspect, the present invention provides the use of an anti-OX40 antibody for the manufacture or preparation of a medicament. In one embodiment, the medicament is for the treatment of cancer. In yet another embodiment, the medicament is for use in a method of treating cancer, including administering to an individual with cancer an effective amount of the medicament. In one such embodiment, the method further includes administering to the individual an effective amount of at least one additional therapeutic agent, such as those described below.

In one aspect, the medicament is for enhancing immune function (for example, by upregulating a cell-mediated immune response) in an individual with cancer, including administering to an individual an effective amount of the medicament. In one aspect, the medicament is for enhancing T cell function in an individual with cancer, including administering to an individual an effective amount of the medicament. In some embodiments, the T cell dysfunctional disorder is cancer. In one aspect, the medicament is used to deplete human OX40-expressing cells (for example, high OX40-expressing cells, for example, OX40-expressing T cells), including administering to an individual an effective amount of the medicament. In some embodiments, the depletion is achieved through ADCC. In some embodiments, the depletion is achieved by phagocytosis. In one aspect, the medicament is used to treat an individual with tumor immunity.

In yet other aspects, a medicament is provided for the treatment of infections (for example, bacterial or viral or other pathogenic infections). In some embodiments, the medicament is used in a method of treating an individual with an infection, including administering to the individual an effective amount of the medicament. In some embodiments, the infection is a viral and/or bacterial infection. In some embodiments, the infection is a pathogen infection.

In yet another aspect, the present invention provides a method for treating cancer. In one embodiment, the method includes administering to an individual with cancer an effective amount of an anti-OX40 antibody. In one such embodiment, the method further includes administering to the individual an effective amount of at least one additional therapeutic agent, for example, as described below. An "individual" according to any of the above embodiments may be a human.

In one aspect, provided is a method for enhancing immune function (for example, by upregulating a cell-mediated immune response) in an individual with cancer, including administering to the individual an effective amount of the anti-human OX40 agonistic antibody. In one aspect, provided is a method for enhancing T cell function in an individual with cancer comprising administering to the individual an effective amount of the anti-human OX40 agonistic antibody. In one aspect, provided is a method for depleting cells expressing human OX40 (for example, cells expressing high levels of OX40, for example, T cells expressing OX40), including administering to the individual an effective amount of the anti-human OX40 agonistic antibody. In some embodiments, the depletion is achieved through ADCC. In some embodiments, the depletion is achieved by phagocytosis. Provided is an anti-human OX40 agonistic antibody for use in the treatment of an individual with tumor immunity.

In some embodiments, examples of cancer further include, but are not limited to, B-cell lymphomas (including low-grade/follicular non-Hodgkin's lymphoma (NHL), small lymphocytic (SL) NHU, intermediate-grade/follicular NHL, intermediate diffuse NHL, high-grade immunoblastic NHL, high-grade lymphoblastic NHL, high-grade small anucleate NHL, bulky disease NHL, mantle cell lymphoma, AIDS-related lymphoma, and Var Waldenstrom's macroglobulinemia), chronic lymphocytic leukemia (CLL), acute lymphoblastic leukemia (ALL), hairy cell leukemia, chronic myeloblastic leukemia, and post-transplant lymphoid Proliferative Disorders (PTLD), as well as abnormal vascular proliferation associated with phakomatoses, edema (such as associated with brain tumors), B cell proliferative disorders, and Meigs syndrome. More specific examples include but are not limited to relapsed or refractory NHL, front line low-grade NHL, stage III/IV NHL, chemotherapy-resistant NHL, precursor B lymphoblastic leukemia and/or lymphoma, small lymphocytic lymphoma, B cells chronic lymphocytic leukemia and/or prolymphocytic leukemia and/or small lymphocytic lymphoma, B-cell prelymphocytic lymphoma, immunocytoma and/or lymphoplasmacytic lymphoma, lymphoplasmacytic B-cell lymphoma, marginal zone B-cell lymphoma, splenic marginal zone lymphoma, extranodal marginal zone-MALT lymphoma, nodal marginal zone lymphoma, hairy cell leukemia, pleocytoma and/or plasma cell myeloma, low-grade/follicular lymphoma, intermediate/follicular NHL, mantle cell lymphoma, follicular center lymphoma, intermediate-grade diffuse NHL, diffuse large B-cell lymphoma, aggressive NHL (including aggressive front-line NHL and aggressive relapsed NHL), relapsed or refractory NHL after autologous stem cell transplantation, primary mediastinal large B-cell lymphoma, primary effusion lymphoma, advanced immunoblast NHL, advanced lymphoblastoid NHL, advanced small anucleated cell NHL, bulky disease NHL, Burkitt's lymphoma, precursor (peripheral) large granular lymphocytic leukemia, mycosis fungoides and/or Sezary syndrome, cutaneous lymphoma, anaplastic large cell lymphoma, and angiocentric lymphoma.

In some embodiments, examples of cancer further include, but are not limited to, B cell proliferative disorders, which may include, but is not limited to, lymphomas (for example, B-cell non-Hodgkin's lymphoma (NHL)) and lymphocytic leukemias. Such lymphomas and lymphocytic leukemias include, for example, a) follicular lymphoma, b) small non-cleaved cell lymphoma/Burkitt's lymphoma (including endemic Burkitt's Lymphoma, sporadic Burkitt's lymphoma and non-Burkitt's lymphoma), c) marginal zone lymphomas (including extranodal marginal zone B-cell lymphomas (mucosal-associated lymphoid tissue lymphoma, MALT), nodal marginal zone B-cell lymphomas and splenic marginal zone lymphomas), d) mantle cell lymphoma (MCL), e) large cell lymphoma (including B-cell diffuse large cell lymphoma (DLCL), diffuse mixed cell lymphoma, immunoblastic lymphoma, primary mediastinal B-cell lymphoma, angiocentric lymphoma-pulmonary B-cell lymphoma), f) hairy cell leukemia, g) lymphocytic lymphoma, waldenstrom's macroglobulinemia, h) acute lymphocytic leukemia (ALL), chronic lymphocytic leukemia (CLL)/small lymphocytic lymphoma (SLL), B-cell prolymphocytic leukemia, i) plasma cell neoplasms, plasma cell myeloma, multiple myeloma, plasmacytoma, and/or j) Hodgkin's disease.

In some embodiments of any method, the cancer is a B cell proliferative disorder. In some embodiments, the B cell proliferative disorder is lymphoma, non-Hodgkin's lymphoma (NHL), aggressive NHL, relapsed aggressive NHL, relapsed painless NHL, refractory NHL, refractory painless NHL, chronic lymphocytic leukemia (CLL), small lymphocytic lymphoma, leukemia, hairy cell leukemia (HCL), acute lymphoblastic leukemia (ALL), or mantle cell lymphoma. In some embodiments, the B cell proliferative disorder is NHL, such as indolent NHL and/or aggressive NHL. In some embodiments, the B cell proliferative disorder is indolent follicular lymphoma or diffuse large B cell lymphoma.

In yet another aspect, the present invention provides pharmaceutical formulations comprising any of the anti-OX40 antibodies provided herein, for example, for use in any of the above methods of treatment. In one embodiment, a pharmaceutical formulation includes any of the anti-OX40 antibodies provided herein and a pharmaceutically acceptable carrier. In another embodiment, a pharmaceutical formulation includes any of the anti-OX40 antibodies provided herein and at least one additional therapeutic agent, for example, as described below.

In some embodiments of any of the methods of the present invention, the anti-human OX40 agonistic antibody kills OX40-expressing cells (e.g., cells expressing high levels of OX40) by inhibiting Treg function (e.g., inhibiting the suppressive function of Tregs), and increases Effector T cell function and/or increase memory T cell function to suppress tumor immunity. In some embodiments of any of the methods of the invention, the anti-human OX40 agonistic antibody kills OX40-expressing cells (e.g., cells expressing high levels of OX40), and increases effector T cell function and/or enhancement of memory T cell function to treat cancer. In some embodiments of any of the methods of the invention, the anti-human OX40 agonistic antibody kills OX40-expressing cells (e.g., cells expressing high levels of OX40), and increases effector T cell function and/or enhancement of memory T cell function to enhance immune function. In some embodiments of any of the methods of the invention, the anti-human OX40 agonistic antibody kills OX40-expressing cells (e.g., cells expressing high levels of OX40), and increases effector T cell function and/or enhancement of memory T cell function to enhance T cell function.

In some embodiments of any of the methods, the anti-human OX40 agonistic antibody is a depleting anti-human OX40 agonistic antibody. In some embodiments, treatment with the anti-human OX40 agonistic antibody results in depletion of cells (for example, depletion of cells expressing OX40, for example, depletion of cells expressing high levels of OX40). In some embodiments, the depletion is achieved through ADCC. In some embodiments, the depletion is achieved by phagocytosis.

In some embodiments of any of the methods, with respect to the Treg function prior to administration of the OX40 agonistic antibody, the anti-human OX40 agonistic antibody inhibits Treg function, for example, by inhibiting Treg suppression of effector and/or memory T cell function (in some embodiments, effector T cell and/or memory T cell proliferation and/or cytokine secretion). In some embodiments of any of the methods, with respect to the effector T cell proliferation prior to administration of the OX40 agonistic antibody, the anti-human OX40 agonistic antibody increases the effector T cell proliferation. In some embodiments of any of the methods, with respect to the memory T cell proliferation prior to administration of the OX40 agonistic antibody, the anti-human OX40 agonistic antibody increases memory T cell proliferation. In some embodiments of any method, with respect to the effector T cell cytokine production prior to administration of the OX40 agonistic antibody, the anti-human OX40 agonistic antibody increases effector T cell cytokine production (for example, gamma interferon production). In some embodiments of any method, with respect to memory T cell cytokine production prior to administration of the OX40 agonistic antibody, the anti-human OX40 agonistic antibody increases memory T cell cytokine production (for example, gamma interferon production). In some embodiments of any of the methods, with respect to the CD4+ effector T cell proliferation and/or CD8+ effector T cell proliferation prior to administration of the OX40 agonistic antibody, the anti-human OX40 agonistic antibody increases CD4+ effector T cell proliferation and/or CD8+ effector T cell proliferation. In some embodiments of any of the methods, with respect to the memory T cell proliferation prior to administration of the OX40 agonistic antibody, the anti-human OX40 agonistic antibody increases memory T cell proliferation (for example, CD4+ memory T cell proliferation). In some embodiments, with respect to the proliferation, cytokine secretion and/or lytic activity prior to administration of the anti-human OX40 agonistic antibody, the CD4+ effector T cells in an individual have enhanced proliferation, cytokine secretion and/or lytic activity.

In some embodiments of any of the methods of the present invention, the number of CD4+ effector T cells is elevated as compared to that prior to the administration of the anti-human OX40 agonistic antibody. In some embodiments, the CD4+ effector T cell cytokine secretion is elevated relative to prior to administration of the anti-human OX40 agonistic antibody. In some embodiments of any of the methods, the CD8+ effector T cells in the individual have enhanced proliferation, cytokine secretion and/or lytic activity as compared to those prior to administration of the anti-human OX40 agonistic antibody. In some embodiments, the number of CD8+ effector T cells is elevated as compared to that prior to the administration of the anti-human OX40 agonistic antibody. In some embodiments, the CD8+ effector T cell cytokine secretion is elevated as compared to that prior to the administration of the anti-human OX40 agonistic antibody.

In some embodiments of any of the methods of the present invention, the anti-human OX40 agonistic antibody binds to a human effector cell, for example, binds to an FcγR expressed by a human effector cell. In some embodiments, the human effector cell performs ADCC effector function. In some embodiments, the human effector cell performs phagocytic effector function.

In some embodiments of any of the methods of the present invention, the anti-human OX40 agonistic antibody including a variant IgGl Fc polypeptide (which includes a mutation that abolishes the binding to human effector cells, for example, the DANA or N297G mutation) has a reduced activity (for example, CD4+ effector T cell functions such as proliferation) as compared to an anti-human OX40 agonistic antibody including a native sequence IgGl Fc portion. In some embodiments, an anti-human OX40 agonistic antibody including a variant IgGl Fc polypeptide (including a mutation that abolishes the binding to human effector cells, for example, the DANA or N297G mutation), does not possess a substantial activity (for example, CD4+ effector T cell functions such as proliferation).

In some embodiments of any of the methods of the present invention, the antibody cross-linking is required for the anti-human OX40 agonistic antibody function. In some embodiments, the function is to stimulate the CD4+ effector T cell proliferation. In some embodiments, the antibody cross-linking is determined by providing an anti-human OX40 agonistic antibody that adheres to a solid surface (for example, a cell culture plate). In some embodiments, the antibody cross-linking is determined by introducing a mutation (for example, DANA or N297S mutation) into the IgGl Fc portion of the antibody and then testing the mutant antibody for function.

In some embodiments of any of the methods, the memory T cells in the individual have enhanced proliferation and/or cytokine secretion relative to that prior to the administration of the anti-human OX40 agonistic antibody. In some embodiments, the number of memory T cells is elevated relative to that prior to the administration of the anti-human OX40 agonistic antibody. In some embodiments, the memory T cell cytokine secretion (level) is elevated relative to that prior to the administration of the anti-human OX40 agonistic antibody. In some embodiments of any of the methods, the Treg in an individual has reduced inhibition on the effector T cell function (for example, proliferation and/or cytokine secretion) relative to that prior to the administration of the anti-human OX40 agonistic antibody. In some embodiments, the number of effector T cells is elevated relative to that prior to the administration of the anti-human OX40 agonistic antibody. In some embodiments, the effector T cell cytokine secretion (level) is elevated relative to that prior to the administration of the anti-human OX40 agonistic antibody.

In some embodiments of any of the methods of the present invention, the number of intratumoral (infiltrating) CD4+ effector T cells (for example, the total number of CD4+ effector T cells, or, for example, the percentage of CD4+ cells in CD45+ cells) is elevated relative to that prior to the administration of the anti-human OX40 agonistic antibody. In some embodiments of any of the methods of the present invention, the number of intratumoral (infiltrating) CD4+ effector T cells expressing interferon-y (for example, total interferon gamma expressing CD4+ cells, or, for example, the percentage of CD4+ cells expressing interferon in the total CD4+ cells) is elevated relative to that prior to the administration of the anti-human OX40 agonistic antibody.

In some embodiments of any of the methods of the present invention, the number of intratumoral (infiltrating) CD8+ effector T cells (for example, the total number of CD8+ effector T cells, or, for example, the percentage of CD8+ cells in CD85+ cells) is elevated relative to that prior to the administration of the anti-human OX40 agonistic antibody. In some embodiments of any of the methods of the present invention, the number of intratumoral (infiltrating) CD8+ effector T cells expressing interferon-y (for example, the percentage of CD8+ cells expressing interferon in the total CD8+ cells) is elevated relative to that prior to the administration of the anti-human OX40 agonistic antibody.

In some embodiments of any of the methods of the present invention, the number of intratumoral (infiltrating) Tregs (e.g., the total number of Tregs or the percentage of Fox3p+ cells in, for example, CD4+ cells) is decreased relative to that prior to the administration of the anti-human OX40 agonistic antibody.

In some embodiments of any of the methods of the present invention, the administration of an anti-human OX40 agonistic antibody is combined with the administration of a tumor antigen. In some embodiments, the tumor antigen includes a protein. In some embodiments, the tumor antigen includes a nucleic acid. In some embodiments, the tumor antigen is a tumor cell.

In some embodiments of any of the methods of the present invention, the cancer exhibits human effector cells (for example, is infiltrated by human effector cells). The methods for detecting human effector cells are well known in the art, including, for example, IHC. In some embodiments, the cancer exhibits a high level of human effector cells. In some embodiments, the human effector cells are one or more of NK cells, macrophages, and monocytes. In some embodiments, the cancer can be any cancer described herein. In some embodiments, the cancer is non-small cell lung cancer (NSCLC), glioblastoma, neuroblastoma, melanoma, breast cancer (for example, triple negative breast cancer), gastric cancer, colorectal cancer (CRC), or hepatocellular carcinoma.

In some embodiments of any of the methods of the present invention, the cancer exhibits FcR-expressing cells (for example, is infiltrated by FcR-expressing cells). The methods for detecting FcR are well known in the art, including, for example, IHC. In some embodiments, the cancer exhibits a high level of FcR-expressing cells. In some embodiments, the FcR is an FcyR.

In some embodiments, the FcR is an activating FcγR. In some embodiments, the cancer is non-small cell lung cancer (NSCLC), glioblastoma, neuroblastoma, melanoma, breast cancer (e.g., triple negative breast cancer), gastric cancer, colorectal cancer (CRC), or hepatocellular carcinoma.

An "individual" according to any of the above embodiments is preferably a human.

The antibodies of the present invention can be used alone or in combination with other agents in therapy. For example, an antibody of the present invention can be co-administered with at least one additional therapeutic agent.

Such combination therapy noted above includes combined administration (where two or more therapeutic agents are contained in the same formulation or separate formulations), and separate administration. In this case, the administration of the antibody of the present invention may occur prior to, concurrently with, and/or post the administration of another therapeutic agent(s) and/or medicament. In one embodiment, the administration of the anti-OX40 antibody and the administration of an additional therapeutic agent are within about one month, or within about one, two or three weeks, or within about 1, 2, 3, 4, 5, or 6 days. The antibodies of the present invention can also be used in combination with radiation therapy.

In some embodiments, the anti-human OX40 agonistic antibody can be administered in combination with chemotherapy or a chemotherapeutic agent. In some embodiments, the anti-human OX40 agonistic antibody can be administered in combination with radiation therapy or a radiation agent. In some embodiments, the anti-human OX40 agonistic antibody can be administered in combination with targeted therapy or a targeted therapeutic agent. In some embodiments, the anti-human OX40 agonistic antibody can be administered in combination with immunotherapy or an immunotherapeutic agent, for example, a monoclonal antibody.

In some embodiments, the anti-human OX40 agonistic antibody can be administered in combination with PARP inhibitors (for example, Olaparanib, Rucaparib, Niraparib, Cediranib, BMN673, Veliparib), Trabectedin, nab-paclitaxel (albumin-bound Paclitaxel, ABRAXANE), Trebananib, Pazopanib, Cediranib, Palbociclib, everolimus, fluorouracil (e.g., FOLFOX, FOLFIRI), IFL, regorafenib, Reolysin, Alimta, Zykadia, Sutent, Torisel (temsirolimus), Inlyta (axitinib, Pfizer), Afinitor (everolimus, Novartis) , Nexavar (sorafenib, Onyx/Bayer), Votrient, Pazopanib, axitinib, IMA-901, AGS_003, cabozantinib, Vinflunine, Hsp90 inhibitors (e.g., apatorsin), Ad-GM-CSF (CT-0070), Temazolomide, IL-2 , IFNa, vinblastine, Thalomid, dacarbazine, cyclophosphamide, lenalidomide, azacytidine, lenalidomide, bortezomid (VELCADE), amrubicine, carfilzomib, pralatrexate, and/or enzastaurin.

In some embodiments, the anti-human OX40 agonistic antibody can be administered in combination with a PD-1 axis binding antagonist. The PD-1 axis binding antagonist herein includes, but is not limited to, PD-1 binding antagonists, PD-L1 binding antagonists and PD-L2 binding antagonists. Alternative names for "PD-1" include CD279 and SLEB2. Alternative names for "PD-L1" include B7-H1, B7-4, CD274 and B7-H. Alternative names for "PD-L2" include B7-DC, Btdc, and CD273. In some embodiments, PD-1, PD-L1, and PD-L2 are human PD-1, PD-L1, and PD-L2. In some embodiments, a PD-1 binding antagonist is a molecule that inhibits the binding of PD-1 to its ligand binding partner. In a specific aspect, the PD-1 ligand binding partner is PD-L1 and/or PD-L2. In another embodiment, the PD-L1 binding antagonist is a molecule that inhibits the binding of PD-L1 to its binding partner. In a specific aspect, the PD-L1 binding partner is PD-1 and/or B7-1. In another embodiment, a PD-L2 binding antagonist is a molecule that inhibits the binding of PD-L2 to its binding partner. In a specific aspect, the PD-L2 binding partner is PD-1. The antagonists can be antibodies, antigen-binding fragments thereof, immunoadhesins, fusion proteins, or oligopeptides. In some embodiments, the PD-1 binding antagonist can be an anti-PD-1 antibody (for example, a human antibody, humanized antibody, or chimeric antibody). In some embodiments, the anti-PD-1 antibody is selected from the group consisting of MDX-1106 (nivolumab, OPDIVO), Merck 3475 (MK-3475, pembrolizumab, KEYTRUDAWPCT-011 (Pidilizumab). In some embodiments, the PD-1 binding antagonist is an immunoadhesin (for example, an immunoadhesin comprising the extracellular or PD-1 binding portion of PD-L1 or PD-L2 fused to a constant region (for example, the Fc region of an immunoglobulin sequence). In some embodiments, the PD-1 binding antagonist is AMP-224. In some embodiments, the PD-L1 binding antagonist is an anti-PD-L1 antibody. In some embodiments, the anti-PD-L1 binding antagonist is selected from the group consisting of YW243.55.S70, MPDL3280A, MEDI4736 and MDX-1105. MDX-1105, also known as BMS-936559, is an anti-PD-L1 antibody as described in WO2007/005874. Antibody YW243.55.S70 is an anti-PD-L1 antibody as described in WO 2010/077634 A1. MDX-1106, also known as MDX-1106-04, ONO-4538, BMS-936558 or nivolumab, is an anti-PD-1 antibody as described in WO2006/121168. Merck 3475, also known as MK-3475, SCH-900475 or pembrolizumab, is an anti-PD-1 antibody as described in WO2009/114335. CT-011, also known as hBAT, hBAT-1 or pidil izumab, is an anti-PD-1 antibody as described in WO2009/101611. AMP-224, also known as B7-DCIg, is a PD-L2-Fc fusion soluble receptor as described in WO2010/027827 and WO2011/066342. In some embodiments, the anti-PD-1 antibody is MDX-1106. Alternative names for "MDX-1106" include MDX-1106-04, 0N0-4538, BMS-936558 or nivoIumab. In some embodiments, the anti-PD-1 antibody is nivolumab (CAS Registry No: 946414-94-4).

In some embodiments, the anti-human OX40 agonistic antibody can be administered in combination with an agonist against an activating costimulatory molecule. In some embodiments, the activating costimulatory molecule can include CD40, CD226, CD28, GITR, CD137, CD27, HVEM, and CD127. In some embodiments, the agonist against an activating costimulatory molecule is an agonistic antibody that binds to CD40, CD226, CD28, OX40, GITR, CD137, CD27, HVEM, or CD127. In some embodiments, an anti-human OX40 agonistic antibody can be administered in combination with an antagonist against an inhibitory costimulatory molecule. In some embodiments, the inhibitory costimulatory molecules can include CTLA-4 (also known as CD152), PD-1, TIM-3, BTLA, VISTA, LAG-3, B7-H3, B7-H4, IDO, TIGIT, MICA/B, or arginase. In some embodiments, the antagonist against inhibitory costimulatory molecules is an antagonistic antibody, binding to CTLA-4, PD-1, TIM-3, BTLA, VISTA, LAG-3 (such as LAG-3-IgG fusion protein (IMP321)), B7-H3, B7-H4, IDO, TIGIT, MICA/B, or arginase.

In some embodiments, the anti-human OX40 agonistic antibody can be administered in combination with an antagonist, for example, a blocking antibody, directed against CTLA 4 (also known as CD152). In some embodiments, the anti-human OX40 agonistic antibody can be administered in combination with ipilimumab (also known as MDX-010, MDX-101, or Yervoy^{®}). In some embodiments, the anti-human OX40 agonistic antibody can be administered in combination with tremelimumab (also known as ticilimumab or CP-675, 206). In some embodiments, the anti-human OX40 agonistic antibody can be administered in combination with an antagonist, for example, a blocking antibody, directed against B7-H3 (also known as CD276). In some embodiments, the anti-human OX40 agonistic antibody can be administered in combination with MGA271. In some embodiments, the anti-human OX40 agonistic antibody can be administered in combination with an antagonist against TGF-β, for example, metelimumab (also known as CAT-192), fresolimumab (also known as GC1008), or LY2157299.

In some embodiments, the anti-human OX40 agonistic antibody can be administered in combination with a therapy including adoptive transfer of chimeric antigen receptor (CAR) expressing T cells (for example, cytotoxic T cells or CTL). In some embodiments, the anti-human OX40 agonistic antibody can be administered in combination with UCART19. In some embodiments, the anti-human OX40 agonistic antibody can be administered in combination with WT128z. In some embodiments, anti-human OX40 agonistic antibodies can be administered in combination with KTE-C19 (Kite). In some embodiments, the anti-human OX40 agonistic antibody can be administered in combination with CTL019 (Novartis). In some embodiments, the anti-human OX40 agonistic antibody can be administered in combination with a therapy including adoptive transfer of T cells comprising a dominant-negative TGFI3 receptor, for example, a dominant-negative TGFWI type receptor. In some embodiments, the anti-human OX40 agonistic antibody can be administered in combination with a therapy including a HERCREEM regimen (see, for example, ClinicalTrials.gov Identifier NCT00889954).

In some embodiments, the anti-human OX40 agonistic antibody can be administered in combination with an antagonist against CD19. In some embodiments, the anti-human OX40 agonistic antibody can be administered in combination with MOR00208. In some embodiments, the anti-human OX40 agonistic antibody can be administered in combination with an antagonist to CD38. In some embodiments, the anti-human OX40 agonistic antibody can be administered in combination with daratumumab.

In some embodiments, the anti-human OX40 agonistic antibody can be administered in combination with an agonist, for example, an activating antibody, directed against CD137 (also known as TNFRSF9, 4-1BB, or ILA). In some embodiments, the anti-human OX40 agonistic antibody can be administered in combination with urelumab (also known as BMS-663513). In some embodiments, the anti-human OX40 agonistic antibody can be administered in combination with an agonist directed against CD40, e.g., an activating antibody. In some embodiments, the anti-human OX40 agonistic antibody can be administered in combination with CP-870893. In some embodiments, the anti-human OX40 agonistic antibody can be administered in combination with an agonist, for example, an agonist of OX40 (also known as CD134). In some embodiments, the anti-human OX40 agonistic antibody can be administered in combination with a different anti-OX40 antibody (for example, AgonOX). In some embodiments, the anti-human OX40 agonistic antibody can be administered in combination with an agonist of CD27, for example, an activating antibody. In some embodiments, the anti-human OX40 agonistic antibody can be administered in combination with CDX-1127. In some embodiments, the anti-human OX40 agonistic antibody can be administered in combination with an antagonist to indoleamine-2,3-dioxygenase (IDO). In some embodiments, the IDO antagonist is 1-methyl-D-tryptophan (also known as I-D-MT).

In some embodiments, the anti-human OX40 agonistic antibody can be administered in combination with an agonist, for example, an activating antibody, of CD137 (also known as TNFRSF9, 4-1BB, or ILA). In some embodiments, the anti-human OX40 agonistic antibody can be administered in combination with urelumab (also known as BMS-663513). In some embodiments, the anti-human OX40 agonistic antibody can be administered in combination with an agonist of CD40, for example, an activating antibody. In some embodiments, the anti-human OX40 agonistic antibody can be administered in combination with CP-870893 or R07009789. In some embodiments, the anti-human OX40 agonistic antibody can be administered in combination with an agonist, for example, an activating antibody, of OX40 (also known as CD134). In some embodiments, the anti-human OX40 agonistic antibody can be administered in combination with an agonist of CD27, for example, an activating antibody. In some embodiments, the anti-human OX40 agonistic antibody can be administered in combination with CDX-1127 (also known as varlilumab). In some embodiments, the anti-human OX40 agonistic antibody can be administered in combination with an antagonist to indoleamine-2,3-dioxygenase (IDO). In some embodiments, the IDO antagonist is 1-methyl-D-tryptophan (also known as 1-D-MT). In some embodiments, the IDO antagonist is the IDO antagonist as described in WO2010/005958, and the contents of which are incorporated by reference herein. In some embodiments, the IDO antagonist is 4-({2-[(aminosulfonyl)amino]ethyl}amino)-N-(3-bromo-4-fluorophenyl)-N'-hydroxy-1,2,5-oxadiazole-3-carboxamidine (for example, as described in Example 23 of WO2010/005958). In some embodiments, the IDO antagonist is as follows:

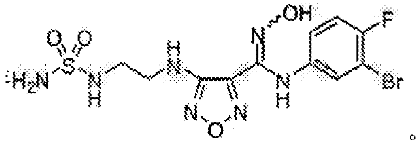

In some embodiments, the IDO antagonist is INCB24360. In some embodiments, the IDO antagonist is Indoximod (the D isomer of 1-methyl-tryptophan). In some embodiments, the anti-human OX40 agonistic antibody can be administered in combination with an antibody-drug conjugate. In some embodiments, the antibody-drug conjugate includes mertansine or monomethyl auristatin E (MMAE). In some embodiments, the anti-human OX40 agonistic antibody can be administered in combination with an anti-NaPi2b antibody-MMAE conjugate (also known as DNIB0600A, RG7599, or lifastuzumab vedotin). In some embodiments, the anti-human OX40 agonistic antibody can be administered in combination with trastuzumab emtansine (also known as T-DM1, ado-trastuzumab emtansine, or KADCYL.A^{®}, Genentech). In some embodiments, the anti-human OX40 agonistic antibody can be administered in combination with an anti-MUC16 antibody-MMAE conjugate, DMUC5754A. In some embodiments, the anti-human OX40 agonistic antibody can be administered in combination with an anti-MUC16 antibody-MMAE conjugate, DMUC4064A. In some embodiments, the anti-human OX40 agonistic antibody can be administered in combination with an endothelin B receptor (EDNBR)-targeting antibody-drug conjugate, for example, an MMAE-conjugated antibody against EDNBR. In some embodiments, the anti-human OX40 agonistic antibody can be combined with an antibody-drug conjugate targeting the lymphocyte antigen 6 complex, locus E (Ly6E), for example, an MMAE-conjugated antibody against Ly6E (also known as DLYE5953A). In some embodiments, the anti-human OX40 agonistic antibody can be administered in combination with polatuzumab vedotin. In some embodiments, the anti-human OX40 agonistic antibody can be administered in combination with an antibody-drug conjugate targeting CD30. In some embodiments, the anti-human OX40 agonistic antibody can be administered in combination with ADCETRIS (also known as brentuximab vedotin). In some embodiments, the anti-human OX40 agonistic antibody can be administered in combination with polatuzumab vedotin.

In some embodiments, the anti-human OX40 agonistic antibody can be administered in combination with an angiogenesis inhibitor. In some embodiments, the anti-human OX40 agonistic antibody can be administered in combination with an antibody directed against VEGF, for example, VEGF-A. In some embodiments, the anti-human OX40 agonistic antibody can be administered in combination with bevacizumab (also known as AVASTIN^{®}, Genentech). In some embodiments, the anti-human OX40 agonistic antibody can be administered in combination with an antibody against Angiopoietin 2 (also known as Ang2). In some embodiments, the anti-human OX40 agonistic antibody can be administered in combination with MEDI3617. In some embodiments, the anti-human OX40 agonistic antibody can be administered in combination with an antibody against VEGFR2. In some embodiments, the anti-human OX40 agonistic antibody can be administered in combination with ramucirumab. In some embodiments, the anti-human OX40 agonistic antibody can be administered in combination with a VEGF receptor fusion protein. In some embodiments, the anti-human OX40 agonistic antibody can be administered in combination with aflibercept. In some embodiments, anti-human OX40 agonistic antibodies can be administered in combination with ziv-aflibercept (also known as VEGF trap or ZALTRAP^{®}). In some embodiments, the anti-human OX40 agonistic antibody can be administered in combination with a bispecific antibody against VEGF and Ang2. In some embodiments, the anti-human OX40 agonistic antibody can be administered in combination with RG7221 (also known as vanucizumab). In some embodiments, the anti-human OX40 agonistic antibody can be administered in combination with an angiogenesis inhibitor and a PD-1 axis binding antagonist (for example, a PD-1 binding antagonist such as an anti-PD-1 antibody, a PD-L1 binding antagonist such as an anti-PD-L1 antibody, and a PD-L2 binding antagonist such as an anti-PD-L2 antibody). In some embodiments, the anti-human OX40 agonistic antibody can be administered in combination with bevacizumab and a PD-1 axis binding antagonist (for example, a PD-1 binding antagonist such as an anti-PD-1 antibody, a PD-L1 binding antagonist such as an anti-PD-L1 antibody, and a PD-L2 binding antagonist such as an anti-PD-L2 antibody). In some embodiments, the anti-human OX40 agonistic antibody can be administered in combination with bevacizumab and MDX-1106 (nivolumab, OPDIVO). In some embodiments, the anti-human OX40 agonistic antibody can be administered in combination with bevacizumab and Merck 3475 (MK-3475, pembrolizumab, KEYTRUDA). In some embodiments, the anti-human OX40 agonistic antibody can be administered in combination with bevacizumab and CT-011 (Pidilizumab). In some embodiments, the anti-human OX40 agonistic antibody can be administered in combination with bevacizumab and YW243.55.S70. In some embodiments, the anti-human OX40 agonistic antibody can be administered in combination with bevacizumab and MPDL3280A. In some embodiments, an anti-human OX40 agonistic antibody can be administered in combination with bevacizumab and MEDI4736. In some embodiments, an anti-human OX40 agonistic antibody can be administered in combination with bevacizumab and MDX-1105.

In some embodiments, the anti-human OX40 agonistic antibody can be administered in combination with an anti-tumor agent. In some embodiments, the anti-human OX40 agonistic antibody can be administered in combination with an agent targeting CSF-1R (also known as M-CSFR or CD115). In some embodiments, the anti-human OX40 agonistic antibody can be administered in combination with an anti-CSF-1R antibody (also known as IMC-CS4 or LY3022855). In some embodiments, the anti-human OX40 agonistic antibody can be administered in combination with the anti-CSF-1R antibody, RG7155 (also known as R05509554 or emactuzumab). In some embodiments, the anti-human OX40 agonistic antibody can be administered in combination with an interferon, for example, interferon-a or interferon-y. In some embodiments, the anti-human OX40 agonistic antibody can be administered in combination with Roferon-A (also known as recombinant interferon α-2a). In some embodiments, the anti-human OX40 agonistic antibody can be administered in combination with GM-CSF (also known as recombinant human granulocyte macrophage colony stimulating factor, rhu GM-CSF, sargramostim, or Leukine^{®}). In some embodiments, the anti-human OX40 agonistic antibody can be administered in combination with IL-2 (also known as aldesleukin or Proleukin^{®}). In some embodiments, the anti-human OX40 agonistic antibody can be administered in combination with IL-12. In some embodiments, the anti-human OX40 agonistic antibody can be administered in combination with IL27. In some embodiments, the anti-human OX40 agonistic antibody can be administered in combination with IL-15. In some embodiments, the anti-human OX40 agonistic antibody can be administered in combination with ALT-803. In some embodiments, the anti-human OX40 agonistic antibody can be administered in combination with an antibody targeting CD20. In some embodiments, the antibody targeting CD20 is onulizumab (also known as GAlO 1 or Gazyva^{®}) or rituximab. In some embodiments, the anti-human OX40 agonistic antibody can be administered in combination with an antibody targeting GITR. In some embodiments, the antibody targeting GITR is TRX518. In some embodiments, the antibody targeting GITR is MK04166 (Merck).

In some embodiments, the anti-human OX40 agonistic antibody can be administered in combination with an inhibitor of Bruton's tyrosine kinase (BTK). In some embodiments, the anti-human OX40 agonistic antibody can be administered in combination with ibrutinib. In some embodiments, the anti-human OX40 agonistic antibody can be administered in combination with an inhibitor of vaccine dehydrogenase 1 (IDH1) and/or vaccine dehydrogenase 2 (IDH2). In some embodiments, the anti-human OX40 agonistic antibody can be administered in combination with AG-120 (Agios).

In some embodiments, the anti-human OX40 agonistic antibody can be administered in combination with onulizumab and a PD-1 axis binding antagonist (for example, a PD-1 binding antagonist such as an anti-PD-1 antibody, a PD-L1 binding antagonist such as an anti-PD-1 PD-L1 antibody, and a PD-L2 binding antagonist such as an anti-PD-L2 antibody).

In some embodiments, the anti-human OX40 agonistic antibody can be administered in combination with a cancer vaccine. In some embodiments, the cancer vaccine is a peptide cancer vaccine, which in some embodiments is a personalized peptide vaccine. In some embodiments, the peptide cancer vaccine is a multivalent long peptide, a multiple peptide, a peptide mixture, a hybrid peptide, or a peptide-pulsed dendritic cell vaccine (see, for example, Yamada et al., Cancer Sci, 104:14- 21, 2013). In some embodiments, the anti-human OX40 agonistic antibody can be administered in combination with an adjuvant. In some embodiments, the anti-human OX40 agonistic antibody can be administered in combination with a treatment including a TLR agonist, such as Poly-ICLC (also known as Hiltonol^{®}), LPS, MPL, or CpG ODN. In some embodiments, the anti-human OX40 agonistic antibody can be administered in combination with tumor necrosis factor (TNF) α. In some embodiments, the anti-human OX40 agonistic antibody can be administered in combination with IL-1. In some embodiments, the anti-human OX40 agonistic antibody can be administered in combination with HMGB1. In some embodiments, the anti-human OX40 agonistic antibody can be administered in combination with an IL-10 antagonist. In some embodiments, the anti-human OX40 agonistic antibody can be administered in combination with an IL-4 antagonist. In some embodiments, the anti-human OX40 agonistic antibody can be administered in combination with an IL-13 antagonist. In some embodiments, the anti-human OX40 agonistic antibody can be administered in combination with an IL-17 antagonist. In some embodiments, the anti-human OX40 agonistic antibody can be administered in combination with an HVEM antagonist. In some embodiments, the anti-human OX40 agonistic antibody can be administered in combination with an ICOS agonist (for example, administering ICOS-L, or an agonistic antibody of ICOS). In some embodiments, the anti-human OX40 agonistic antibody can be administered in combination with a therapy targeting CX3CL1. In some embodiments, the anti-human OX40 agonistic antibody can be administered in combination with a therapy targeting CXCL9. In some embodiments, the anti-human OX40 agonistic antibody can be administered in combination with a therapy targeting CXCL10. In some embodiments, the anti-human OX40 agonistic antibody can be administered in combination with a therapy targeting CCL5. In some embodiments, the anti-human OX40 agonistic antibody can be administered in combination with an LFA-I or ICAM1 agonist. In some embodiments, the anti-human OX40 agonistic antibody can be administered in combination with a selectin agonist.

In some embodiments, the anti-human OX40 agonistic antibody can be administered in combination with an inhibitor of B-Raf In some embodiments, the anti-human OX40 agonist antibody can be administered in combination with vemurafenib (also known as Zelboraf^{®}). In some embodiments, the anti-human OX40 agonistic antibody can be administered in combination with dabrafenib (also known as Tafinlar^{®}). In some embodiments, the anti-human OX40 agonistic antibody can be administered in combination with encorafenib (LGX818).

In some embodiments, the anti-human OX40 agonistic antibody can be administered in combination with an EGFR inhibitor. In some embodiments, the anti-human OX40 agonistic antibody can be administered in combination with erlotinib (also known as Tarceva^{®}). In some embodiments, the anti-human OX40 agonistic antibody can be administered in combination with an inhibitor of EGFR-T790M. In some embodiments, the anti-human OX40 agonistic antibody can be administered in combination with gefitinib. In some embodiments, the anti-human OX40 agonistic antibody can be administered in combination with afatinib. In some embodiments, the anti-human OX40 agonistic antibody can be administered in combination with cetuximab (also known as Erbitux^{®}). In some embodiments, the anti-human OX40 agonistic antibody can be administered in combination with panitumumab (also known as Vectibix^{®}). In some embodiments, the anti-human OX40 agonistic antibody can be administered in combination with rociletinib. In some embodiments, the anti-human OX40 agonistic antibody can be administered in combination with AZD9291. In some embodiments, the anti-human OX40 agonistic antibody can be administered in combination with an inhibitor of MEK, such as MEK1 (also known as MAP2K1) and/or MEK2 (also known as MAP2K2). In some embodiments, the anti-human OX40 agonistic antibody can be administered in combination with cobimetinib (also known as CDC-0973 or XL-518). In some embodiments, the anti-human OX40 agonistic antibody can be administered in combination with trametinib (also known as Mekinist^{®}). In some embodiments, the anti-human OX40 agonistic antibody can be administered in combination with binimetinib.

In some embodiments, the anti-human OX40 agonistic antibody can be administered in combination with an inhibitor of B-Raf (for example, vemurafenib or dabrafenib) and an inhibitor of MEK (for example, MEK1 and/or MEK2) (e.g., cobimetinib or trametinib). In some embodiments, the anti-human OX40 agonistic antibody can be administered in combination with an inhibitor of ERK (e.g., ERK1/2). In some embodiments, the anti-human OX40 agonistic antibody can be administered in combination with GDC-0994. In some embodiments, the anti-human OX40 agonistic antibody can be administered in combination with an inhibitor of B-Raf, an inhibitor of MEK, and an inhibitor of ERK1/2. In some embodiments, the anti-human OX40 agonistic antibody can be administered in combination with an inhibitor of EGFR, an inhibitor of MEK, and an inhibitor of ERK1/2. In some embodiments, the anti-human OX40 agonistic antibody can be administered in combination with one or more MAP kinase pathway inhibitors. In some embodiments, the anti-human OX40 agonistic antibody can be administered in combination with CK127. In some embodiments, the anti-human OX40 agonistic antibody can be administered in combination with an inhibitor of K-Ras.

In some embodiments, the anti-human OX40 agonistic antibody can be administered in combination with an inhibitor of c-Met. In some embodiments, the anti-human OX40 agonistic antibody can be administered in combination with onartuzumab (also known as MetMAb). In some embodiments, the anti-human OX40 agonistic antibody can be administered in combination with an inhibitor of anaplatic lymphoma kinase (ALK). In some embodiments, the anti-human OX40 agonistic antibody can be administered in combination with AF802 (also known as CH5424802 or alectinib). In some embodiments, the anti-human OX40 agonistic antibody can be administered in combination with crizotinib. In some embodiments, the anti-human OX40 agonistic antibody can be administered in combination with ceritinib. In some embodiments, the anti-human OX40 agonistic antibody can be administered in combination with an inhibitor of phosphatidylinositol 3-kinase (PI3K). In some embodiments, the anti-human OX40 agonistic antibody can be administered in combination with buparlisib (BKM-120). In some embodiments, the anti-human OX40 agonistic antibody can be administered in combination with pictilisib (also known as GDC-0941). In some embodiments, the anti-human OX40 agonistic antibody can be administered in combination with buparlisib (also known as BKM-120). In some embodiments, the anti-human OX40 agonistic antibody can be administered in combination with perifosine (also known as KRX-0401). In some embodiments, the anti-human OX40 agonistic antibody can be administered in combination with a delta-selective inhibitor of phosphatidylinositol 3-kinase (PI3K). In some embodiments, the anti-human OX40 agonistic antibody can be administered in combination with idelalisib (also known as GS-1101 or CAL-101). In some embodiments, the anti-human OX40 agonistic antibody can be administered in combination with taselisib (also known as GDC-0032). In some embodiments, the anti-human OX40 agonistic antibody can be administered in combination with BYL-719. In some embodiments, the anti-human OX40 agonistic antibody can be administered in combination with an inhibitor of Akt. In some embodiments, the anti-human OX40 agonistic antibody can be administered in combination with MK2206. In some embodiments, the anti-human OX40 agonistic antibody can be administered in combination with GSK690693. In some embodiments, the anti-human OX40 agonistic antibody can be administered in combination with ipatasertib (also known as CDC-0068). In some embodiments, an anti-human OX40 agonist antibody can be administered in combination with an inhibitor of mTOR. In some embodiments, the anti-human OX40 agonistic antibody can be administered in combination with sirolimus (also known as rapamycin). In some embodiments, the anti-human OX40 agonistic antibody can be administered in combination with temsirolimus (also known as CCI-779 or Torisel^{®}). In some embodiments, the anti-human OX40 agonistic antibody can be administered in combination with everolimus (also known as RAD001). In some embodiments, the anti-human OX40 agonistic antibody can be administered in combination with ridaforolimus (also known as AP-23573, MK_8669, or deforolimus). In some embodiments, the anti-human OX40 agonistic antibody can be administered in combination with OSI-027. In some embodiments, the anti-human OX40 agonistic antibody can be administered in combination with AZD8055. In some embodiments, the anti-human OX40 agonistic antibody can be administered in combination with INK128. In some embodiments, the anti-human OX40 agonistic antibody can be administered in combination with a dual PI3K/mTOR inhibitor. In some embodiments, the anti-human OX40 agonistic antibody can be administered in combination with XL765. In some embodiments, the anti-human OX40 agonistic antibody can be administered in combination with GDC-0980. In some embodiments, the anti-human OX40 agonistic antibody can be administered in combination with BEZ235 (also referred to as NVP-BEZ235). In some embodiments, the anti-human OX40 agonistic antibody can be administered in combination with BGT226. In some embodiments, the anti-human OX40 agonistic antibody can be administered in combination with GSK2126458. In some embodiments, the anti-human OX40 agonistic antibody can be administered in combination with PF-04691502. In some embodiments, the anti-human OX40 agonistic antibody can be administered in combination with PF-05212384 (also known as PKI-587).

In some embodiments, the anti-human OX40 agonistic antibody can be administered in combination with an agent that selectively degrades the estrogen receptor. In some embodiments, the anti-human OX40 agonistic antibody can be administered in combination with GDC-0927. In some embodiments, the anti-human OX40 agonistic antibody can be administered in combination with an inhibitor of HER3. In some embodiments, the anti-human OX40 agonistic antibody can be administered in combination with duligotuzumab. In some embodiments, the anti-human OX40 agonistic antibody can be administered in combination with an inhibitor of LSD1. In some embodiments, the anti-human OX40 agonistic antibody can be administered in combination with an inhibitor of MDM2. In some embodiments, the anti-human OX40 agonistic antibody can be administered in combination with an inhibitor of BCL2. In some embodiments, the anti-human OX40 agonistic antibody can be administered in combination with venetoclax. In some embodiments, the anti-human OX40 agonistic antibody can be administered in combination with an inhibitor of CHK1. In some embodiments, the anti-human OX40 agonistic antibody can be administered in combination with CDC-0575. In some embodiments, the anti-human OX40 agonistic antibody can be administered in combination with an inhibitor of the activated hedgehog signaling pathway. In some embodiments, the anti-human OX40 agonistic antibody can be administered in combination with ERIVEDGE.

In some embodiments, the anti-human OX40 agonistic antibody can be administered in combination with radiation therapy. In some embodiments, the anti-human OX40 agonistic antibody can be administered in combination with gemcitabine. In some embodiments, the anti-human OX40 agonistic antibody can be administered in combination with nab-paclitaxel (ABRAXANE). In some embodiments, the anti-human OX40 agonistic antibody can be administered in combination with trastuzumab. In some embodiments, the anti-human OX40 agonistic antibody can be administered in combination with TVECs. In some embodiments, the anti-human OX40 agonistic antibody can be administered in combination with IL27. In some embodiments, the anti-human OX40 agonistic antibody can be administered in combination with cyclophosphamide. In some embodiments, the anti-human OX40 agonistic antibody can be administered in combination with an agent that recruits T cells to the tumor. In some embodiments, the anti-human OX40 agonistic antibody can be administered in combination with lirilumab (IPH2102/BMS-986015). In some embodiments, the anti-human OX40 agonistic antibody can be administered in combination with ldelalisib. In some embodiments, the anti-human OX40 agonistic antibody can be administered in combination with antibodies targeting CD3 and CD20. In some embodiments, the anti-human OX40 agonistic antibody can be administered in combination with REGN1979. In some embodiments, the anti-human OX40 agonistic antibody can be administered in combination with antibodies targeting CD3 and CD19. In some embodiments, the anti-human OX40 agonistic antibody can be administered in combination with blinatumomab.

In some embodiments, the anti-human OX40 agonistic antibody can be administered in combination with an oncolytic virus. In some embodiments, the anti-human OX40 agonistic antibody can be administered in combination with carboplatin and nab-paclitaxel. In some embodiments, the anti-human OX40 agonistic antibody can be administered in combination with carboplatin and paclitaxel. In some embodiments, the anti-human OX40 agonistic antibody can be administered in combination with cisplatin and pemetrexed. In some embodiments, the anti-human OX40 agonistic antibody can be administered in combination with cisplatin and gemcitabine. In some embodiments, the anti-human OX40 agonistic antibody can be administered in combination with FOLFOX. In some embodiments, the anti-human OX40 agonistic antibody can be administered in combination with FOLFIRI.

The combination therapies noted above include combined administration (where two or more therapeutic agents are contained in the same formulation or separate formulations), and separate administration. In this case, the administration of the antibodies of the present invention can occur prior to, concurrently with, and/or subsequent to administration of an additional therapeutic agent and/or adjuvant. The antibodies of the present invention may also be used in combination with radiation therapy.

The antibodies of the present invention (and any other therapeutic agent) can be administered by any suitable means, including parenteral, intrapulmonary, and intranasal, and if desired for local therapy, intralesional administration. Parenteral infusions include intramuscular, intravenous, intraarterial, intraperitoneal, and subcutaneous administration. Depending in part on whether the administration is in a short term or a long term, dosing may be by any suitable route (for example, by injection, such as intravenous or subcutaneous injection). Various dosing schedules are contemplated herein, including, but not limited to, single administration, multiple administrations over multiple time points, bolus administration, and pulse infusion.

The antibodies of the present invention can be formulated, dosed and administered in a manner consistent with good medical practice. Factors to be considered in this context include the particular condition being treated, the particular mammal being treated, the clinical condition of the individual patient, the cause of the condition, the site of drug delivery, the method of administration, the administration schedule and other factors known to the medical practitioner. The antibodies do not need to be, but are optionally, formulated with one or more agents currently used to prevent or treat the disorder in question. The effective amount of such other agents depends on the amount of antibody present in the formulation, the type of disorder or treatment, and other factors described above. They are generally used at the same dosages and in the same routes of administration as described herein, or at about 1% to 99% of the dose described herein, or at any dose and any route determined empirically/clinically to be appropriate.

For the prevention or treatment of disease, the appropriate dose of an antibody of the invention (when used alone or in combination with one or more other therapeutic agents) may depend on the type of disease to be treated, the type of antibody, the severity of the disease and the course of the disease, whether the antibody is administered for prophylactic or therapeutic purposes, previous therapy, the patient's clinical history and response to the antibody, and the discretion of the attending physician. The antibodies are suitable for administration to a patient in one or a series of treatments. Depending on the type and severity of the disease, about 1 µg/kg to about 40 mg/kg of the antibody can be administered to a patient as an initial candidate dose by, for example, one or more divided administrations or by continuous infusion. A typical daily dose may range from about 1 µg/kg to about 100 mg/kg or more, depending on the factors described above. For repeated administration over several days or longer, depending on the condition, the treatment is usually continued until the desired suppression of disease symptoms occurs. The doses may be administered intermittently, for example, weekly or every three weeks (for example, such that the patient receives from about 2 to about 20 doses, or for example, about 6 doses of the antibody). A higher initial dose can be administered, followed by one or more lower doses. However, other dose regimens may also be useful. The progress of the therapy can be easily monitored by conventional techniques and assays.

It is appreciated that any of the above formulations or therapeutic methods may be practiced using the immunoconjugates of the present invention in place of or in addition to an anti-OX40 antibody.

In some embodiments, an intravenous (IV) infusion of the anti-OX40 antibody disclosed herein can be administered to a patient every 3, 4, 5, 6, 7, or 8 weeks. The dose of the anti-OX40 antibody can be 5 mg, 10 mg, 15 mg, 30 mg, 50 mg, 100 mg, 150 mg, 200 mg, 300 mg, or 500 mg per patient. In some embodiments, the patient may have renal cell carcinoma, hepatocellular carcinoma, or head and neck squamous cell carcinoma.

In some embodiments, the anti-OX40 antibody can be administered to a patient in a dose and dosing regimen that achieves one or more of the following:
Desired changes in immune cells in the patient's blood and tumor microenvironment (TME), such as:
- Proliferation (for example, Ki67) and activation (for example, CD69 and CD25) markers on CD4+ or CD8+ T cells measured by flow cytometry on peripheral blood from the patient;
- OX40 receptor level and occupancy level of the anti-OX40 antibody on CD4+ CD25+ T cells measured by flow cytometry on peripheral blood from the patient; and
- Target engagement (for example, OX40 downregulation), CD8+ and CD4+ T cell proliferation (for example, Ki67 upregulation) measured on matched pre- and post-treatment tumor biopsies from the patient by means of multiplex immunohistochemistry (mIHC) or multiplex immunofluorescence (mIF).

In some embodiments, the dose and frequency of administration of an anti-OX40 antibody herein is selected or determined in order to achieve an exposure profile for the patient that ensures an adequate peak-to-trough ratio and minimizes accumulation upon repeated dosing, thereby preventing prolonged receptor saturation and subsequent T cell depletion, and resulting in sustained immune-mediated antitumor activity in the patient.

In some embodiments, certain pharmacodynamic (PD) findings, such as desired changes in immune cells and/or TME of a patient as described above, are assessed along with the exposure profile to select or determine the dose and/or frequency of administration of the anti-OX40 antibody.

### Example

### Example 1. Preparation of anti-OX40 antibody HFB10-1E1hG1

The nucleotide sequences encoding the heavy and light chains of the synthetic anti-OX40 antibody HFB10-1E1hG1 (SEQ ID NOs: 24 and 32) (Goldwisdom Corporation) were synthesized; they were cloned into the vector pFUSE respectively; both the heavy and light chain-containing plasmids were transiently co-transfected at a ratio of 1:1 into 293F suspension cells with PEI to express the full-length antibody. The purification was performed using Superdex^{™} 200 Increase prepacked columns with the AKTA system after 1 week of culture.

### Example 2. Binding properties of HFB10-1E1hG1

### Example 2.1 Binding properties of HFB10-1E1hG1 with the OX40 protein

For pre-test ELISA, 96-well plates were coated overnight with 5 µg/ml of an anti-OX40 antibody, including reference 1, reference 2, reference 3, reference 4, HFB10-1E1hG1 and an isotype control. The next day, the plates were blocked with 1% BSA (Sangon Biotech, cat. no. A600332-0100) in PBST for 2 hours at 37°C before adding a biotinylated OX40 recombinant protein with a defined concentration(s).

The EC80 of biotinylated OX40 recombinant protein binding to anti-OX40 antibody was measured. The EC80 of reference 1 was 0.09 nM; the EC80 of reference 2 was 0.22 nM; the EC80 of reference 3 was 0.16 nM; EC80 of reference 4 was 0.24 nM; the EC80 of HFB10-1E1hG1 was 0.71 nM; and the EC80 of OX40L was 1.6 nM, as shown in Fig. 1-1.

### Example 2.2 Binding properties of HFB10-1E1hG1 to OX40 protein expressed on the surface of 293T cells

To overexpress human-OX40 (Sinobiological, Cat. HG10481-UT), cynomolgus-OX40 (Sinobiological, Cat. CG90846-UT), mouse-OX40 (Sinobiological, Cat. MG50808-UT) or human CD40 (Sinobiological, Cat. HG10774-UT) were used as the target. DNA plasmids encoding these targets were transiently transfected into 293T cells according to the instructions of Lipusectamine LTX Reagent and PLUS Reagent (Thermo, Cat. 15338100). 48 hours after the transfection, the cells were harvested for future use. To determine the binding affinity, the harvested cells were incubated with defined concentrations of primary antibody HFB10-1E1hG1 for 1 hour at 4°C. Then, after washing twice with PBS, the cells were incubated with a secondary antibody goat anti-human IgG PE (1:200; Abcam, Cat. ab98596) for 30 minutes at room temperature. The detection was performed with a Beckman CytoFLEX flow cytometer.

The EC50 for binding of HFB10-1E1hG1 to human OX40 protein expressed on the surface of 293T cells was 2 nM (MFI), as shown in Fig. 1-2.

The EC50 for binding of HFB10-1E1hG1 to cynomolgus monkey OX40 protein expressed on the surface of 293T cells was 2.9 nM (MFI), as shown in Fig. 1-3.

HFB10-1E1hG1 did not bind mouse OX40 protein (MFI) expressed on the surface of 293T cells, as shown in Fig. 1-4.

HFB10-1E1hG1 did not bind human CD40 protein (MFI) expressed on the surface of 293T cells, as shown in Fig. 1-5.

The EC50s for the binding of HFB10-1E1hG1, Reference 1, Reference 2, Reference 3 and Reference 4 to human OX40 protein expressed on the surface of 293T cells, respectively, were shown in Fig. 1-6. The EC50 of HFB10-1E1hG1 was 2.02 nM (MFI); the EC50 of reference 1 was 2.67 nM (MFI); the EC50 of reference 2 was 4.17 nM (MFI); the EC50 of reference 3 was 1.92 nM (MFI); and the EC50 of reference 4 was 2.11 nM (MFI).

The EC50s for the binding of HFB10-1E1hG1, reference 1, reference 2, reference 3 and reference 4 to cynomolgus monkey OX40 protein expressed on the surface of 293T cells, respectively, were shown in Fig. 1-7. The EC50 of HFB10-1E1hG1 was 2.94 nM (MFI); the EC50 of reference 1 was 2.91 nM (MFI); the EC50 of reference 2 was 6.13 nM (MFI); the EC50 of reference 3 was 2.57 nM (MFI); and the EC50 of reference 4 was 3.54 nM (MFI).

### Example 3. Agonistic activity of HFB10-1E1hG1

### Example 3.1 Agonistic activity of HFB10-1E1hG1 in Jurkat reporter cells

Anti-OX40 antibodies can be divided into two categories according to their different ways of activating, the first class of OX40 agonistic activity is independent of the Fc receptor cross-linking; the other class requires Fc receptor cross-linking for OX40 agonistic activity. Tumor tissue and surrounding draining lymph nodes have more tumor-associated inflammatory cells. The Fc receptor FcγR2b is more likely to be clustered around tumor cells. Therefore, "cross-linked antibody" agonists have higher tissue selectivity. Only in the tumor microenvironment can the antibody have a significant agonistic effect, while in the normal tissue parts of the body, the effect will remain at a low level. This can improve the safety window for treatment.

Recombinant Jurkat reporter cells expressing the GFP gene under the control of the NF-kb response element with constitutive expression of human OX40 were used in this study. To determine the agonistic activity of HFB10-1E1hG1, 96-well plates were coated overnight with 5 µg/ml anti-human IgG Fc specific antibody (Sigma, Cat. SAB3701275). Defined concentrations of HFB10-1E1hG1 was added to one well along with 1 × 10⁵ Jurkat reporter cells. In another experiment, 50 nM OX40L (Acrobiosystems, Cat. OXL-H52Q8) was added along with HFB10-1E1hG1 to determine the cooperative effect. After 24 hours of incubation, the Jurkat reporter cells were harvested. In addition, a Beckman CytoFLEX flow cytometer was used to detect GFP-positive signals to indicate the agonistic activity of HFB10-1E1hG1 in the Jurkat reporter cells.

The agonistic activity of HFB10-1E1hG1 is anti-human IgG cross-linking dependent. The EC50 of HFB10-1E1hG1 was 2.9 nM (MFI of GFP) in the presence of cross-linking with anti-human IgG, as shown in Fig. 2-1. Without cross-linking with anti-human IgG, the EC50 of HFB10-1E1hG1 was much greater than that when it was cross-linked with anti-human IgG, as shown in Fig. 2-2. Trimeric OX40L recombinant protein alone could activate NF-kb signaling at EC50=45 nM (MFI of GFP) without cross-linking with anti-human IgG, as shown in Fig. 2-2.

HFB10-1E1hG1 showed a cooperative agonistic effect with OX40L in the presence of cross-linking with anti-human IgG. The addition of HFB 10-1E1hG1 together with OX40L enhanced the MFI of GFP as compared to the component alone, as shown in Fig. 2-3.

### Example 3.2 Agonistic activity of HFB10-1E1hG1 in primary CD4+ T cells

To determine the agonistic activity of HFB10-1E1hG1 in primary CD4+ T cells, 96-well plates were coated overnight with 0.3 µg/ml or 1 µg/ml anti-CD3 antibody (Thermo, Cat. 16-0037-81) and 5 µg/ml anti-human IgG Fc specific (Sigma, Cat. SAB3701275). The next day, the primary CD4+ T cells were harvested according to the instructions of the CD4+ T cell isolation kit (Miltenyi, Cat. 130-045-101). Defined concentrations of HFB10-1E1hG1 and 2 µg/ml anti-CD28 antibody (Thermo, Cat. 16-0289-81) were added to one well along with 1 × 10⁵ primary CD4+ T cells. In another experiment, 50 nM OX40L (Acrobiosystems, Cat. OXL-H52Q8) was added together with HFB10-1E1hG1 to determine the cooperative effect thereof. After 3 days of incubation, the level of IL-2 secretion in the supernatant was detected according to the instructions of human IL-2 DuoSet ELISA kit (R&D, Cat. DY202-05), so as to indicate the agonistic activity of HFB10-1E1hG1 in the primary CD4+ T cells.

To determine the agonistic activity of HFB10-1E1hG1 in primary CD4+ T cells, purified CD4+ T cells were pre-activated with 1 µg/ml anti-CD3 antibody and 2 µg/ml anti-CD28 antibody. The plates were pre-coated with 5 µg/ml anti-human IgG to facilitate the cross-linking with HFB 10-1E1hG1. After three days of incubation, the agonistic activity of HFB10-1E1hG1 in primary CD4+ T cells was determined by IL-2 secretion, yielding an EC50 of 0.2 nM, as shown in Fig. 2-4.

In the primary CD4+ T cells, HFB10-1E1hG1 showed a cooperative agonistic effect with OX40L. Incubation of HFB10-1E1hG1 with 50 nM OX40L enhanced IL-2 secretion as compared to the components alone, as shown in Fig. 2-5.

### Example 4. Pharmacokinetic testing of HFB10-1E1hG1

384-well plates were coated overnight with F(ab')₂ goat anti-human IgG Fc specific antibody (Jackson IR, Cat. 109-006-098) at 1 µg/ml in 30 µl/well PBS. After three washes with PBST buffer, the plates were blocked with a blocking buffer containing 1 mM EDTA, 0.05% Tween and 2% BSA in PBS for 1 hour at 37°C. Then, starting at 1/150, in serial dilutions of 1/3, the mouse serum samples were added at 15 µL/well and incubated at 37°C for 2 hours. After washing three times with a PBST buffer, the secondary antibody peroxidase-goat anti-human IgG (Jackson IR, Cat. 109-035-003) was added at 1/5000 and incubated at 37°C for 0.5 hours. The TMB substrate (Biolegend, Cat. 421101) was then added and incubated for an additional 15 minutes. Next, an ELISA stop solution (Beijing Dingguo Changsheng Biotechnology Co., Ltd.) was added. The plates were read at 450 nm with a Multiskan Sky Microplate Spectrophotometer (ThermoFisher).

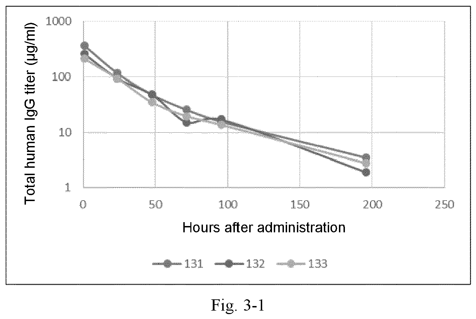

10 mg/kg HFB10-1E1hG1 was administered intravenously to hOX40 knock-in mice (131, 132, 133, purchased from the Shanghai Southern Model Organism Research Center). Mouse sera were collected at 1 hour, 24 hours, 48 hours, 72 hours, 96 hours and 196 hours after administration, respectively. The half-life of HFB10-1E1hG1 in hOX40-KI mouse serum was determined to be 24 hours, as shown in Fig. 3-1. The data points at 0 hours were the data points 131, 132, and 133 from top to bottom.

### Example 5. In vivo antitumor efficacy of HFB10-1E1hG1

hOX40 knock-in mice were purchased from the Shanghai Southern Model Organism Research Center. After being quarantined for 5 days, each mouse was subcutaneously inoculated with 8 × 10⁵ MC38 tumor cells (provided by Professor Zhang Hongkai, Nankai University) in 100 µl PBS. When tumor size reached 70 to 100 mm³, anti-OX40 antibody treatment was initiated by intraperitoneal administration of the anti-OX40 antibody to mice at 10 mg/kg and q3dx5 in 100 µl PBS. The tumor size and mouse body weight were measured twice weekly. A caliper was used to measure the tumor size in both directions. The volume of the tumor was expressed in mm³ using the following formula: V = 0.5a × b², where a and b were the long and short diameters of the tumor, respectively.
Day 7: MC38 tumor cell inoculation, 35 8-week-old mice
   - MC38 cells were obtained from Prof. Zhang Hongkai
   - 8 × 10⁵ MC38 cells were inoculated per mouse
Day 0: Mean tumor size was 75 mm³ (40 to 120 mm³)
   - 5 mice per group, 4 groups in total:
      - PBS
      - Reference antibody 1
      - HFB10-1E1hG1
      - Reference antibody 2
   - Initiated by intraperitoneal administration of 10 mg/kg Q3Dx5 antibody.
Day 18: Tumors in the PBS control group reached 2000 mm³ in size; the mice were sacrificed.
   - Tissues: blood, LN, liver, spleen, tumor
   - Phenotypic analysis: T cells CD3/CD4/CD8; Treg CD4/CD25/Foxp3; NK CD3/CD16/CD56

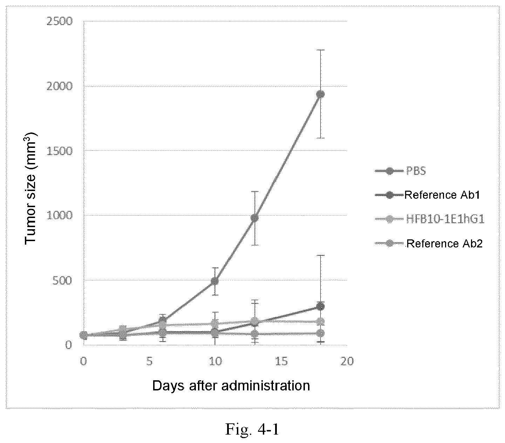

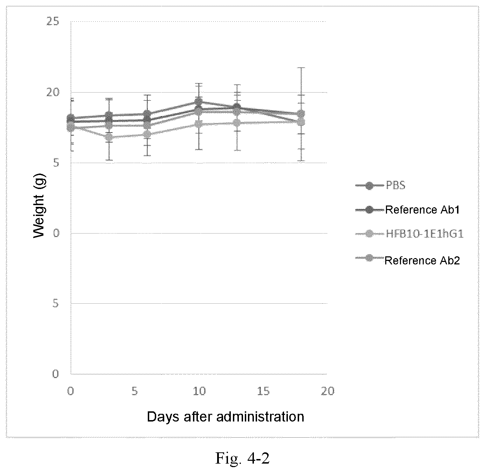

The experiments showed that HFB10-1E1hG1 significantly inhibited tumor growth as compared to the PBS control, and showed no side effects that may result in significant weight loss in mice, as shown in Fig. 4-1 and Fig. 4-2. In Fig. 4-1, the data points on the far right were PBS, reference antibody 1, HFB10-1E1hG1 and reference antibody 2 in an order of from top to bottom. In Fig. 4-2, the second data points from the left were PBS, reference antibody 1, reference antibody 2, and HFB10-1E1hG1 in an order of from top to bottom.

Antibody dose-response efficacy was studied in the hOX40 knock-in mice. The specific groups in the study were as follows:
Group 1: PBS: Q3D x 5, intraperitoneal;
Group 2: HFB10-1E1hG1: 1 mg/kg, intraperitoneal, Q3/4D x 5, intraperitoneal;
Group 3: HFB10-1E1hG1: 0.1 mg/kg, intraperitoneal, Q3/4D x 5, intraperitoneal;
Group 4: Reference antibody 1: 1 mg/kg, intraperitoneal, Q3D x 5, intraperitoneal.

A total of 20 hOX40 knock-in mice were used in the study and were divided into 4 groups, with 5 mice in each group.

Mice were inoculated with the MC38 tumor cells. Five antibody injections were performed on days 0, 3, 6, 10, and 13, starting when the mean tumor size was 75 mm³. The tumor size and mouse body weight were measured twice weekly for up to three weeks or until the tumor size was greater than 2000 mm³.

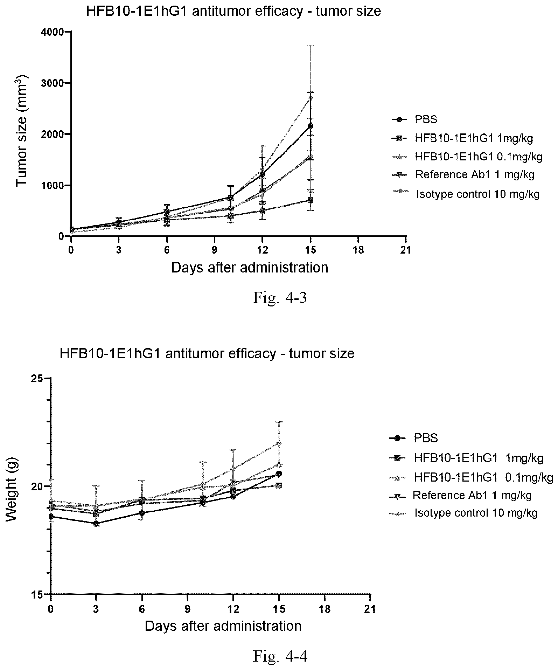

The response results of tumor size and body weight of mice under different doses of HFB10-1E1hG1 were shown in Fig. 4-3 and Fig. 4-4. The experiments showed that HFB10-1E1hG1 at 1 mg/kg exhibited the best antitumor efficacy in vivo.

### Example 6. In vitro characterization of HFB10-1E1hG1 malleability characteristics

### Example 6.1 Accelerated stability experiment of HFB10-1E1hG1

Antibody samples (HFB10-1E1hG1, 3.1 mg/mL, Lot #CP181130004) were concentrated to 10 mg/mL (Lot #JW20181203, Lot #20190624) with a centrifugal filter device (Millipore, Cat #UFC503096). An appropriate amount of the concentrated antibody sample was then transferred to a clean 600 µl tube and incubated at 25°C and 40°C for 7, 14, and 30 days, respectively.

The incubated samples were analyzed by SEC-HPLC and SDS-PAGE:
For SEC-HPLC, 50 µg of each treated sample was injected and tested in 1x PBS buffer at pH 7.4 (diluted with the Milli-Q pure water from a 10x PBS buffer (Sangon, Cat #E607016-0500), at a flow rate of 0.7 mL/minutes for 40 minutes/test). Absorbance was detected at UV 280 nm (untreated samples stored at 4°C were also loaded for analysis). The results were shown in Table 1 below. No significant increase in the aggregation or degradation peaks of HFB10-1E1hG1 was observed on the SEC curve even after incubation at 25°C and 40°C for up to 30 days, indicating that HFB10-1E1hG1 has good stability under such treatment conditions. The slight degradation observed after 30 days of incubation at 40°C may indicate its instability with prolonged incubation at a high temperature.

**Table 1 SEC Analysis - Temperature**

| Antibody | Condition | Aggregation% | Monomer% | Small fragment% |
|---|---|---|---|---|
| HFB10-1E1hG1 | Stored at 4°C | 1.70 | 98.31 | 0 |
| | 25°C, 7 days | 2.01 | 97.99 | 0 |
| | 25°C, 14 days | 1.96 | 98.04 | 0 |
| | 25°C, 30 days | 2.06 | 97.94 | 0 |
| | 40°C, 7 days | 3.99 | 96.00 | 0 |
| | 40°C, 14 days | 3.75 | 96.10 | 0 |
| | 40°C, 30 days | 3.6 | 95.18 | 1.22 |

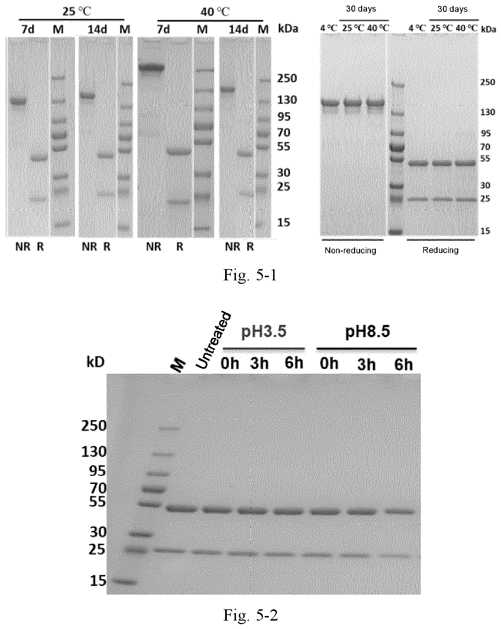

For SDS-PAGE, 4 µg of each treated sample was loaded onto a 4% to 20% gradient gel under non-reducing and reducing conditions, respectively. The gel was run in a tris-glycine buffer at 150 V for 1 hour and stained in a staining solution (TaKaRa, Cat #T9320A) for more than 1 hour, and then destained several times in distilled water and images were taken on a white light plate (untreated samples stored at 4°C were also loaded for analysis). The results were shown in Fig. 5-1.

After incubation at 25°C and 40°C for 30 days, no obvious aggregation or degradation bands of HFB10-1E1hG1 were observed on SDS-PAGE images, indicating that HFB10-1E1hG1 has good stability under such treatment conditions. In addition, after 30 days of incubation at 40°C, the observation of non-reducing gels and slightly degraded bands with the aggregated bands on the reduced gel may indicate its instability with prolonged incubation at a high temperature.

### Example 6.2 Degradation experiment of HFB10-1E1hG1

1. Low pH pressure test:
   An appropriate amount of the antibody (HFB10-1E1hG1, 3.1 mg/mL) was transferred into a 600 µl tube, then 2M acetic acid (v/v of acid to antibody sample, the final pH is adjusted to about 3.5) was added at a ratio of 1:20. The sample was mixed well and incubated at room temperature for 0, 3 or 6 hours. After the incubation, the pH of the antibody solution was adjusted to 7.4 with a neutralization buffer (1M Tris-HCl at pH 9.0 was added to the incubated sample at a ratio of 13:100, v/v). The sample was then analyzed by SEC-HPLC and SDS-PAGE as previously described (see Accelerated Stability Experiments; Note: the untreated sample stored at 4°C was also loaded for analysis).
   2. High pH pressure test:
      An appropriate amount of the antibody (HFB10-1E1hG1, 3.1 mg/mL) was transferred into a 600 µl tube, then 1 M Tris-HCl pH 8.5 (v/v, the final pH was adjusted to about 8.5) was added at a ratio of 1:25. The sample was mixed well and incubated at room temperature for 0 or 6 hours. The sample was then analyzed by SEC-HPLC and SDS-PAGE (under only the reducing conditions) as previously described.

The SEC analysis was shown in Table 2. After incubation in the corresponding pH 3.5 and pH 8.5 solutions for up to 6 hours, no increase in aggregation or degradation peaks of HFB10-1E1hG1 was observed on the SEC curve, indicating that HFB10-1E1hG1 has good stability under such treatment conditions.

**Table 2 SEC Analysis - pH**

| Antibody | Condition | Aggregation% | Monomer% | Small fragment% |
|---|---|---|---|---|
| HFB10-1E1hG1 | Stored at 4°C | 1.47 | 97.57 | 0.96 |
| | pH3.5, 0 h | 1.21 | 97.49 | 1.3 |
| | pH3.5, 3 h | 1.15 | 97.63 | 1.22 |
| | pH3.5, 6 h | 1.25 | 97.62 | 1.13 |
| | pH8.5, 0 h | 2.16 | 97.48 | 0.37 |
| | pH8.5, 6 h | 2.11 | 97.53 | 0.36 |

SDS-PAGE analysis was shown in Fig. 5-2. After incubation in low pH and high pH buffers for up to 6 hours, no change in HFB10-1E1hG1 was observed on the SDS-PAGE gel, indicating good stability of HFB10-1E1hG1 under such processing conditions.

### Example 6.3 Oxidative stress experiment of HFB10-1E1hG1

An appropriate amount of the antibody (HFB10-1E1hG1, 3.1 mg/mL) was transferred into a 600 µl tube, then H₂O₂ (0.1% and 1%, respectively) or t-BHP (a final concentration of 0.1%) was added. The sample was mixed well and incubated at room temperature for 0 or 6 hours. The sample was then analyzed by SEC-HPLC and SDS-PAGE as previously described.

Note: 1) The untreated sample stored at 4°C was loaded for analysis; 2) The SEC running buffer was prepared in-house, which contained 100 mM NaH₂PO4, 150 mM NaCl, at pH 6.8.

The SEC analysis was shown in Table 3. After 6 hours of incubation in the corresponding solutions of 0.1% H₂O₂, 1% H₂O₂ and 0.1% t-BHP (tert-butyl hydroperoxide), no apparent change in HFB10-1E1hG1 was observed on the SEC curve, indicating that HFB10-1E1hG1 had good stability under such treatment conditions.

**Table 3 SEC Analysis - Oxidative Stress**

| Antibody | Condition | Aggregation% | Monomer% | Small fragment% |
|---|---|---|---|---|
| HFB10-1E1hG1 | Stored at 4 °C | 2.99 | 96.89 | 0.12 |
| | 0.1% H₂O₂, 0 h | 2.75 | 97.11 | 0.14 |
| | 0.1% H₂O₂, 6 h | 2.78 | 97.05 | 0.17 |
| | 1% H₂O₂, 0 h | 2.81 | 96.99 | 0.20 |
| | 1% H₂O₂, 6 h | 2.56 | 97.13 | 0.31 |
| | 0.1% t-BHP, 0 h | 3.02 | 96.81 | 0.18 |
| | 0.1% t-BHP, 6 h | 2.82 | 96.96 | 0.23 |

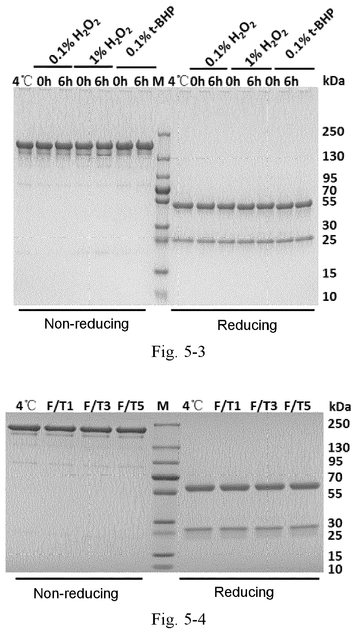

The SDS-PAGE analysis was shown in Fig. 5-3. After 6 hours of incubation in the corresponding solutions of 0.1% H₂O₂, 1% H₂O₂ and 0.1% t-BHP (tert-butyl hydroperoxide), no apparent change in HFB10-1E1hG1 was observed on the SDS-PAGE gel, except for the slightly degraded bands on the non-reducing gel. This indicates that HFB10-1E1hG1 has good stability under such processing conditions.

### Example 6.4 Freeze-thaw experiment of HFB10-1E1hG1

The antibody sample (HFB10-1E1hG1, 3.1 mg/mL, Lot #CP181130004) was concentrated to 10 mg/mL (Lot #JW20181203) with a centrifugal filter device (Millipore, Cat #UFC503096). Next, an appropriate amount of concentrated antibody sample was transferred to a clean 600 µl tube (3x tube), the sample was frozen in liquid nitrogen for 2 minutes, and then thawed in a water bath at room temperature, and the same procedure was further performed 2, 4 or more times.

Note: 1) The untreated sample stored at 4°C was loaded for analysis; 2) The SEC running buffer was prepared in-house, which contained 100 mM NaH₂PO₄, 150 mM NaCl, at pH 6.8.

For DSF-based thermal stability analysis: 3 µg of each treated sample was used with a ProteoStat assay kit (Enzo Life Sciences, Cat #ENZ-51027-K400) in a PCR plate (Bio-Rad plate, Cat#HSP9655; Bio-Rad membrane, Cat#MSB1001) in each 25 µl reaction according to the manufacturer's instructions. The heating program was set up on a Bio-Rad PCR machine (C1000 touch, CFX96 real-time system) as follows: 25°C for 2 minutes, increasing by 0.5°C to 95°C every 10 seconds. Fluorescence absorbance was read using the mode of Texas Red. The Tm value was related to the lowest point of -dF/dT.

The SEC analysis was shown in Table 4. The DSF analysis was shown in Table 5. The SEC analysis showed that no obvious change in HFB10-1E1hG1 was observed on the SEC curve after up to 5 cycles of freeze/thaw treatment. The DSF analysis showed that the Tm value of HFB10-1E1hG1 did not change significantly after the same treatment, indicating that HFB10-1E1hG1 had good stability under such treatment conditions.

**Table 4 SEC Analysis - Freeze Thaw**

| Antibody | Condition | Aggregation% | Monomer% | Small fragment% |
|---|---|---|---|---|
| HFB10-1E1hG1 | Stored at 4 °C | 3.15 | 96.85 | 0 |
| | F/T cycle 1 | 3.03 | 96.97 | 0 |
| | F/T cycle 3 | 3.03 | 96.96 | 0 |
| | F/T cycle 5 | 2.95 | 97.05 | 0 |

**Table 5 DSF Analysis - Freeze Thaw**

| Antibody | Condition | Tm (°C) |
|---|---|---|
| HFB10-1E1hG1 | Stored at 4 °C | 74.5 |
| | F/T cycle 1 | 74.5 |
| | F/T cycle 3 | 74.25 |
| | F/T cycle 5 | 74.25 |

SDS-PAGE analysis was shown in Fig. 5-4. No apparent change in HFB10-1E1hG1 was observed on SDS-PAGE gels after up to 5 cycles of freeze/thaw treatment, indicating good stability of HFB10-1E1hG1 under such treatment conditions.

### Example 7

Binding of agonistic antibodies to OX-40 has been shown to result in receptor downregulation, but this was observed in vitro and in clinical trials (Wang et al. Cancer Research 2019). It had been further hypothesized that the consequent loss of target expression observed in patients after the first injection could limit the success of OX-40 agonistic antibodies in clinical trials (Wang et al. Cancer Research 2019). By using ex vivo activated naive T cells isolated from PBMCs, the present invention shows that the treatment with HFB10-1E1hG1 resulted in less post-treatment reduction on the OX-40 level as compared to the baseline. Unique binding epitopes along with optimized binding kinetics minimize the target degradation, thereby avoiding loss of target expression after the first injection. It thus allows continuous administration of the drug to the patient.

### Example 8

### Human pharmacokinetic (PK) prediction of HFB10-1E1hG1

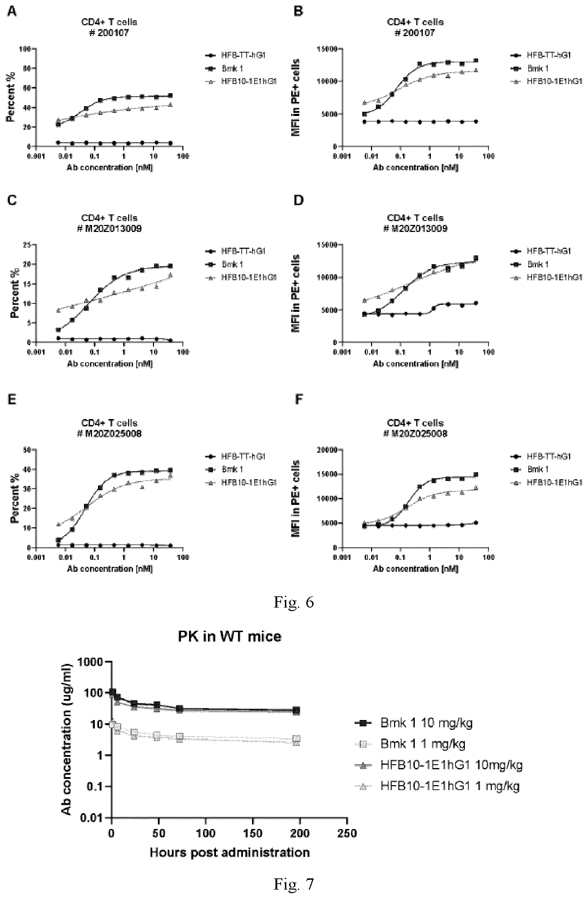

The PK data in cynomolgus monkeys were fitted to a 2-compartment model. Fig. 6 showed the fitted PK curves and observed data. The RMSE of fitting was 0.17 and the R² was 0.99, indicating a good fit to the observed data.

Human 2-compartment PK parameters were predicted from the corresponding monkey parameters based on the Rule of Exponent (ROE) (Dong et al., 2011).

The human PK curves were then simulated using the predicted parameters. Fig. 7 showed an example of a single-dose human PK profile of 1 mg/kg following a 1 hour intravenous infusion.

### Lowest human PAD prediction based on HFB10-1E1hG1 PK data in hOX40 KI mice

The Cmax and AUC (0 to 72 h) values of HFB10-1E1hG1 at a dose of 10 mg/kg in hOX40 KI mice were 160.5 µg/mL and 2591 µg/mL^{∗}h, respectively. Assuming it was linear PK, the Cmax and AUC (0-72h) values at the 1 mg/kg dose were 16 µg/mL and 259 µg/mL^{∗}h, respectively.

At the dose of 1 mg/kg, the predicted human Cmax and AUC (0 to 72 h) values were 23.7 µg/mL and 1213 µg/mL^{∗}h, respectively. Assuming it was linear PK in humans, the lowest PAD was predicted to be 0.68 mg/kg (based on Cmax) or 0.21 mg/kg (based on AUC (0 to 72 h)). To be conservative, the minimum human PAD was assumed to be a fixed dose of 0.21 mg/kg or 15 mg (assuming a standard body weight of 70 kg).

### Considerations for dosing frequency in humans

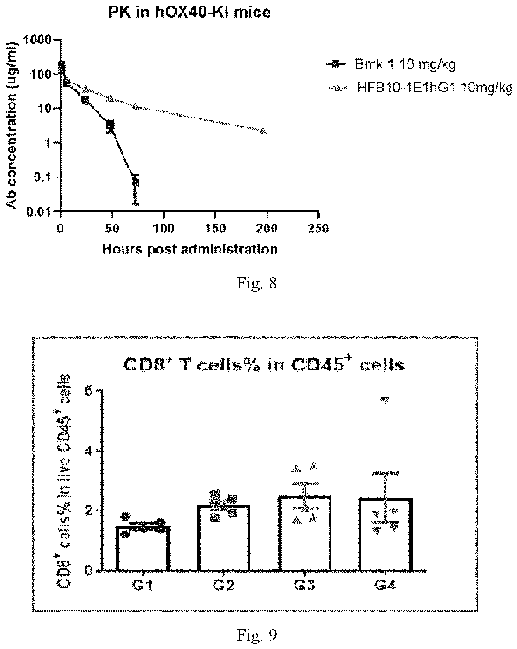

To maximize the OX40 agonism and the subsequent expansion of effector T cells and depletion of regulatory T cells in the tumor microenvironment, it should be taken in account not to saturate the OX40 receptor for a prolonged period of time. One strategy to ensure adequate time between OX40 agonism is to avoid significant accumulation of the agonist of interest. We thus simulated the PK profile of HFB10-1E1hG1 on a schedule of every 2 weeks (Q2W), 3 weeks (Q3W), or 4 weeks (Q4W). Fig. 8 showed the simulated PK curves at Q4W.

We then calculated the accumulation indices of Cmax and Cmin for each schedule, as well as the Cmax/Cmin ratio at steady state.

In addition to practical considerations, the 15 mg starting dose and Q4W schedule were selected for the first-in-human study of HFB10-1E1hG1 based on the calculated AI and Cmax/Cmin ratio.

The human PK of HFB10-1E1hG1 was predicted to be similar to typical IgG1 monoclonal antibodies (mAbs), with low clearance (CL, 0.084 mL/h/kg), low volume of distribution (Vdₛₛ, 0.084 L/kg) and typical mAb half-life (T_{1/2}, 731 h, about 30 days).

The lowest human PAD for HFB10-1E1hG1 was predicted based on the human PK predictions from cynomolgus PK, the minimum effective doses from MC38 tumor efficacy studies in hOX40 KI mice, and the HFB10-1E1hG1 exposure data in hOX40 KI mice. Extrapolation of the KI mouse exposure/potency relationship to humans showed a minimum PAD of 0.21 mg/kg in humans. For convenience, an equivalent fixed dose of 15 mg is recommended as the starting dose in humans. Based on the predicted steady-state PK profiles at different dosing frequencies, the recommended dosing frequency is once every 4 weeks (Q4W).

We herein assessed the binding of HFB10-1E1hG1 to the activated primary monkey T cells using flow cytometry. The results were shown in Fig. 1.

Bmk 1 that can recognize CD4+ T cells was used as a positive control. HFB10-1E1hG1 was shown to bind to the activated CD3/CD28 primary monkey T cells isolated from three different donors (Lot # 200107, M20Z013009 and M20Z025008). The EC50 values calculated from the MFI dose-response curves were 0.09 nM, 0.19 nM and 0.17 nM, respectively.

The linear detection range of the ELISA assay was between 125-1000 pg/ml in experiment 20200730 and 125-2000 pg/ml in experiment 20200731, respectively. The LLOQ in both experiments was 18.75 ng/ml.

We herein assessed the pharmacokinetics of Bmk 1 and HFB10-1E1hG1d by i.v. administration in single doses of 10 mg/kg and 1 mg/kg in WT mice and in hOX40-KI mice using a single dose of 10 mg/kg. Plots of mean concentrations of Bmk 1 and HFB10-1E1hG1 versus time were shown in Fig. 7 and 8 on a linear and semi-log scale.

Systemic exposure of HFB10-1E1hG1 and Bmk1 was achieved in all treated mice. In WT mice (Fig. 7), HFB10-1E1hG1 and Bmk 1 exhibited similar linear clearance with dose proportionality. The dose ratio of HFB10-1E1hG1 to Bmk 1 was 1:10, while the ratios of Cmax and AUC (0-72h) of HFB10-1E1hG1 were 1:6.8 and 1:7.9, respectively; and the dose ratio of Bmk 1 were 1:11.8 and 1:8.6, respectively.

The serum clearance of HFB10-1E1hG1 at 1 mg/kg and 10 mg/kg was 0.71 ± 0.34 and 1.06 ± 0.77 ml/h/kg in wild-type mice, respectively, while that in hOX40- 3.09±0.27 ml/h/kg in KI mice. Similar differences in serum clearance were also observed in Bmk 1 PK. The serum clearance of Bmk 1 in wild-type mice was 0.45±0.13 and 0.62±0.10 ml/h/kg at 1 mg/kg and 10 mg/kg, respectively; while it was 7.24±2.24 ml/h/kg at 10 mg/kg in hOX40-KI mice. Since hOX40 is only expressed in hOX40-KI mice, the higher clearance rate in hOX40-KI mice may be due to target-mediated drug disposition (TMDD).

The steady-state volume of distribution (Vdss) values of HFB10-1E1hG1 at 1 mg/kg and 10 mg/kg were 0.21 ± 0.03 and 0.26 ± 0.02 L/kg in wild-type mice, while at 10 mg/kg, it was 0.14±0.01 L/kg in hOX40-KI mice. Similarly, the Vdss values of Bmk 1 were 0.19±0.02 and 0.21±0.01 L/kg in wild-type mice, and 0.08±0.03 L/kg in hOX40-KI mice.

Significant differences in T1/2were also observed between WT and hOX40 KI mice for these two antibodies. In wild-type mice, the terminal half-lives (T_{1/2}) of HFB10-1E1hG1 at 10 mg/kg and 1 mg/kg were 239.72±140.48 h and 274.52±193.12 h, respectively. The terminal half-lives of Bmk1 at 10 mg/kg and 1 mg/kg were 252.04±51.82 h and 321.05±75.89 h, respectively. For HFB10-1E1hG1 and Bmk 1 at a dose of 10 mg/kg, the T1/2 values thereof were significantly shorter in hOX40 KI mice, which were 38.65±4.84 h and 5.45±1.09 h, respectively.

### Example 9. Pharmacodynamic studies

Method: Thirty female human OX40 knock-in mice (C57BL/6 background) were subcutaneously inoculated with MC-38 tumor cells on the right side thereof for tumor growth. Nine days after tumor inoculation, 20 mice with tumor sizes of 76 to 226 mm³ (mean tumor size of 155 mm³) were selected and divided into 4 groups with 5 mice in each group based on their tumor volume with stratified randomization. Treatment from the day of randomization (defined as Day 0 (D0)) included: isotype control, 10 mg/kg; HFB10-1E1hG1, 0.1 mg/kg; HFB10-1E1hG1, 1 mg/kg; and HFB10-1E1hG1, 10 mg/kg. All treatments were administered by i.p. injection on D0, D3 and D7. Tumor size and animal body weight were measured at least three times a week. Six hours after the third dose, tumor samples were collected for flow cytometry (FCM) analysis, blood samples were collected for receptor occupancy (RO) analysis, and plasma samples were sent to HiFiBiO.

Results: The PD effect of HFB10-1E1hG1 was tested in MC-38 tumor-bearing hOX40 KI mice. In both the blood and tumor microenvironment (mainly in tumors), HFB10-1E1hG1 resulted in a decrease in OX40 expression represented as a percentage of positive and MFI on T cells, mainly on CD4⁺ T cells. At the same time, HFB10-1E1hG1 also significantly reduced the Treg population. In the tumor microenvironment, the proportion of CD4⁺ T cells was reduced, mainly driven by the reduction of Tregs (CD25⁺ FOXP3⁺ cells); the proliferation of CD4⁺ T cells (Ki67⁺ cells) was increased while CD69⁺ T cells were decreased. The PD1 expression is reduced for CD8⁺ T cells in the tumor microenvironment. All of these observations were dose-dependent in the HFB10-1E1hG1-treated group. Taken together, the foregoing data suggested that HFB10-1E1hG1 treatment can activate the OX40 signaling pathway, leading to observations in the tumor microenvironment that may contribute to durable responses in efficacy studies.

The effect of HFB10-1E1hG1 on OX40 expression in blood samples was analyzed using a mouse anti-hOX40 antibody (clone ACT35) that was uncompetitive for HFB10-1E1hG1. The total OX40 expression on CD4⁺, CD4⁺CD25⁺ and CD11b⁺ monocytes was measured (data not shown). HFB10-1E1hG1 treatment resulted in a significant downregulation of OX40 expression (percent positive and MFI) for CD4⁺ and CD4⁺CD25⁺ T cells, including Tregs. No changes in OX40 expression on CD11b⁺ cells were observed.

To assess target engagement following HFB10-1E1hG1 treatment, anti-human IgG (hIgG) was used in combination with non-competing Ab ACT35 in FCM analysis. The percentage of hIgG⁺ population on OX40⁺ CD4⁺ T cells and OX40⁺ CD4⁺ CD25⁺ T cells was low. However, the hIgG⁺ signal expressed as MFI on OX40⁺ CD4⁺ CD25⁺ cells (including Treg) demonstrated a significant dose-dependent increase in HFB10-1E1hG1.

On OX40⁺ CD11b⁺ cells, a high level of hIgG⁺ signal could be observed in the isotype group and in the group of 10 mg/kg HFB10-1EhG1 treatment. It correlated with the treatment dose, indicating that hIgG was captured by FcgRs on CD11b⁺ cells.

Infiltrating immune cell populations in tumor tissues were analyzed by flow cytometry ("FCM"). For all FCM analysis plots in this example:
- G1: isotype control group; G2: HFB10-1E1hG1 0.1 mg/kg treatment group; G3: HFB10-1E1hG1 1 mg/kg treatment group; G4: HFB10-1E1hG1 10 mg/kg treatment group
- Each dot represented data from an individual animal, rectangular bars represented group means, and error bars represented SEM.

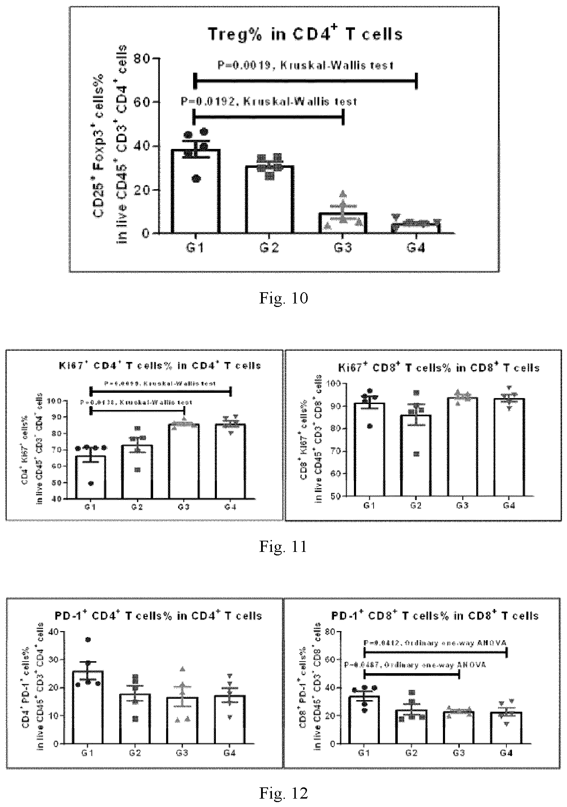

The percentages of T cells (CD3⁺), CD4⁺ T, CD8⁺ T cells and Treg (CD3⁺ CD4⁺ CD25⁺ Foxp3⁺) were measured and the selected results were shown in Fig. 9 and 10. Naive T cells (CD3⁺CD44-CD62L⁺), memory T cells (CD3⁺CD44⁺CD62L⁺) and effector T cells (CD3⁺CD44⁺CD62L-) were also measured (data not shown). Under the current experimental conditions, compared with the isotype control group (G1), the groups treated with HFB10-1E1hG1 (G3 and G4, but not G2) showed significantly reduced percentages of CD4⁺ T cells, Tregs, naive T cells and naive CD4⁺ T cells. Moreover, all HFB10-1E1hG1 treatments resulted in a significant reduction in the percentages of memory T cells and memory CD4⁺ T cells. While all HFB10-1E1hG1-treated groups showed a decrease in the percentage of memory CD8⁺ T cells, only G2 and G3 reached statistical significance. No significant changes were observed in T cell (CD3⁺), CD8⁺ T cell and effector T cell populations between the treatment groups.

The effects of HFB10-1EhG1 treatment on OX40 expression, activation and proliferation of different T cell subtypes in tumors were further investigated. The effect of HFB10-1E1hG1 on total OX40 expression was analyzed using a mouse anti-hOX40 antibody (clone ACT35) that was uncompetitive for HFB10-1E1hG1. OX40 expression on CD3⁺, CD4⁺, CD8⁺ T cells and Treg (CD4⁺ CD25⁺ FoxP3⁺) was measured (data not shown). The OX40 expression on CD3⁺ and CD4⁺ T cells was significantly reduced in both percentage and MFI in the HFB10-1E1hG1-treated group as compared to the isotype control group (G1). For Tregs, HFB10-1E1hG1 treatment did not significantly reduce the OX40 expression represented as a percentage, but significantly reduced the OX40 expression represented by MFI (data not shown). On CD8⁺ T cells in the HFB10-1E1hG1-treated group, the OX40 expression represented as a percentage was significantly reduced, but the reduction in MFI did not reach a significant difference.

The treatment with HFB10-1E1hG1 (G3 and G4) resulted in significantly lower percentages of CD69⁺CD4⁺ T cells and PD-1⁺CD8⁺ T cells as compared to the isotype control group (G1) (Fig. 12). The same treatment also resulted in a significant increase in the percentage of proliferating (Ki67⁺) CD4⁺ T cells (Fig. 11). All of these observations were dose-dependent in the HFB10-1E1hG1 treated group.

### Example 10: Epitope Mapping

The monoclonal anti-OX40 antibody HFB301001 was developed as an agonistic antibody. Table 1 showed the CDR, VH/VL and HC/LC sequences of HFB301001.

Binding epitopes were characterized by epitope binning using a competitive ELISA assay to explore OX40 binding epitopes of different anti-OX40 antibodies relative to previously published selected antibodies and OX40 ligand binding epitopes. Briefly, plates were coated with 50 µl of 1 µg/ml anti-OX40 capture antibody, OX40L or isotype control, and incubated overnight at 4°C. The plates were then blocked with 1% BSA in PBST for 2 hours at room temperature. Next, 50 µl/well of Biotin-OX40 (Lot #160921111, ChemPartner; Tokyo, JP) was added. The biotin-OX40 was diluted 1 to 3 times at 8 points starting from 0.5 µg/mL, and then incubated at 37°C for 1 hour. HRP-conjugated streptavidin (1/5000 dilution; 50 µl/well) was then added and incubated for 1 hour at 37°C, then 100 µl/well of TMB was added and incubated for 15 minutes at room temperature. Reactions were terminated by adding 50 µl/well of an ELISA stop solution. The OD values were determined at a wavelength of 450 nm using a Thermo Multiscan sky spectrophotometer.

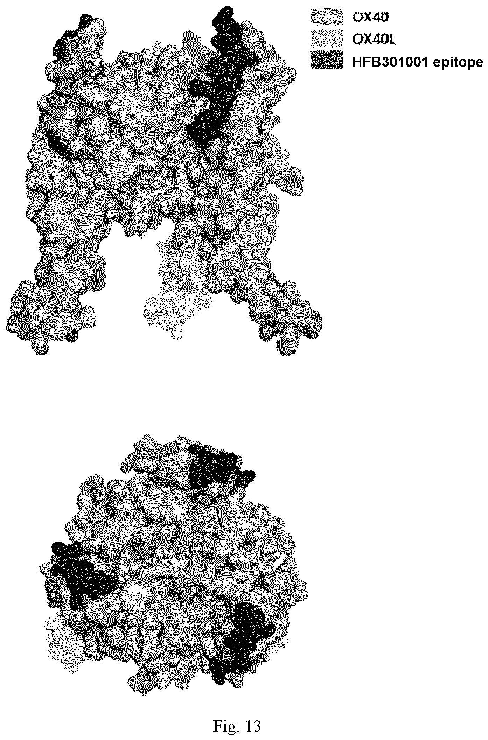

More detailed resolution of bound epitopes was obtained using hydrogen deuterium exchange mass spectrometry. Briefly, H/D exchange epitope mapping was performed using mass spectrometry (HDX-MS) to determine the amino acid residues of OX40 (recombinant human OX40) that interact with HFB301001. A general description of the H/D exchange method was described, for example, in Ehring (1999) Analytical Biochemistry 267(2):252-259; and Engen and Smith (2001) Anal. Chem. 73:256A-265A. The His-tagged OX40 antigenic protein was purchased from Sinobiological (Cat. No. 10481-H08H; Sinobiological, Inc.; Beijing, CN). The HDX-MS experiments were performed on an integrated HDX/MS platform for peptide mass measurement provided by Novabioassays LLC and a Thermo Q Exactive HF mass spectrometer. 4.5 µL of each sample were incubated with 75 µL of a deuterium oxide labeling buffer (IX PBS, pD 7.4) at 20°C for 300 s, 600 s, 1800 s, and 3600 s. The hydrogen/deuterium exchange was Quenched by adding 80 µL of a buffer with 4 M guanidine hydrochloride and 0.85 M TCEP (final pH 2.5). The quenched samples were then subjected to on-column pepsin/protease XIII digestion followed by LC-MS analysis. The mass spectra were recorded in MS-only mode. Pepsin/Protease XIII digestion, LC-MS and data analysis were then performed. After HDX labeling, the samples were denatured by adding 80 µL of the buffer with 4 M guanidine hydrochloride and 0.85 M TCEP (final pH 2.5), and then incubating at 20°C for 3 minutes. The mixture was then subjected to on-column pepsin/protease XIII digestion using an internal packed pepsin/protease XIII (w/w, 1:3) column. The resulting peptides were analyzed using a UPLC-MS system composed of a Waters Acquity UPLC coupled to a Q Exactive^{™} plus Hybrid Quadrupole-Orbitrap mass spectrometer (Thermo; Waltham, MA). Peptides were separated on a 50 × 1 mm C8 column with a 20.5 min gradient starting from 2% to 33% of a solvent B (0.2% formic acid in acetonitrile). Solvent A was water with 0.1% formic acid. The injection valve and the enzyme column and its associated connecting tubing were located in a cooling box maintained at 15°C. The second switch valve, C8 column and its associated connecting stainless steel tubing were located in a refrigerated circulation box maintained at -6°C. Peptide identification was performed by searching MS/MS data for the HFB301001 sequence using Byonics (Protein Metrics; San Carlos, CA) software modified to consider non-specific enzymatic digestion and human glycosylation as common variables. The mass tolerances for precursor and product ions were 10 ppm and 0.02 Da, respectively. Raw MS data was processed using HDX WorkBench software (version 3.3) to analyze H/D exchange MS data (J. Am. Soc. Mass Spectrom. 2012, 23(9), 1512-1521). Deuterium uptake levels were calculated using the average mass difference between the deuterated peptide and its unlabeled form (t0). Deuterium uptake levels of the same peptides were compared in samples of antigen alone and in samples of antigen-antibody complexes. Relative differences greater than 5% were considered significant. Multiple overlapping peptides showing significant levels of deuterium uptake defined the epitope regions. A total of 46 peptides from hOX40-his were identified from hOX40.his alone and hOX40-his as complexed with HFB301001 samples, representing 70% sequence coverage of hOX40 (Fig. 13). Any peptide that exhibited a percentage difference in D uptake values greater than 5% was defined as being significantly protected. For hOX40-his, the peptide in the region corresponding to amino acids 56-74 CSRSQNTVCRPCGPGFYND was significantly protected by HFB301001 and was thus defined as an epitope. As shown in Fig. 13, epitope regions were mapped onto a 3D model of the OX40/OX40L complex, adapted from Compaan and Hymowitz. Structure. 2006, 14(8), 1321-30. The epitope of HFB301001 was defined as amino acids 56-74 of SEQ ID NO: 33, CSRSQNTVCRPCGPGFYND (SEQ ID NO: 34).

### Example 11: Agonistic activity of OX40 ligand

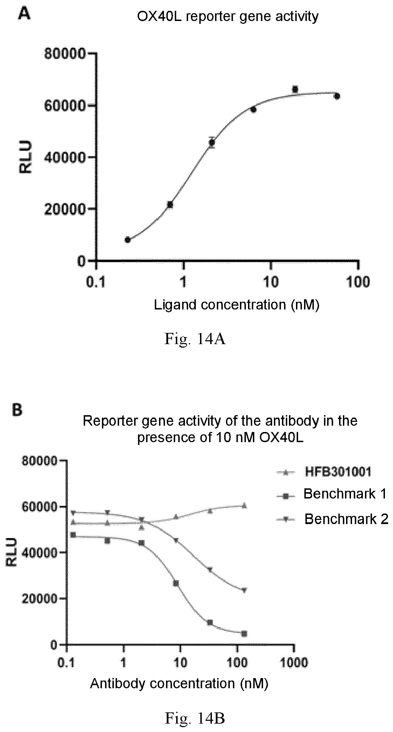

To determine the agonistic activity of the OX40 ligand (OX40L), the OX40 bioassay kit (Promega, Cat. No. CS197704; Promega; Madison, WI) was used according to the manufacturer's protocol. The assay used a genetically engineered Jurkat T cell line (OX40 effector cells) expressing human OX40 and a luciferase reporter gene driven by a response element. Briefly, OX40 effector cells were cultured in an assay buffer the day before use. On the day of the assay, OX40L was serially diluted and added to the plate. Bioluminescence signal was quantified using the Bio-Glo Luciferase Assay System after 5 hours of induction. The results showed that OX40L induced bioluminescence signals in OX40 effector cells in a dose-dependent manner (Fig. 14A).

To investigate the effect of anti-OX40 antibodies on the agonistic activity of OX40-L, OX40 effector cells were incubated with serial dilutions of anti-OX40 antibody (HFB301001, benchmark 1 or benchmark 2) in the presence of 10 nM OX40L. Benchmark 1 and Benchmark 2 were previously published anti-OX40 antibodies and were chosen because the antibody epitopes on OX40 were furthest away from the cell membrane. Other benchmarks were described as binding near the ligand-binding pocket of OX40. After 5 hours of induction, the bioluminescence signal was quantified using the Bio-Glo Luciferase Assay System. Both Benchmark 1 and Benchmark 2 blocked OX40L agonism in a dose-dependent manner, but not HFB301001 (Fig. 14B). The results showed that HFB301001 can recognize a different binding site on OX40 than Benchmark 1 and Benchmark 2. The binding site does not interfere with the agonistic function of OX40L.

### Example 12: OX40 receptor modulation

In order to study the receptor modulation effect of anti-OX40 antibody binding to OX40 receptor, CD4⁺ T cells were isolated from human peripheral blood mononuclear cells (PBMCs) using Miltenyi's Human CD4⁺ T Isolation Kit (Cat. 130-096-533; Miltenyi Biotec; Bergisch Gladbach, Germany). Primary CD4⁺ T cells were incubated with serial dilutions of either the reference antibody or HFB301001, and with plate-bound anti-CD3 antibody at 0.5 µg/mL (clone OKT3, eBioscience, catalogue 16-0037-81) and a soluble anti-CD28 antibody at 2 µg/mL (clone CD28.2, eBioscience, catalogue 16-0289-81; eBioscience, Inc.; San Diego, CA), they were simultaneously activated. After 3 days of incubation, total OX40 surface expression was detected by staining with anti-OX40 antibody (PerCP-Cy5.5, clone ACT35, Thermo Fisher, catalog 17-1347-42). Compared to isotype controls, HFB301001 enhanced OX40 expression, which was stably retained on the cell surface (Fig. 15A). For all treatments using the benchmarks, the OX40 surface expression was U-shaped with increasing antibody concentration: the OX40 level decreased, reaching the lowest point thereof around 1 nM and then increasing again.

### Example 13: Determination of Binding Affinity

Biofilm layer interference (BLI) experiments were performed on the Octet QKe instrument to determine the binding affinity of HFB301001 to the recombinant dimeric (mFc-tagged) form of human OX40 protein. Various concentrations of recombinant human OX40 protein were mixed with sensor-captured test antibodies or isotype controls at pH 7.4 and 25°C, followed by a dissociation phase. Changes in binding signal were recorded and kinetic binding parameters were determined using a 1:1 binding model with mass transport limitations. The running buffer was made into PBS, pH 7.4, containing 0.1% BSA and 0.1% Tween 20. Anti-human Fc capture sensors were rinsed with the running buffer prior to the kinetic binding experiments. The instrument was adjusted to have a temperature of 25°C. Test antibodies and isotype control antibodies were diluted to 200 nM with the running buffer. OX40-mFc protein was diluted to 250, 125, 62.5, 31.25 and 15.125 nM with the running buffer. Binding of OX40 protein to antibody was measured with the running buffer at pH 7.4 and 25°C. Experiments were performed in duplicate. Briefly, the anti-human Fc capture sensor was first baselined in the running buffer for 300 seconds. The sensor was then mixed with a 200 nM test antibody solution for 600 seconds to load the antibody. The sensor was then baselined again in the running buffer for 300 seconds, followed by association with various concentrations of OX40 antigen for 900 seconds. Dissociation was then performed by immersing the sensor in the running buffer for an additional 900 seconds. In parallel, one sensor was selected for a reference experiment in which all loading, association and dissociation steps were performed in the running buffer. Another sensor was chosen as a negative control experiment in which the HFB-TT-hG1 isotype control antibody was used for the loading step, and 500 nM OX40-mFc protein was used for the association step. Sensorgrams were analyzed by Fortebio data analysis software version 8.2 (ForteBio; Fremont, CA). Signals were first subtracted from negative control experiments and aligned through a baseline step. Kinetic parameters were obtained by overall fitting these specific sensorgrams with different antigen concentrations to a 1:1 binding model. The equilibrium dissociation constant (KD) was calculated based on the ratio of the dissociation rate constant to the association rate constant (KD = kd/ka). Kinetic binding parameters for the interaction of HFB301001 with OX40 protein from human were determined using the BLI technique. The assay used a range of concentrations of OX40 protein to interact with captured HFB10-1E1 and other antibodies at 25°C and pH 7.4. The following binding affinity table summarized the calculated kinetic binding parameters. In the experiments conducted at 25°C and pH 7.4, HFB301001 bound to recombinant human OX40 (hOX40.mFc) protein with high affinity with a KD value of 4.5 nM. The other two comparative antibodies showed slower dissociation rates, resulting in smaller KD values of 0.49 nM for Benchmark 1 and 0.58 nM for Benchmark 2. The kinetic binding parameters of HFB301001 interaction with human OX40-mFc were determined by BLI technique at 25°C and pH 7.4. HFB301001 can specifically bind to recombinant human OX40-mFc protein with single-digit nM affinity. HFB301001 showed a faster dissociation rate as compared to other tested comparative antibodies.

### Binding affinity table

| **Antibody** | **Kinetic binding parameters** | | | | | |
|---|---|---|---|---|---|---|
| | Experiment A | | | Experiment B | | |
| | kₐ (1/Ms) | k_{d} (1/s) | K_{D} (M) | kₐ (1/Ms) | k_{d} (1/s) | K_{D} (M) |
| **HFB301001** | 9.7E+04 | 4.3E-04 | 4.5E-09 | 9.7E+04 | 4.3E-04 | 4.4E-09 |
| **Benchmark** 1 | 1.5E+05 | 7.4E-05 | 4.9E-10 | 9.8E+04 | 5.9E-05 | 6.0E-10 |
| **Benchmark 2** | 1.1E+05 | 6.1E-05 | 5.8E-10 | 1.5E+05 | 7.6E-05 | 5.1E-10 |

### Example 14. In vivo tumor model

The MC-38 murine colon cancer model was used for human OX40 (hOX40) knock-in (KI) mice (catalog NM-HU-00041, Shanghai Model Organisms Center, Inc.). To evaluate the pharmacodynamic (PD) effects of HFB301001 in the MC-38 murine colorectal cancer model, 45 female hOX40 KI mice were inoculated subcutaneously on the right side thereof with MC-38 tumor cells (8×10⁵ cells per mouse) for tumor development. Eight days after tumor inoculation, 12 mice with tumors ranging from 104 to 243 mm³ (mean tumor size 181 mm³) were selected; they were divided into 3 groups with 4 mice in each group based on stratified randomization according to their tumor volume. Mice were injected with the anti-OX40 antibody HFB301001 at a dose of 10 mg/kg, the anti-OX40 antibody benchmark 1 at a dose of 10 mg/kg, and an IgG1 isotype control at a dose of 10 mg/kg. All treatments were administered by intraperitoneal (i.p) injection on day (D) D0.

OX40 expression on CD4+ T cells was measured by flow cytometry 24 hours after the third treatment with 10 mg/kg anti-OX40 antibody. Benchmark 1 induced a significant downregulation of OX40 expression on CD4⁺ T cells in blood, but not HFB301001 (Fig. 15B).

To evaluate the in vivo antitumor activity of HFB301001 in a subcutaneous MC-38 murine colorectal cancer model, 26 female hOX40 KI mice were inoculated subcutaneously on the right side thereof with MC-38 tumor cells (8×10⁵ cells per mouse) for tumor development. Seven days after tumor inoculation, 20 mice with tumor size of 101 to 175 mm³ (mean tumor size of 133 mm³) were selected; they were divided into 4 treatment groups with 5 mice in each group based on stratified randomization according to their tumor volume. Mice were injected with anti-OX40 antibody HFB301001 at doses of 1 mg/kg and 0.1 mg/kg, anti-OX40 antibody benchmark 1 at 1 mg/kg, and IgG1 isotype control at 10 mg/kg. All treatments were administered by intraperitoneal injection on D0, D3, D6, D10 and D13. Treatment time points were indicated by arrows, error bars indicated standard error of the mean, and significance was calculated by one-way ANOVA at the last time point (^{∗}: p-value < 0.05, ^{∗∗}: p-value < 0.01). Tumor size for each treatment group was measured at D0, D3, D6, D10, D12, and D15 after randomization by tumor diameter (width, length) measured using a digital caliper. As shown in Fig. 16, HFB301001 resulted in significant tumor growth inhibition as compared to control, and was better than benchmark 1.

To assess the in vivo antitumor activity of HFB301001 in a subcutaneous MC-38 murine colorectal cancer model, 80 female hOX40 KI mice were inoculated subcutaneously on the right side thereof with MC-38 tumor cells (8×10⁵ cells per mouse) for tumor development. Seven days after tumor inoculation, 65 mice with tumors ranging from 42 to 147 mm³ (mean tumor size 82 mm³) were selected; they were divided into 7 treatment groups with 10 mice in each group (except for the PBS group, which had only 5 mice) based on stratified randomization according to their tumor volume. Treatment began on the day of randomization (defined as Day 0 (D0)). Mice were injected with anti-OX40 antibody HFB301001 at doses of 10 mg/kg, 1 mg/kg and 0.1 mg/kg, anti-OX40 antibody benchmark 1 at doses of 10 mg/kg and 1 mg/kg, and IgG1 isotype control, at the dose of 10 mg/kg. All treatments were administered by intraperitoneal injection on D0, D3, D6, D10 and D13. Tumor size and animal body weight were measured at least three times a week after treatment initiation through day 61. Significance was calculated by log-rank test (^{∗}: p-value < 0.05, ^{∗∗}: p-value < 0.01). Survival was followed for 60 days after randomization. The percent survival of mice treated with 10 mg/kg HFB301001 was significantly higher than that of mice treated with 10 mg/kg of benchmark 1 (Fig. 17).

To further investigate the effect of treatment with the anti-OX40 antibody HFB301001, the changes induced in tumor T cells following treatment with the anti-OX40 antibody were measured by flow cytometry 24 hours after the third treatment. Eight days after the tumor inoculation, 12 mice with tumors ranging from 104 to 243 mm³ (mean tumor size 181 mm³) were selected; they were divided into 3 groups with 4 mice in each group based on stratified randomization according to their tumor volume. Significant increases in KI67⁺ cells with HFB301001 were observed in both CD4⁺ and CD8⁺ T cells (Fig. 18A to 18B). In addition, a significant reduction of PD-1⁺ cells with HFB301001 was observed in both CD4⁺ and CD8⁺ T cells (Fig. 18C to 18D). Moreover, both benchmark and HFB301001 resulted in a significant reduction in tumor Tregs, with the HFB301001 group having a more pronounced decrease (Fig. 18E).

### References

Wang, R., Gao, C., Raymond, M., Dito, G., Kabbabe, D., Shao, X., Hilt, E., Sun, Y., Pak, I., Gutierrez, M., Melero, I., Spreafico, A., Carvajal, R., Ong, M., Olszanski, A., Milburn, C., Thudium, K., Yang, Z., Feng, Y., Fracasso, P., Korman, A., Aanur, P., Huang, S., Quigley, M. (2019). An Integrative Approach to Inform Optimal Administration of OX40 Agonist Antibodies in Patients with Advanced Solid Tumors, Clinical Cancer Research, https://dx.doi.org/10.1158/1078-0432.CCR-19-0526.

While the principles of the present invention have been described above in connection with preferred embodiments, it should be understood that this description has been given by way of example only, and not as a limitation on the scope of the present invention.

### Sequence listing

| No. | Title | Sequence |
|---|---|---|
| SEQ ID NO: 1 | HFB10-1E1hG1 heavy chain variable region CDR1 domain amino acid sequence | GFTFSSYA |
| SEQ ID NO: 2 | HFB10-1E1hG1 heavy chain variable region CDR2 domain amino acid sequence | ISGSGGST |
| SEQ ID NO: 3 | HFB10-1E1hG1 heavy chain variable region CDR3 domain amino acid sequence | ARDLSSSWYLDAFDI |
| SEQ ID NO: 4 | HFB10-1E1hG1 heavy chain variable region amino acid sequence | EVQLLESGGGLVQPGGSLRLSCAASGFT FSSYAMSWVRQAPGKGLEWVSAISGSG GSTYYADSVKGRFTISRDNSKNTLYLQM NSLRAEDTAVYYCARDLSSSWYLDAFDI WGQGTMVTVSS |
| SEQ ID NO: 5 | HFB10-1E1hG1 heavy chain constant region amino acid sequence | EVQLLESGGGLVQPGGSLRLSCAASGFT FSSYAMSWVRQAPGKGLEWVSAISGSG GSTYYADSVKGRFTISRDNSKNTLYLQM NSLRAEDTAVYYCARDLSSSWYLDAFDI WGQGTMVTVSS ASTKGPSVFPLAPSSKSTSGGTAALGCLV KDYFPEPVTVSWNSGALTSGVHTFPAVL QSSGLYSLSSVVTVPSSSLGTQTYICNVN HKPSNTKVDKKVEPKSCDKTHTCPPCPA PELLGGPSVFLFPPKPKDTLMISRTPEVT CVVVDVSHEDPEVKFNWYVDGVEVHN AKTKPREEQYNSTYRVVSVLTVLHQDW LNGKEYKCKVSNKALPAPIEKTISKAKG QPREPQVYTLPPSREEMTKNQVSLTCLV KGFYPSDIAVEWESNGQPENNYKTTPPV LDSDGSFFLYSKLTVDKSRWQQGNVFSC SVMHEALHNHYTQKSLSLSPGK |
| SEQ ID NO: 6 | HFB10-1E1hG1 heavy chain signal peptide amino acid sequence | MDLLHKNMKHLWFFLLLVAAPRWVLS |
| SEQ ID NO: 7 | HFB10-1E1hG1 heavy chain full-length amino acid sequence (without signal peptide) | EVQLLESGGGLVQPGGSLRLSCAASGFT FSSYAMSWVRQAPGKGLEWVSAISGSG GSTYYADSVKGRFTISRDNSKNTLYLQM NSLRAEDTAVYYCARDLSSSWYLDAFDI WGQGTMVTVSS ASTKGPSVFPLAPSSKSTSGGTAALGCLV KDYFPEPVTVSWNSGALTSGVHTFPAVL QSSGLYSLSSVVTVPSSSLGTQTYICNVN HKPSNTKVDKKVEPKSCDKTHTCPPCPA PELLGGPSVFLFPPKPKDTLMISRTPEVT CVVVDVSHEDPEVKFNWYVDGVEVHN AKTKPREEQYNSTYRVVSVLTVLHQDW LNGKEYKCKVSNKALPAPIEKTISKAKG QPREPQVYTLPPSREEMTKNQVSLTCLV KGFYPSDIAVEWESNGQPENNYKTTPPV LDSDGSFFLYSKLTVDKSRWQQGNVFSC SVMHEALHNHYTQKSLSLSPGK |
| SEQ ID NO: 8 | HFB10-1E1hG1 heavy chain full-length amino acid sequence (with signal peptide) | MDLLHKNMKHLWFFLLLVAAPRWVLS EVQLLESGGGLVQPGGSLRLSCAASGFT FSSYAMSWVRQAPGKGLEWVSAISGSG GSTYYADSVKGRFTISRDNSKNTLYLQM NSLRAEDTAVYYCARDLSSSWYLDAFDI WGQGTMVTVSS ASTKGPSVFPLAPSSKSTSGGTAALGCLV KDYFPEPVTVSWNSGALTSGVHTFPAVL QSSGLYSLSSVVTVPSSSLGTQTYICNVN HKPSNTKVDKKVEPKSCDKTHTCPPCPA PELLGGPSVFLFPPKPKDTLMISRTPEVT CVVVDVSHEDPEVKFNWYVDGVEVHN AKTKPREEQYNSTYRVVSVLTVLHQDW LNGKEYKCKVSNKALPAPIEKTISKAKG QPREPQVYTLPPSREEMTKNQVSLTCLV KGFYPSDIAVEWESNGQPENNYKTTPPV LDSDGSFFLYSKLTVDKSRWQQGNVFSC SVMHEALHNHYTQKSLSLSPGK |
| SEQ ID NO: 9 | HFB10-1E1hG1 light chain variable region CDR1 domain amino acid sequence | QGISSW |
| SEQ ID NO: 10 | HFB10-1E1hG1 light chain variable region CDR2 domain amino acid sequence | AAS |
| SEQ ID NO: 11 | HFB10-1E1hG1 light chain variable region CDR3 domain amino acid sequence | QQANSFPLT |
| SEQ ID NO: 12 | HFB10-1E1hG1 light chain variable region amino acid sequence | DIQMTQSPSSVSASVGDRVTITCRASQGI SSWLAWYQQKPGKAPKLLIYAASSLQS GVPSRFSGSGSGTDFTLTISSLQPEDFATY YCQQANSFPLTFGGGTKLEIK |
| SEQ ID NO: 13 | HFB10-1E1hG1 light chain constant region amino acid sequence | |
| SEQ ID NO: 14 | HFB10-1E1hG1 light chain signal peptide amino acid sequence | MDLLHKNMKHLWFFLLLVAAPRWVLS |
| SEQ ID NO: 15 | HFB10-1E1hG1 light chain full-length amino acid sequence (without signal peptide) | DIQMTQSPSSVSASVGDRVTITCRASQGI SSWLAWYQQKPGKAPKLLIYAASSLQS GVPSRFSGSGSGTDFTLTISSLQPEDFATY YCQQANSFPLTFGGGTKLEIK RTVAAPSVFIFPPSDEQLKSGTASVVCLL NNFYPREAKVQWKVDNALQSGNSQESV TEQDSKDSTYSLSSTLTLSKADYEKHKV YACEVTHQGLSSPVTKSFNRGEC |
| SEQ ID NO: | HFB10-1E1hG1 light chain full- | MDLLHKNMKHLWFFLLLVAAPRWVLS |
| 16 | length amino acid sequence (with signal peptide) | DIQMTQSPSSVSASVGDRVTITCRASQGI SSWLAWYQQKPGKAPKLLIYAASSLQS GVPSRFSGSGSGTDFTLTISSLQPEDFATY YCQQANSFPLTFGGGTKLEIK RTVAAPSVFIFPPSDEQLKSGTASVVCLL NNFYPREAKVQWKVDNALQSGNSQESV TEQDSKDSTYSLSSTLTLSKADYEKHKV YACEVTHQGLSSPVTKSFNRGEC |
| SEQ ID NO: 17 | HFB10-1E1hG1 heavy chain variable region CDR1 domain nucleotide encoding sequence | GGCTTCACCTTCTCCTCTTACGCC |
| SEQ ID NO: 18 | HFB10-1E1hG1 heavy chain variable region CDR2 domain nucleotide encoding sequence | ATCTCCGGCTCTGGCGGATCTACC |
| SEQ ID NO: 19 | HFB10-1E1hG1 heavy chain variable region CDR3 domain nucleotide encoding sequence | GCCAGAGATCTGTCCTCCTCCTGGTAT CTGGACGCCTTTGATATC |
| SEQ ID NO: 20 | HFB10-1E1hG1 heavy chain variable region nucleotide encoding sequence | GAAGTTCAGCTGCTGGAATCTGGCGGA GGACTGGTTCAGCCTGGCGGCTCTTTG AGACTGTCTTGTGCCGCCTCTGGCTTC ACCTTCTCCTCTTACGCCATGTCCTGGG TCCGACAGGCTCCTGGAAAAGGACTG GAATGGGTGTCCGCTATCTCCGGCTCT GGCGGATCTACCTACTACGCCGACTCC GTGAAGGGCAGATTCACCATCAGCCG GGACAACTCCAAGAACACCCTGTACCT GCAGATGAACTCCCTGAGAGCCGAGG ACACCGCCGTGTACTACTGTGCCAGAG ATCTGTCCTCCTCCTGGTATCTGGACG CCTTTGATATCTGGGGCCAGGGCACCA TGGTCACCGTGTCCTCT |
| SEQ ID NO: 21 | HFB10-1E1hG1 heavy chain constant region nucleotide encoding sequence | GCTTCTACCAAGGGACCCTCTGTGTTC CCTCTGGCTCCTTCCTCTAAATCCACCT CTGGTGGCACCGCTGCTCTGGGCTGTC TGGTCAAGGATTACTTCCCTGAGCCTG TGACCGTGTCTTGGAACTCTGGTGCTC TGACCTCCGGCGTGCACACATTCCCTG CTGTGCTGCAGTCCTCTGGCCTGTACT CTCTGTCCTCTGTCGTGACCGTGCCTTC TAGCTCTCTGGGCACCCAGACCTACAT CTGCAACGTGAACCACAAGCCTTCCAA CACCAAGGTGGACAAGAAGGTGGAAC CCAAGTCCTGCGACAAGACCCACACCT GTCCTCCATGTCCAGCTCCAGAACTGC TCGGCGGACCTTCCGTGTTCCTGTTTCC TCCAAAGCCTAAGGACACCCTGATGAT CTCTCGGACCCCTGAAGTGACCTGCGT GGTGGTGGATGTGTCTCACGAGGACCC |
| | | AGAAGTGAAGTTCAATTGGTACGTGGA CGGCGTGGAAGTGCACAACGCCAAGA CCAAGCCTAGAGAGGAACAGTACAAC TCCACCTACAGAGTGGTGTCCGTGCTG ACCGTGCTGCACCAGGATTGGCTGAAC GGCAAAGAGTACAAGTGCAAGGTGTC CAACAAGGCCCTGCCTGCTCCTATCGA AAAGACCATCTCTAAGGCCAAGGGCC AGCCTCGCGAACCTCAGGTTTACACCC TGCCTCCAAGCCGGGAAGAGATGACC AAGAACCAGGTGTCCCTGACCTGCCTG GTTAAGGGCTTCTACCCCTCCGATATC GCCGTGGAATGGGAGTCTAACGGCCA GCCTGAGAACAACTACAAGACAACCC CTCCTGTGCTGGACTCCGACGGCTCAT TCTTCCTGTACTCCAAGCTGACAGTGG ACAAGTCCAGATGGCAGCAGGGCAAC GTGTTCTCCTGTTCTGTGATGCACGAG GCCCTGCACAACCACTACACACAGAA GTCCCTGTCTCTGTCCCCTGGCAAG |
| SEQ ID NO: 22 | HFB10-1E1hG1 heavy chain signal peptide nucleotide encoding sequence | ATGGACCTGCTGCACAAGAACATGAA GCACCTGTGGTTCTTTCTGCTGCTGGTG GCCGCTCCTAGATGGGTGCTGTCT |
| SEQ ID NO: 23 | HFB10-1E1hG1 heavy chain full-length nucleotide encoding sequence (without signal peptide) | GAAGTTCAGCTGCTGGAATCTGGCGGA GGACTGGTTCAGCCTGGCGGCTCTTTG AGACTGTCTTGTGCCGCCTCTGGCTTC ACCTTCTCCTCTTACGCCATGTCCTGGG TCCGACAGGCTCCTGGAAAAGGACTG GAATGGGTGTCCGCTATCTCCGGCTCT GGCGGATCTACCTACTACGCCGACTCC GTGAAGGGCAGATTCACCATCAGCCG GGACAACTCCAAGAACACCCTGTACCT GCAGATGAACTCCCTGAGAGCCGAGG ACACCGCCGTGTACTACTGTGCCAGAG ATCTGTCCTCCTCCTGGTATCTGGACG CCTTTGATATCTGGGGCCAGGGCACCA TGGTCACCGTGTCCTCTGCTTCTACCA AGGGACCCTCTGTGTTCCCTCTGGCTC CTTCCTCTAAATCCACCTCTGGTGGCA CCGCTGCTCTGGGCTGTCTGGTCAAGG ATTACTTCCCTGAGCCTGTGACCGTGT CTTGGAACTCTGGTGCTCTGACCTCCG GCGTGCACACATTCCCTGCTGTGCTGC AGTCCTCTGGCCTGTACTCTCTGTCCTC TGTCGTGACCGTGCCTTCTAGCTCTCTG GGCACCCAGACCTACATCTGCAACGTG AACCACAAGCCTTCCAACACCAAGGTG GACAAGAAGGTGGAACCCAAGTCCTG CGACAAGACCCACACCTGTCCTCCATG TCCAGCTCCAGAACTGCTCGGCGGACC |
| | | TTCCGTGTTCCTGTTTCCTCCAAAGCCT AAGGACACCCTGATGATCTCTCGGACC CCTGAAGTGACCTGCGTGGTGGTGGAT GTGTCTCACGAGGACCCAGAAGTGAA GTTCAATTGGTACGTGGACGGCGTGGA AGTGCACAACGCCAAGACCAAGCCTA GAGAGGAACAGTACAACTCCACCTAC AGAGTGGTGTCCGTGCTGACCGTGCTG CACCAGGATTGGCTGAACGGCAAAGA GTACAAGTGCAAGGTGTCCAACAAGG CCCTGCCTGCTCCTATCGAAAAGACCA TCTCTAAGGCCAAGGGCCAGCCTCGCG AACCTCAGGTTTACACCCTGCCTCCAA GCCGGGAAGAGATGACCAAGAACCAG GTGTCCCTGACCTGCCTGGTTAAGGGC TTCTACCCCTCCGATATCGCCGTGGAA TGGGAGTCTAACGGCCAGCCTGAGAA CAACTACAAGACAACCCCTCCTGTGCT GGACTCCGACGGCTCATTCTTCCTGTA CTCCAAGCTGACAGTGGACAAGTCCAG ATGGCAGCAGGGCAACGTGTTCTCCTG TTCTGTGATGCACGAGGCCCTGCACAA CCACTACACACAGAAGTCCCTGTCTCT GTCCCCTGGCAAG |
| SEQ ID NO: 24 | HFB10-1E1hG1 heavy chain full-length nucleotide encoding sequence (with signal peptide) | ATGGACCTGCTGCACAAGAACATGAA GCACCTGTGGTTCTTTCTGCTGCTGGTG GCCGCTCCTAGATGGGTGCTGTCT GAAGTTCAGCTGCTGGAATCTGGCGGA GGACTGGTTCAGCCTGGCGGCTCTTTG AGACTGTCTTGTGCCGCCTCTGGCTTC ACCTTCTCCTCTTACGCCATGTCCTGGG TCCGACAGGCTCCTGGAAAAGGACTG GAATGGGTGTCCGCTATCTCCGGCTCT GGCGGATCTACCTACTACGCCGACTCC GTGAAGGGCAGATTCACCATCAGCCG GGACAACTCCAAGAACACCCTGTACCT GCAGATGAACTCCCTGAGAGCCGAGG ACACCGCCGTGTACTACTGTGCCAGAG ATCTGTCCTCCTCCTGGTATCTGGACG CCTTTGATATCTGGGGCCAGGGCACCA TGGTCACCGTGTCCTCTGCTTCTACCA AGGGACCCTCTGTGTTCCCTCTGGCTC CTTCCTCTAAATCCACCTCTGGTGGCA CCGCTGCTCTGGGCTGTCTGGTCAAGG ATTACTTCCCTGAGCCTGTGACCGTGT CTTGGAACTCTGGTGCTCTGACCTCCG GCGTGCACACATTCCCTGCTGTGCTGC AGTCCTCTGGCCTGTACTCTCTGTCCTC TGTCGTGACCGTGCCTTCTAGCTCTCTG |
| | | GGCACCCAGACCTACATCTGCAACGTG AACCACAAGCCTTCCAACACCAAGGTG GACAAGAAGGTGGAACCCAAGTCCTG CGACAAGACCCACACCTGTCCTCCATG TCCAGCTCCAGAACTGCTCGGCGGACC TTCCGTGTTCCTGTTTCCTCCAAAGCCT AAGGACACCCTGATGATCTCTCGGACC CCTGAAGTGACCTGCGTGGTGGTGGAT GTGTCTCACGAGGACCCAGAAGTGAA GTTCAATTGGTACGTGGACGGCGTGGA AGTGCACAACGCCAAGACCAAGCCTA GAGAGGAACAGTACAACTCCACCTAC AGAGTGGTGTCCGTGCTGACCGTGCTG CACCAGGATTGGCTGAACGGCAAAGA GTACAAGTGCAAGGTGTCCAACAAGG CCCTGCCTGCTCCTATCGAAAAGACCA TCTCTAAGGCCAAGGGCCAGCCTCGCG AACCTCAGGTTTACACCCTGCCTCCAA GCCGGGAAGAGATGACCAAGAACCAG GTGTCCCTGACCTGCCTGGTTAAGGGC TTCTACCCCTCCGATATCGCCGTGGAA TGGGAGTCTAACGGCCAGCCTGAGAA CAACTACAAGACAACCCCTCCTGTGCT GGACTCCGACGGCTCATTCTTCCTGTA CTCCAAGCTGACAGTGGACAAGTCCAG ATGGCAGCAGGGCAACGTGTTCTCCTG TTCTGTGATGCACGAGGCCCTGCACAA CCACTACACACAGAAGTCCCTGTCTCT GTCCCCTGGCAAG |
| SEQ ID NO: 25 | HFB10-1E1hG1 light chain variable region CDR1 domain nucleotide encoding sequence | CAGGGCATCTCTAGCTGG |
| SEQ ID NO: 26 | HFB10-1E1hG1 light chain variable region CDR2 domain nucleotide encoding sequence | GCCAGTTCT |
| SEQ ID NO: 27 | HFB10-1E1hG1 light chain variable region CDR3 domain nucleotide encoding sequence | CAGCAGGCCAACAGCTTCCCTCTGACC |
| SEQ ID NO: 28 | HFB10-1E1hG1 light chain variable region nucleotide encoding sequence | GACATCCAGATGACCCAGTCTCCATCC TCCGTGTCTGCCTCTGTGGGCGACAGA GTGACCATCACCTGTAGAGCCTCTCAG GGCATCTCTAGCTGGCTGGCCTGGTAT CAGCAGAAGCCTGGAAAGGCCCCTAA GCTGCTGATCTACGCTGCCAGTTCTCT GCAGTCTGGCGTGCCCTCTAGATTCTC TGGCTCTGGATCTGGCACCGACTTCAC CCTGACAATCTCTAGCCTGCAGCCTGA GGACTTCGCCACCTACTACTGTCAGCA GGCCAACAGCTTCCCTCTGACCTTTGG CGGCGGAACAAAGCTGGAAATCAAG |
| SEQ ID NO: 29 | HFB10-1E1hG1 light chain constant region nucleotide encoding sequence | AGAACCGTGGCTGCCCCTTCCGTGTTC ATCTTCCCACCATCTGACGAGCAGCTG AAGTCCGGCACAGCTTCTGTCGTGTGC CTGCTGAACAACTTCTACCCTCGGGAA GCCAAGGTGCAGTGGAAGGTGGACAA TGCCCTGCAGTCCGGCAACTCCCAAGA GTCTGTGACCGAGCAGGACTCCAAGG ACTCTACCTACAGCCTGTCCTCCACAC TGACCCTGTCTAAGGCCGACTACGAGA AGCACAAGGTGTACGCCTGTGAAGTG ACCCACCAGGGACTGTCTAGCCCCGTG ACCAAGTCTTTCAACAGAGGCGAGTGC |
| SEQ ID NO: 30 | HFB10-1E1hG1 light chain signal peptide nucleotide encoding sequence | ATGGACCTGCTGCACAAGAACATGAA GCACCTGTGGTTCTTTCTGCTGCTGGTG GCCGCTCCTAGATGGGTGCTGTCT |
| SEQ ID NO: 31 | HFB10-1E1hG1 light chain full-length nucleotide encoding sequence (without signal peptide) | GACATCCAGATGACCCAGTCTCCATCC TCCGTGTCTGCCTCTGTGGGCGACAGA GTGACCATCACCTGTAGAGCCTCTCAG GGCATCTCTAGCTGGCTGGCCTGGTAT CAGCAGAAGCCTGGAAAGGCCCCTAA GCTGCTGATCTACGCTGCCAGTTCTCT GCAGTCTGGCGTGCCCTCTAGATTCTC TGGCTCTGGATCTGGCACCGACTTCAC CCTGACAATCTCTAGCCTGCAGCCTGA GGACTTCGCCACCTACTACTGTCAGCA GGCCAACAGCTTCCCTCTGACCTTTGG CGGCGGAACAAAGCTGGAAATCAAGA GAACCGTGGCTGCCCCTTCCGTGTTCA TCTTCCCACCATCTGACGAGCAGCTGA AGTCCGGCACAGCTTCTGTCGTGTGCC TGCTGAACAACTTCTACCCTCGGGAAG CCAAGGTGCAGTGGAAGGTGGACAAT GCCCTGCAGTCCGGCAACTCCCAAGAG TCTGTGACCGAGCAGGACTCCAAGGAC TCTACCTACAGCCTGTCCTCCACACTG ACCCTGTCTAAGGCCGACTACGAGAAG CACAAGGTGTACGCCTGTGAAGTGACC CACCAGGGACTGTCTAGCCCCGTGACC AAGTCTTTCAACAGAGGCGAGTGC |
| SEQ ID NO: 32 | HFB10-1E1hG1 light chain full-length nucleotide encoding sequence (with signal peptide) | ATGGACCTGCTGCACAAGAACATGAA GCACCTGTGGTTCTTTCTGCTGCTGGTG GCCGCTCCTAGATGGGTGCTGTCT GACATCCAGATGACCCAGTCTCCATCC TCCGTGTCTGCCTCTGTGGGCGACAGA GTGACCATCACCTGTAGAGCCTCTCAG GGCATCTCTAGCTGGCTGGCCTGGTAT CAGCAGAAGCCTGGAAAGGCCCCTAA GCTGCTGATCTACGCTGCCAGTTCTCT GCAGTCTGGCGTGCCCTCTAGATTCTC |
| | | TGGCTCTGGATCTGGCACCGACTTCAC CCTGACAATCTCTAGCCTGCAGCCTGA GGACTTCGCCACCTACTACTGTCAGCA GGCCAACAGCTTCCCTCTGACCTTTGG CGGCGGAACAAAGCTGGAAATCAAGA GAACCGTGGCTGCCCCTTCCGTGTTCA TCTTCCCACCATCTGACGAGCAGCTGA AGTCCGGCACAGCTTCTGTCGTGTGCC TGCTGAACAACTTCTACCCTCGGGAAG CCAAGGTGCAGTGGAAGGTGGACAAT GCCCTGCAGTCCGGCAACTCCCAAGAG TCTGTGACCGAGCAGGACTCCAAGGAC TCTACCTACAGCCTGTCCTCCACACTG ACCCTGTCTAAGGCCGACTACGAGAAG CACAAGGTGTACGCCTGTGAAGTGACC CACCAGGGACTGTCTAGCCCCGTGACC AAGTCTTTCAACAGAGGCGAGTGC |
| SEQ ID NO: 33 | | MCVGARRLGRGPCAALLLLGLGLSTVT GLHCVGDTYPSNDRCCHECRPGNGMVS RCSRSQNTVCRPCGPGFYNDVVSSKPCK PCTWCNLRSGSERKQLCTATQDTVCRC RAGTQPLDSYKPGVDCAPCPPGHFSPGD NQACKPWTNCTLAGKHTLQPASNSSDAI CEDRDPPATQPQETQGPPARPITVQPTEA WPRTSQGPSTRPVEVPGGRAVAAILGLG LVLGLLGPLAILLALYLLRRDQRLPPDA HKPPGGGSFRTPIQEEQADAHSTLAKI |
| SEQ ID NO: 34 | | CSRSQNTVCRPCGPGFYND |

## Claims

1. An isolated anti-OX40 antibody or antigen-binding fragment thereof, comprising:
a heavy chain variable region having a heavy chain CDR1 structural domain as shown in SEQ ID NO: 1, a heavy chain CDR2 structural domain as shown in SEQ ID NO: 2, and a heavy chain CDR3 structural domain as shown in SEQ ID NO: 3; and
a light chain variable region having a light chain CDR1 domain as shown in SEQ ID NO: 9, a light chain CDR2 domain as shown in SEQ ID NO: 10, and a light chain CDR3 domain as shown in SEQ ID NO: 11.

2. The anti-OX40 antibody or antigen-binding fragment thereof according to claim 1, comprising:
a heavy chain variable region as shown in SEQ ID NO: 4, or a heavy chain variable region having a homology of at least 80%, 81%, 82%, 83%, 84%, 85%, 86%, 87%, 88%, 89%, 90%, 91%, 92%, 93%, 94%, 95%, 96%, 97%, 98%, 99% or 100% with SEQ ID NO: 4; and
a light chain variable region as shown in SEQ ID NO: 12, or a light chain variable region having a homology of at least 80%, 81%, 82%, 83%, 84%, 85%, 86%, 87%, 88%, 89%, 90%, 91%, 92%, 93%, 94%, 95%, 96%, 97%, 98%, 99% or 100% with SEQ ID NO: 12.

3. The anti-OX40 antibody or antigen-binding fragment thereof according to claim 1 or 2, further comprising a heavy chain constant region and a light chain constant region;
preferably, the heavy chain constant region is a heavy chain constant region as shown in SEQ ID NO: 5, or a heavy chain constant region having a homology of at least 80%, 81%, 82%, 83%, 84%, 85%, 86%, 87%, 88%, 89%, 90%, 91%, 92%, 93%, 94%, 95%, 96%, 97%, 98%, 99% or 100% with SEQ ID NO: 5; and/or
preferably, the light chain constant region is a light chain constant region as shown in SEQ ID NO: 13, or a light chain constant region having a homology of at least 80%, 81%, 82%, 83%, 84%, 85%, 86%, 87%, 88%, 89%, 90%, 91%, 92%, 93%, 94%, 95%, 96%, 97%, 98%, 99% or 100% with SEQ ID NO: 13.

4. The anti-OX40 antibody or antigen-binding fragment thereof according to any one of claims 1 to 3, further comprising a heavy chain signal peptide linked to the heavy chain variable region and/or a light chain signal peptide linked to the light chain variable region;
preferably, the heavy chain signal peptide is a heavy chain signal peptide as shown in SEQ ID NO: 6, or a heavy chain signal peptide having a homology of at least 80%, 81%, 82%, 83%, 84%, 85%, 86%, 87%, 88%, 89%, 90%, 91%, 92%, 93%, 94%, 95%, 96%, 97%, 98%, 99% or 100% with SEQ ID NO: 6; and/or
preferably, the light chain signal peptide is a light chain signal peptide as shown in SEQ ID NO: 14, or a light chain signal peptide having a homology of at least 80%, 81%, 82%, 83%, 84%, 85%, 86%, 87%, 88%, 89%, 90%, 91%, 92%, 93%, 94%, 95%, 96%, 97%, 98%, 99% or 100% with SEQ ID NO: 14.

5. The anti-OX40 antibody or antigen-binding fragment thereof according to any one of claims 1 to 4, which is an IgG antibody or an antigen-binding fragment thereof, preferably an IgG1 antibody or an antigen-binding fragment thereof.

6. The anti-OX40 antibody or antigen-binding fragment thereof according to any one of claims 1 to 5, which is a monoclonal antibody or an antigen-binding fragment thereof.

7. The anti-OX40 antibody or antigen-binding fragment thereof according to any one of claims 1 to 6, wherein the antigen-binding fragment is Fab, Fab', F(ab')2, Fv, scFv or sdAb.

8. An antibody-drug conjugate, comprising the anti-OX40 antibody or antigen-binding fragment thereof according to any one of claims 1 to 7, and an additional therapeutic agent; preferably, the anti-OX40 antibody or antigen-binding fragment thereof is linked to the additional therapeutic agent via a linker.

9. A nucleic acid, encoding the anti-OX40 antibody or antigen-binding fragment thereof according to any one of claims 1 to 7.

10. The nucleic acid according to claim 9, comprising a heavy chain variable region nucleotide encoding sequence as shown in SEQ ID NO: 20, and/or a light chain variable region nucleotide encoding sequence as shown in SEQ ID NO: 28;
preferably, the nucleic acid further comprising a heavy chain constant region nucleotide encoding sequence as shown in SEQ ID NO: 21, and/or a light chain constant region nucleotide encoding sequence as shown in SEQ ID NO: 29.

11. An expression vector, comprising the nucleic acid according to claim 9 or 10.

12. A host cell, comprising the nucleic acid according to claim 9 or 10 or the expression vector according to claim 11.

13. A method for preparing the anti-OX40 antibody or antigen-binding fragment thereof according to any one of claims 1 to 7, comprising culturing the host cell according to claim 12 under a condition suitable for expressing the antibody or antigen-binding fragment thereof, and recovering an expressed antibody or antigen-binding fragment thereof from a culture medium.

14. A pharmaceutical composition, comprising the anti-OX40 antibody or antigen-binding fragment thereof according to any one of claims 1 to 7, or the antibody-drug conjugate according to claim 8, or the nucleic acid according to claim 9 or 10, or the expression vector according to claim 11; and a pharmaceutically acceptable carrier.

15. The anti-OX40 antibody or antigen-binding fragment thereof according to any one of claims 1 to 7, or the antibody-drug conjugate according to claim 8, or the pharmaceutical composition according to claim 14, which is used in the treatment of cancer.

16. The anti-OX40 antibody or antigen-binding fragment thereof, or the antibody-drug conjugate, or the pharmaceutical composition according to claim 15, wherein the cancer is selected from squamous cell carcinoma (for example, epithelial squamous cell carcinoma), lung cancer (comprising small cell lung cancer, non-small cell lung cancer, adenocarcinoma of the lung, and squamous cell carcinoma of the lung), peritoneal carcinoma, hepatocellular carcinoma, gastric cancer (comprising gastrointestinal cancer and gastrointestinal stromal cancer), pancreatic cancer, glioblastoma, cervical cancer, ovarian cancer, liver cancer, bladder cancer, urinary tract cancer, liver tumor, breast cancer, colon cancer, rectal cancer, colorectal cancer, endometrial or uterine cancer, salivary gland cancer, kidney cancer, prostate cancer, vulvar cancer, thyroid cancer, liver cancer, anal cancer, penile cancer, melanoma, superficial diffuse melanoma, malignant lentigo melanoma, acral melanoma, nodular melanoma, multiple myeloma and B-cell lymphoma, chronic lymphocytic leukemia (CLL), acute lymphoblastic leukemia (ALL), hairy cell leukemia, chronic myeloblastoid leukemia, and post-transplantation lymphoproliferative disorder (PTLD), abnormal vascular proliferation associated with keloids, edema (for example, associated with brain tumors), and Meigs syndrome, brain tumor and brain cancer, head and neck cancer, and associated metastases.

17. A method for the treatment of cancer, comprising: administering to a subject in need thereof a therapeutically effective amount of the anti-OX40 antibody or antigen-binding fragment thereof according to any one of claims 1 to 7, or the antibody-drug conjugate according to claim 8, or the pharmaceutical composition according to claim 14, so as to treat the cancer.

18. The method according to claim 17, wherein the cancer is selected from squamous cell carcinoma (for example, epithelial squamous cell carcinoma), lung cancer (comprising small cell lung cancer, non-small cell lung cancer, adenocarcinoma of the lung, and squamous cell carcinoma of the lung), peritoneal carcinoma, hepatocellular carcinoma, gastric cancer (comprising gastrointestinal cancer and gastrointestinal stromal cancer), pancreatic cancer, glioblastoma, cervical cancer, ovarian cancer, liver cancer, bladder cancer, urinary tract cancer, liver tumor, breast cancer, colon cancer, rectal cancer, colorectal cancer, endometrial or uterine cancer, salivary gland cancer, kidney cancer, prostate cancer, vulvar cancer, thyroid cancer, liver cancer, anal cancer, penile cancer, melanoma, superficial diffuse melanoma, malignant lentigo melanoma, acral melanoma, nodular melanoma, multiple myeloma and B-cell lymphoma, chronic lymphocytic leukemia (CLL), acute lymphoblastic leukemia (ALL), hairy cell leukemia, chronic myeloblastoid leukemia, and post-transplantation lymphoproliferative disorder (PTLD), abnormal vascular proliferation associated with keloids, edema (for example, associated with brain tumors), and Meigs syndrome, brain tumor and brain cancer, head and neck cancer, and associated metastases.

19. A use of the anti-OX40 antibody or antigen-binding fragment thereof according to any one of claims 1 to 7, or the antibody-drug conjugate according to claim 8, or the pharmaceutical composition according to claim 14 in the preparation of a medicament for the treatment of cancer.

20. The use according to claim 19, wherein the cancer is selected from squamous cell carcinoma (for example, epithelial squamous cell carcinoma), lung cancer (comprising small cell lung cancer, non-small cell lung cancer, adenocarcinoma of the lung, and squamous cell carcinoma of the lung), peritoneal carcinoma, hepatocellular carcinoma, gastric cancer (comprising gastrointestinal cancer and gastrointestinal stromal cancer), pancreatic cancer, glioblastoma, cervical cancer, ovarian cancer, liver cancer, bladder cancer, urinary tract cancer, liver tumor, breast cancer, colon cancer, rectal cancer, colorectal cancer, endometrial or uterine cancer, salivary gland cancer, kidney cancer, prostate cancer, vulvar cancer, thyroid cancer, liver cancer, anal cancer, penile cancer, melanoma, superficial diffuse melanoma, malignant lentigo melanoma, acral melanoma, nodular melanoma, multiple myeloma and B-cell lymphoma, chronic lymphocytic leukemia (CLL), acute lymphoblastic leukemia (ALL), hairy cell leukemia, chronic myeloblastoid leukemia, and post-transplantation lymphoproliferative disorder (PTLD), abnormal vascular proliferation associated with keloids, edema (for example, associated with brain tumors), and Meigs syndrome, brain tumor and brain cancer, head and neck cancer, and associated metastases.

21. The anti-OX40 antibody or antigen-binding fragment thereof according to any one of claims 1 to 7, or the antibody-drug conjugate according to claim 8, or the pharmaceutical composition according to claim 14, which is used in one or more of the following: inhibit Treg function (for example, inhibit the suppressive function of Treg), kill cells expressing OX40 (for example, cells expressing a high level of OX40), enhance an effector T cell function and/or enhance a memory T cell function, reduce tumor immunity, enhance T cell function and/or deplete OX40 expressing cells.

22. A use of the anti-OX40 antibody or antigen-binding fragment thereof according to any one of claims 1 to 7, or the antibody-drug conjugate according to claim 8, or the pharmaceutical composition according to claim 14 in the preparation of a medicament for one or more of the following: inhibit Treg function (for example, inhibit the suppressive function of Treg), kill cells expressing OX40 (for example, cells expressing a high level of OX40), enhance an effector T cell function and/or enhance a memory T cell function, reduce tumor immunity, enhance T cell function and/or deplete OX40 expressing cells.

23. A pharmaceutical combination, comprising the anti-OX40 antibody or antigen-binding fragment thereof according to any one of claims 1 to 7, or the antibody-drug conjugate according to claim 8, or the pharmaceutical composition according to claim 14; and one or more additional therapeutic agents.

24. A kit, comprising the anti-OX40 antibody or antigen-binding fragment thereof according to any one of claims 1 to 7, or the antibody-drug conjugate according to claim 8, or the pharmaceutical composition according to claim 14, and preferably further comprising a drug delivery device.
